# EUROPEAN PATENT APPLICATION

(11) **EP 4 717 225 A2**
(43) Date of publication of application: **01.04.2026**
(21) Application number: 26156202.9
(22) Date of filing: 24.11.2021
(51) Int. Cl.: A61B 34/30

(54) **SYSTEM FOR REPEATABLE ALIGNMENT OF BODILY TISSUE FOR PROGRAMME OF EXTERNAL RADIOTHERAPY TREATMENT**

(30) Priority: 24.11.2020 EP 20209528
(62) Divisional of application: 21806117.4
(71) Applicant: Pelviray IP Ltd, 3032 Limassol (CY)
(72) Inventor: POPOWSKI, Georg, 1206 Geneva (CH)
(74) Representative: De Clercq & Partners

(57) **Abstract**

Provided is a radiotherapy treatment system (100) for assisting treatment of a subject in an external radiotherapy programme comprising one or more external radiotherapy treatment sessions, the system (100) comprising: a robotic arm, RA, (400, 400a) having a base end (422, 422a) and an effector end (424, 424a), wherein the base end (422, 422a) is mounted on or mountable in fixed relation to a radiotherapy treatment table (512) for treating a subject (50), the effector end (424, 424a) is disposed with a RA fitting (430, 430a) for dismountable attachment to a positioning tool (200) having a proximal and distal end, the distal end configured for insertion into a canal of the subject (50); a processing unit (440) comprising at least one processor and a memory, wherein the processing unit (440) is configured to control and fix movement of the robotic arm, RA, (400, 400a) during the external radiotherapy treatment session; and the positioning tool (200) comprising a steering guide (300) having a proximal (40) and distal (20) end, comprising a rigid effector shaft (310) at the distal end (20) configured for insertion into the canal (602) of the subject (50), or for attachment to an inserter (204) configured for insertion into the canal (602) of the subject (50) a rigid handle portion (316) disposed at the proximal end (40) in fixed relation to the effector shaft (310) for controlling the position and/or direction of the effector shaft (310) and configured for attachment to the RA fitting (430, 430a), a rigid transmission (314) joining the handle portion (316) to the effector shaft (310); wherein the positioning tool (200) is configured to move and/or fix the canal (602) for the external radiotherapy treatment session responsive to movements of the robotic arm.

## Description

### Field of the invention

The present system and method concern the field of fractionated radiotherapy. In particular, it concerns a robotic arm in combination with a positioning tool that repeatedly and reproducibly places bodily with respect to an ionising radiotherapy beam over the course of treatment.

### Background to the invention

Radiation therapy is a standard treatment for many patients presenting with various cancers including around the breast and pelvic region. Treatment techniques using a radiotherapy device having a rotating ionising-radiation treatment head allow the delivery of very high doses focally to a target for treatment (e.g. a tumour, organ), while sparing adjacent healthy tissue with a great accuracy. Conformal radiotherapy, where the shape of the ionising radiation beam dynamically changes using multileaf collimator as the ionising-radiation treatment head rotates, allows the shape of the tumour to be followed very closely by the beam in three-dimensions. Such systems are manufactured by Varian, CyberKnife,Tomotherapy (Accuray), Shinva, etc. These technologies offer the greatest benefit provided the movements of the targets are reduced to the minimum.

Prior to treatment of the subject by the radiotherapy device, three-dimensional images of the treatment region of the subject are taken by typically CT-scan or, less often, by MRI. From these images, the position target(s) in relation to a beam intersection volume (*e.g.* isocentre) of the ionising radiation beam is determined. The radiation oncologist usually draws the shape of tumor areas on each CT and/or MR slice, as well as the contours of healthy tissues to be protected, which should receive less dose. He defines doses that have to be received by each structure. Physicists and radiation oncology technicians use a dedicated software in order to calculate the "amount" of radiation to be delivered or not through the 360° rotating arm, as well as radiation isodoses around tumour volumes, beam shapes, movements of the treatment head. Data from the simulation, including three-dimensional positional information of beam intersection volume(s), are transferred to the software that is used to operate the radiotherapy device (linear accelerator or other device emitting ionizing radiation). This phase is called "treatment simulation" or "simulation".

The majority of tissue structures lying around the pelvic region - bladder, anus, prostate, rectum, cervix, uterus, vagina - are not affixed to pelvic walls and may move significantly from day when medical images of the treatment region are taken for simulation, to the day when the first treatment by external radiotherapy is delivered by a radiotherapy device.

The subject lying on the table will usually have one or more ink marks, tattoos, or other marks provided on the body, and will be positioned in respect of these marks using one or more laser beams located on lateral walls and projecting along an axis of the radiotherapy treatment table. Most modern external radiotherapy machines have a built-in medical imaging unit that can take (e.g. X-ray, low quality CT scan performed) images of the subject on same table used to treat the patient. This allows, among other things, to ensure a fine-tuning of the position of the tissue target with respect to beam intersection volume of the ionising radiation beam emitted by the radiotherapy device. After this first positioning using laser beam, it is usually the bony structures which are accurately visualized by the built-in medical imaging unit, which are used to precisely position the subject on the treatment table. The subject will always be placed in a reproducible position on both treatment and simulation tables. This positioning of subject, however, does not take into account the displacement of internal structures.

In order to allow surrounding healthy tissue to heal, the radiotherapy treatment is usually fractionated - the total radiation dose split into a number of smaller doses delivered over a period of days, weeks or months. Fractional external radiotherapy may last for 6-7 weeks, usually 5 fractions a week, and positions of the subject are aligned with the ionising radiation beam precisely at every session at millimetre accuracy to focus on the tumour and reduce damage to healthy tissue. This is achieved by aligning the subject accurately on the radiotherapy treatment table using projected laser lines that cross ink marks, tattoo marks on the subject. However, even though the subject is accurately aligned, the positions of the internal organs vary, principally because they are not directly attached to a bony structure. For instance, according to our own investigations, the cervix may move up to 2 cm in each direction, which requires an introduction of large volume margins around the original treatment zone, increasing treated volume, which leads to higher rates of acute and late side effects. Movement of the uterine cervix between sessions may be caused by the filling or emptying of the bladder and/or the rectum, because of bowel movements and partially because of respiratory movements. Therefore, patients may be asked to empty their rectum and fill their bladder before each radiotherapy session. This allows a reduction in uterus displacements, but does not prevent them in a reproducible manner. Indeed, patients are often not able to keep the bladder filled to the same extent during the whole treatment duration (e.g. 28 - 30 sessions) because towards the end of the duration inflammation of the bladder prevents bladder full filling. In addition, emptying the rectum brings both anterior and posterior rectal walls together inside the high dose isodose volumes surrounding the cervix with its 16-20 mm treatment margin which adds to the toxicity of the radiotherapy.

Therefore, there is a need for a system and method allowing repeatable fixing tissue structures in order to benefit from high treatment accuracy.

### Summary of the invention

Provided herein is a radiotherapy treatment system (100) for assisting treatment of a subject in an external radiotherapy programme comprising one or more external radiotherapy treatment sessions, the system (100) comprising:
- a robotic arm, RA, (400, 400a) having a base end (422, 422a) and an effector end (424, 424a), wherein
   - the base end (422, 422a) is mounted on or mountable in fixed relation to a radiotherapy treatment table (512) for treating a subject (50),
   - the effector end (424, 424a) is disposed with a RA fitting (430, 430a) for dismountable attachment to a positioning tool (200) having a proximal and distal end, the distal end configured for insertion into a canal of the subject (50),
      and
- a processing unit (440) comprising at least one processor and a memory,
wherein the processing unit (440) is configured to control movement of the robotic arm, RA, (400, 400a) to position the positioning tool (200) at one or more treatment poses, wherein a treatment pose corresponds a spatial alignment by the positioning tool (200) of a canal (602) of the subject (50) in relation to an ionising radiation beam emitted by an ionising-radiation treatment head (518) during the external radiotherapy treatment session.

Also provided is a radiotherapy treatment system (100) for assisting treatment of a subject in an external radiotherapy programme comprising one or more external radiotherapy treatment sessions, the system (100) comprising:
- a robotic arm, RA, (400, 400a) having a base end (422, 422a) and an effector end (424, 424a), wherein
- the base end (422, 422a) is mounted on or mountable in fixed relation to a radiotherapy treatment table (512) for treating a subject (50),
- the effector end (424, 424a) is disposed with a RA fitting (430, 430a) for dismountable attachment to a positioning tool (200) having a proximal and distal end, the distal end configured for insertion into a canal of the subject (50),
   and
- a processing unit (440) comprising at least one processor and a memory, wherein the processing unit (440) is configured to control and fix movement of the robotic arm, RA, (400, 400a) during the external radiotherapy treatment session, and
- the positioning tool (200) comprising:
   - a steering guide (300) having a proximal (40) and distal (20) end, comprising:
      - a rigid effector shaft (310) at the distal end (20) configured for insertion into the canal (602) of the subject (50), or for attachment to an inserter (204) configured for insertion into the canal (602) of the subject (50),
      - a rigid handle portion (316) disposed at the proximal end (40) in fixed relation to the effector shaft (310) for controlling the position and/or direction of the effector shaft (310) and configured for attachment to the RA fitting (430, 430a),
      - a rigid transmission (314) joining the handle portion (316) to the effector shaft (310),
wherein the positioning tool (200) is configured to move and/or fix the canal (602) for the external radiotherapy treatment session responsive to movements of the robotic arm.

The processing unit (440) may be configured to position the positioning tool (200) at one or more treatment poses, wherein a treatment pose corresponds a spatial alignment by the positioning tool (200) of a canal (602) of the subject (50) for the external radiotherapy treatment.

The processing unit (440) may be configured to determine the one or more treatment poses from one or more empirically-determined simulation poses of the positioning tool (200).

The spatial alignment by the positioning tool (200) of the canal (602) of the subject (50):
- may bring bodily tissue (608) connected to the canal (602) into the ionising radiation beam emitted by an ionising-radiation treatment head (518) during the external radiotherapy treatment session,
   or
- may move bodily tissue (608) connected to the canal (602) away from ionising radiation beam emitted by an ionising-radiation treatment head (518) during the external radiotherapy treatment session.

The processing unit (440) may be configured to determine a treatment pose of positioning tool (200) comprising:
- receiving (a) a set of simulation-determined parameters comprising one or more empirically-determined simulation poses of the positioning tool (200) with respect to a positional reference such as a simulation of a beam intersection volume, a simulation of an isocentre, or a bony pelvis of the subject placed on and in known position with a simulation table (522),
- receiving (b) a positional relationship (SPx,y,z) of the RA base end (422, 422b) of the RA mounted on the simulation table (522) with respect to a positional reference such as a simulation of a beam intersection volume, a simulation of an isocentre, or a bony pelvis of the subject placed on and in known position with a simulation table (522),
- receiving (c) data on a positional relationship (TPx,y,z) of the RA base end (422, 422a) of the RA mounted on the radiotherapy treatment table (512) with respect to a positional reference such as a beam intersection volume, an isocentre, and/or a bony pelvis of the subject of the subject placed on and in known position with a radiotherapy treatment table (512), and-determining from (a), (b) and (c) one or more treatment poses of the positioning tool (200) that corresponds with the one or more empirically-determined simulation poses of the positioning tool (200).

The processing unit (440) may be configured to:
- receive real-time information as to the *in-situ* pose of the positioning tool (200) after the pose of the positioning tool (200) has been adjusted to correspond with the one or more empirically-determined simulation poses of the positioning tool (200),
- provide real-time manual, automatic or semi-automatic guidance to fine-tune the pose of the positioning tool (200) (to further correspond with the one or more empirically-determined simulation poses of the positioning tool (200)),
optionally wherein the real-time information as to the *in-situ* pose of the positioning tool (200) is obtained from a medical imaging unit disposed in known relation to the radiotherapy treatment table (512) or from a positional transponder reader disposed in known relation to the radiotherapy treatment table (512).

The radiotherapy treatment system (100) may further comprise:
- the radiotherapy treatment table (512), wherein the radiotherapy treatment table (512) is disposed with a measurement gauge (514) for determining a position of the RA (400, 400a) base end (422, 422a) with respect to the subject (50), in particular with respect to the subject aligned with at least one preferably two projected laser reference lines,
- a simulation table (522), wherein the base end (422, 422b) of the RA (400, 400b) is mounted on or mountable in fixed relation to the simulation table (522) for determining one or more empirically-determined simulation poses of the positioning tool (200), wherein the simulation table (522) is disposed with a measurement gauge (524) for determining a position of the RA (400, 400b) base end (422, 422b) with respect to the subject (50), in particular with respect to the subject aligned with at least one preferably two projected laser reference lines.

- optionally wherein both measurement gauges (514, 524) have an identical scale.

The radiotherapy treatment system (100) may further comprise a knee support (450) configured to support the knees of the subject lying in a semi-supine position on the radiotherapy treatment table (512) or simulation table (522), wherein the knee support (450):
- comprises a body (451) having a base end (453) and opposing supporting side (457) configured to support both posterior each knee of the subject on the radiotherapy treatment table (512) or simulation table (522),
- is configured for dismountable attachment to and in fixed relation to the radiotherapy treatment table (512) or simulation table (522),
- body (451) comprises a slot (454) into which a base support (432, 432a, 432b) of the RA can lockably slide in one or more positions defined in relation to the radiotherapy treatment table (512) or simulation table (522) and/or to the body (451) of the knee support (451), and
one or both lateral sides (456, 458) of the knee support (450) may be markable or be pre-disposed with a reference marking (464, 465) to indicate a position where a projected laser reference line (516, 526) crosses the one or both two lateral sides (456, 458).

The radiotherapy treatment system (100) may further comprise a knee support (450) configured to support the knees of the subject lying in a semi-supine position on the radiotherapy treatment table (512) or simulation table (522), wherein the knee support (450):
- comprises a body (451) having a base end (453) and opposing supporting side (457) configured to support both posterior each knee of the subject on the radiotherapy treatment table (512) or simulation table (522),
- the body contains the base end (422, 422a) of the robotic arm, RA, (400, 400a).

The knee support (450) may contain two or more parts that mutually attach together to form the knee support (450), wherein
- one part of the knee support (450, a) contains two knee rests (466, 468), one supporting each knee posterior, and optionally a battery pack and optionally the processing unit,
- another part of the knee support (450, b) contains a base support (432, 432a, 432b) of the robotic arm, RA, (400, 400a).

The RA fitting (430, 430a) may be connected to the RA (400, 400a) via linkage fuse, wherein the linkage fuse is configured to:
- tolerate a working load without breaking, and
- break with an application of an abusive load releasing mechanical connection between the RA fitting and the RA (400, 400a).
- break responsive to activation of an emergency button

Further provided is a positioning tool (200) for positioning a canal (602) of a subject in relation to an ionising radiation beam emitted by an ionising-radiation treatment head (518) during an the external radiotherapy treatment session of an external radiotherapy programme comprising one two or more of the external radiotherapy treatment sessions, which positioning tool (200) comprises:
- a steering guide (300) having a proximal (40) and distal (20) end, comprising:
   - a rigid effector shaft (310) at the distal end (20) configured for insertion into the canal (602) of the subject (50), or for attachment to an inserter (204) configured for insertion into the canal (602) of the subject (50), and
   - a rigid handle portion (316) disposed at the proximal end (40) in fixed relation to the effector shaft (310) for controlling the position and/or direction of the effector shaft (310);
   - a rigid transmission (314) joining the handle portion (316) to the effector shaft (310),
wherein
- the positioning tool (200) is configured to move and/or fix the canal (602) for the external radiotherapy treatment session, and
wherein:
an inflatable effector shaft balloon (315) is disposed over the effector shaft (310), the inflatable effector shaft balloon (315) being repeatably inflatable for dilation of the canal (602), and repeatably deflatable for insertion and withdrawal of the effector shaft (310) into or from the canal for each external radiotherapy treatment session,
   and/or
an inflatable transmission balloon (322) is disposed over the distal end (20) of the transmission (314), the inflatable transmission balloon (322) being repeatably inflatable for dilation of the canal (602), and repeatably deflatable for insertion and withdrawal of the transmission (314) into or from the canal for each external radiotherapy treatment session.

Further provided is a positioning tool (200) for positioning a canal (602) of a subject in relation to an ionising radiation beam emitted by an ionising-radiation treatment head (518) during the external radiotherapy treatment session, which positioning tool (200) comprises:
- a steering guide (300) having a proximal (40) and distal (20) end, comprising:
   - an effector shaft (310) at the distal end (20) configured for insertion into the canal (602) of the subject (50), or for attachment to an inserter (204) configured for insertion into the canal (602) of the subject (50), and
   - a handle portion (316) disposed at the proximal end (40) in fixed relation to the effector shaft (310) for controlling the position and/or direction of the effector shaft (310);
   - a transmission (314) joining the handle portion (316) to the effector shaft (310).
wherein the positioning tool (200) is configured to move and/or fix the canal (602) in relation to an ionising radiation beam emitted by an ionising-radiation treatment head (518) for the external radiotherapy treatment session.

The effector shaft (310) may be disposed with an inflatable effector shaft balloon (315), and/or
the distal end (20) of the transmission (314) may be disposed with an inflatable transmission balloon (322).

The inflatable effector shaft balloon (315) may be expansion limited, wherein balloon (315) expansion reproducibly stops at a limited inflation size, and is configured to centre the effector shaft within the canal (602), and/or

The inflatable transmission balloon (322) may be expansion limited, wherein the balloon (322) expansion reproducibly stops at a limited inflation size and configured to centre the transmission (314) within the canal (602).

The inflatable effector shaft balloon (315) may be in fluid connection with an inflation lumen (324) that extends in a proximal (40) direction of the steering guide (300),
optionally wherein:
- the effector shaft balloon (315) inflation lumen (324) is disposed as a channel within a body of at least a part of the effector shaft (310) and/or of the transmission portion (314), or
- the effector shaft balloon (315) inflation lumen (324) is within a tubing (333) disposed within a passage (335) within a body of at least a part of the transmission portion (314);
   and/or
- a fitting (329) such as a Luer fitting is disposed at the proximal end of the effector shaft balloon (315) inflation lumen (328) for connection to a pump
   and/or

- the inflatable transmission balloon (322) may be in fluid connection with an inflation lumen (328) that extends in a proximal (40) direction of the steering guide (300),
optionally wherein:
- the inflatable transmission balloon (322) inflation lumen (328) is disposed as a channel within a body of at least a part of the transmission portion (314), or
- the inflatable transmission balloon (322) inflation lumen (328) is within a tubing (327) disposed within a passage (331) within a body of at least a part of the transmission portion (314);
   and/or
- a fitting (329) such as a Luer fitting is disposed at the proximal end of the inflation lumen (328) for connection to a pump.

The effector shaft (310) may adopt an angle alpha with respect to the transmission (314) and alpha is:
90 to 260 deg and the effector shaft (310) is configured for attachment to an inserter (204) configured for insertion into the canal (602) of the subject (50), or
170 to 190 deg and the effector shaft (310) is configured for direct insertion into a vaginal canal or anal canal of the subject, or
90 to 260 deg and the effector shaft (310) is configured for direct insertion into a rectal canal of the subject, or
   and/or

- the handle portion (316) adopts an angle beta with respect to the transmission (314) and beta is:
   40 to 150 deg and the effector shaft (310) is configured for attachment to an inserter (204) configured for insertion into the canal (602) of the subject (50), or
   70 to 150 deg and the effector shaft (310) is configured for direct insertion into a vaginal canal or anal canal of the subject, or
   40 to 130 deg and the effector shaft (310) is configured for direct insertion into a rectal canal of the subject.

The positioning tool (200) may further comprise an inserter (204) having a proximal (40) and distal (20) end which inserter (204) comprises:
- an elongated member (210) configured for insertion through an entrance of and into the canal; and
wherein the effector shaft (310) at the distal end (20) is attached or dismountably attachable to the elongated member (210).

The positioning tool (200) may be disposed with one or more position-determining transponders (252a-c; 256a-b) configured to transmit real-time information as to the *in-situ* pose of the positioning tool (200) to a positional transponder reader.

The positioning tool (200) may be at least partly visible by a medical imaging unit, optionally wherein the medical imaging unit is built-in to a radiotherapy device (510) that emits the ionising radiotherapy beam for the external radiotherapy treatment session.

The positioning tool (200) may be made at least partly from a material that is MRI compatible material, and
is MRI visible and/or
contains one or more MRI visible markers.

The handle portion (316) may be configured for dismountable attachment to a robotic arm, RA, fitting (430, 430a) disposed at and effector end (424, 424a) of the RA (400, 400a, 400b), optionally wherein the handle portion (316) is provided with a grip locator (330) comprising one or more notches (334) and/or one or more protrusions and/or one or more corners (332) that co-operates with the RA fitting (430) comprising a gripper such that the gripper grips the handle portion (316) with positional repeatability and reduced play or backlash,
optionally, wherein the grip locator (330) comprises:
- one or more notches (334) each having a direction, and
- a corner (332) along an axial direction of the handle portion (316),
wherein at least one notch direction is different from the axial direction, and at least one of the one or more notches is disposed within a span of the corner.

The steering guide (300) may be provided with an image capture system (360) configured to captures images from a distal tip (361) of the effector shaft (310) allowing insertion of the effector shaft (310) into the inserter (204) elongated member lumen (214) under guidance of images captured.

The radiotherapy treatment system (100) may further comprise the positioning tool (200).

The handle portion (316) of the steering guide (300) is disposed with a docking beacon (340) configured to provide real-time information as to the position and optionally orientation of the steering guide (300) relative to the RA fitting (430), wherein the controller (440) is configured to provide manual, semi-automatic or automatic guidance to allow docking of the RA fitting (430) to the handle portion (316),
optionally wherein:
- the docking beacon is passive or active, or a combination of passive and active,
- an active docking beacon is configured to wirelessly emit information about the orientation of the handle portion (316),
- a passive docking beacon comprises a body of a predefined geometric shape recognisable by a vision-guided system of the RA,
- the docking beacon (340) is detachable or non-detachable from the handle portion (316).

The RA (400, 400a) may be provided with a switchable zero-gravity mode, wherein:
- said RA (400, 400a) in a zero-gravity-on mode:
- allows manual guidance of the RA fitting (430, 430a, 430b) for docking with the handle portion (261); and
- continues to register the pose of the RA fitting (430, 430a, 430b),

- said RA (400, 400a) in a zero-gravity-off mode:
   - initially determines the pose of the RA fitting (430, 430a, 430b) from a last registration of the pose of the RA fitting (430, 430a, 430b) upon exiting the zero-gravity-on mode, without an intervening calibration manoeuvre.

The radiotherapy treatment system (100) may be configured such that a pivot point is assignable to the positioning tool (200) that is a point or region of the positioning tool (200) around which movements of positioning tool (200) are pivoted.

Further provided is a radiotherapy treatment system (100) for assisting treatment of a subject in an external radiotherapy programme comprising two or more external radiotherapy treatment sessions, the system (100) comprising:
- a robotic arm, RA, (400, 400a) having a base end (422, 422a) and an effector end (424, 424a), wherein
- the base end (422, 422a) is mounted on or mountable in fixed relation to a radiotherapy treatment table (512) for treating a subject (50),
- the effector end (424, 424a) is disposed with a RA fitting (430, 430a) for dismountable attachment to a positioning tool (200) having a proximal and distal end, the distal end configured for insertion into a canal of the subject (50), or for attachment to an inserter (204) configured for insertion into the canal (602) of the subject (50),
- a processing unit (440) comprising at least one processor and a memory, wherein the processing unit (440) is configured to control movement of the robotic arm, RA, (400, 400a) to position the positioning tool (200) at one or more treatment poses during the external radiotherapy treatment session,
wherein the processing unit (440) is configured to receive an input of a pivot point assignable to the positioning tool (200), wherein the pivot point is a point or region of the positioning tool (200) around which movements of positioning tool (200) by the RA, (400, 400a) are pivoted.

The processing unit (440) may be further configured to maintain the pivot point at a spatially fixed position, and to limit pivoting rotations such that they are centred on the spatially fixed pivot point.

The radiotherapy treatment system (100) may further comprise the steering guide (300) comprising:
- a rigid effector shaft (310) at the distal end (20) configured for insertion into a canal (602) of the subject (50), or for attachment to an inserter (204) configured for insertion into the canal (602) of the subject (50),
- a rigid handle portion (316) disposed at the proximal end (40) in fixed relation to the effector shaft (310) for controlling the position and/or direction of the effector shaft (310).
- a rigid transmission (314) joining the handle portion (316) to the effector shaft (310), wherein the pivot point is disposed on the effector shaft (310) or on the transmission (314) of the steering guide (300), or on the inserter (204).

The positioning tool (200) may be disposed with a scale for reading off a distance for measuring a position of the pivot point.

The pivot point may be positionable corresponding to an entrance to the canal of the subject.

The pivot point may be positionable corresponding to:
- an introit of the vagina, or
- an introit of the anus.

Further provided is a radiotherapy treatment system (100) for assisting treatment of a subject in an external radiotherapy programme comprising two or more external radiotherapy treatment sessions, the system (100) comprising:
- a robotic arm, RA, (400, 400a) having a base end (422, 422a) and an effector end (424, 424a), wherein
- the base end (422, 422a) is mounted on or mountable in fixed relation to a radiotherapy treatment table (512) for treating a subject (50),
- the effector end (424, 424a) is disposed with a RA fitting (430, 430a) for dismountable attachment to a positioning tool (200) having a proximal and distal end, the distal end configured for insertion into a canal of the subject (50), or for attachment to an inserter (204) configured for insertion into the canal (602) of the subject (50), and
- a processing unit (440) comprising at least one processor and a memory, wherein the processing unit (440) is configured to control and fix movement of the robotic arm, RA, (400, 400a) to position the positioning tool (200) at one or more treatment poses during the external radiotherapy treatment session,
wherein the radiotherapy treatment system (100) further comprises:
- the radiotherapy treatment table (512), wherein the radiotherapy treatment table (512) is disposed with a measurement gauge (514) for determining a position of the RA (400, 400a) base end (422, 422a) with respect to the subject (50), in particular with respect to the subject aligned with at least one preferably two projected laser reference lines,
- a simulation table (522), wherein the base end (422, 422b) of the RA (400, 400b) is mounted on or mountable in fixed relation to the simulation table (522) for determining one or more empirically-determined simulation poses of the positioning tool (200), wherein the simulation table (522) is disposed with a measurement gauge (524) for determining a position of the RA (400, 400b) base end (422, 422b) with respect to the subject (50), in particular with respect to the subject aligned with at least one preferably two projected laser reference lines,
- optionally wherein both measurement gauges (514, 524) have an identical scale.

The processing unit (440) may be configured to determine the one or more treatment poses from one or more empirically-determined simulation poses of the positioning tool (200).

The spatial alignment by the positioning tool (200) of the canal (602) of the subject (50) may:
- bring bodily tissue (608) connected to the canal (602) into the ionising radiation beam emitted by an ionising-radiation treatment head (518) during the external radiotherapy treatment session,
   or
- move bodily tissue (608) connected to the canal (602) away from ionising radiation beam emitted by an ionising-radiation treatment head (518) during the external radiotherapy treatment session.

The processing unit (440) may be configured to determine a treatment pose of positioning tool (200) comprising:
- receiving (a) a set of simulation-determined parameters comprising one or more empirically-determined simulation poses of the positioning tool (200) with respect to a positional reference such as a simulation of a beam intersection volume, a simulation of an isocentre, or a bony pelvis of the subject placed on and in known position with a simulation table (522),
- receiving (b) a positional relationship (SPx,y,z) of the RA base end (422, 422b) of the RA mounted on the simulation table (522) with respect to a positional reference such as a simulation of a beam intersection volume, a simulation of an isocentre, or a bony pelvis of the subject placed on and in known position with a simulation table (522),
- receiving (c) data on a positional relationship (TPx,y,z) of the RA base end (422, 422a) of the RA mounted on the radiotherapy treatment table (512) with respect to a positional reference such as a beam intersection volume, an isocentre, and/or a bony pelvis of the subject of the subject placed on and in known position with a radiotherapy treatment table (512), and-determining from (a), (b) and (c) one or more treatment poses of the positioning tool (200) that corresponds with the one or more empirically-determined simulation poses of the positioning tool (200).

The processing unit (440) may be configured to:
- receive real-time information as to the in-situ pose of the positioning tool (200) after the pose of the positioning tool (200) has been adjusted to correspond with the one or more empirically-determined simulation poses of the positioning tool (200),
- provide real-time manual, automatic or semi-automatic guidance to fine-tune the pose of the positioning tool (200),
optionally wherein the real-time information as to the in-situ pose of the positioning tool (200) is obtained from a medical imaging unit disposed in known relation to the radiotherapy treatment table (512) or from a positional transponder reader disposed in known relation to the radiotherapy treatment table (512).

The radiotherapy treatment system (100) may further comprise a knee support (450) configured to support the knees of the subject lying in a semi-supine position on the radiotherapy treatment table (512) or simulation table (522), wherein the knee support (450):
- comprises a body (451) having a base end (453) and opposing supporting side (457) configured to support both posterior each knee of the subject on the radiotherapy treatment table (512) or simulation table (522),
- is configured for dismountable attachment to and in fixed relation to the radiotherapy treatment table (512) or simulation table (522),
- body (451) comprises a slot (454) into which a base support (432, 432a, 432b) of the RA can lockably slide in one or more positions defined in relation to the radiotherapy treatment table (512) or simulation table (522) and/or to the body (451) of the knee support (451),
   and
one or both lateral sides (456, 458) of the knee support (450) may be markable or be pre-disposed with a reference marking (464, 465) to indicate a position where a projected laser reference line (516, 526) crosses the one or both two lateral sides (456, 458).

The radiotherapy treatment system (100) may further comprise a knee support (450) configured to support the knees of the subject lying in a semi-supine position on the radiotherapy treatment table (512) or simulation table (522), wherein the knee support (450):
- comprises a body (451) having a base end (453) and opposing supporting side (457) configured to support both posterior each knee of the subject on the radiotherapy treatment table (512) or simulation table (522),
- the body contains the base end (422, 422a) of the robotic arm, RA, (400, 400a).

The knee support (450) may contain two or more parts that mutually attach together to form the knee support (450), wherein
- one part of the knee support (450, a) contains two knee rests (466, 468), one supporting each knee posterior, and optionally a battery pack and optionally the processing unit,
- another part of the knee support (450, b) contains a base support (432, 432a, 432b) of the robotic arm, RA, (400, 400a).

The RA fitting (430, 430a) may be connected to the RA (400, 400a) via linkage fuse, wherein the linkage fuse is configured to:
- tolerate a working load without breaking, and
- break with an application of an abusive load releasing mechanical connection between the RA fitting and the RA (400, 400a).
- break responsive to activation of an emergency button.

### Figure Legends

**FIG.** 1 depicts an exemplary positioning tool comprising a steering guide provided herein, with alternative placements of optional guiding strand passage exit and alternative placements of optional balloons.
**FIG. 1A** depicts angle gamma viewed along eyeline (e) in FIG. 1A.
**FIG. 2** schematic illustration of a robotic arm mountable to a radiotherapy treatment table or simulation table.
**FIG.** 3 depicts an isometric view of an exemplary positioning tool comprising an inserter.
**FIG. 4A-E** depicts various longitudinal cross-sectional views of an inserter. In FIG. 4A, the elongated member lumen is without steering guide or guiding strand. In FIG. 4B, the elongated member lumen is provided with a dismountably-attached guiding strand. In FIG. 4C, the steering guide is permanently attached. In FIG. 4D and 4E, the elongated member lumen is provided with a dismountable guiding strand. **FIG. 4F** depicts a removable guiding strand with a threaded end of FIG. 4E.
**FIG. 5** schematic illustration of radiotherapy treatment room containing a radiotherapy treatment table and radiotherapy device.
**FIG. 6** schematic illustration of radiotherapy simulation room containing a simulation table and a medical imaging device.
**FIG. 7** flow chart depicting a computer implemented method according to the present invention.

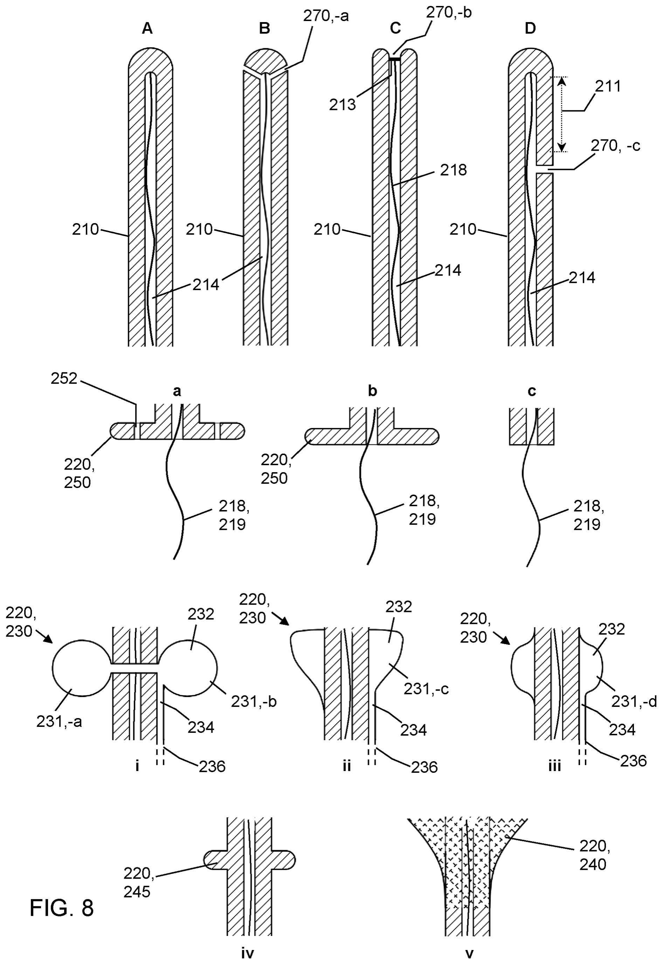

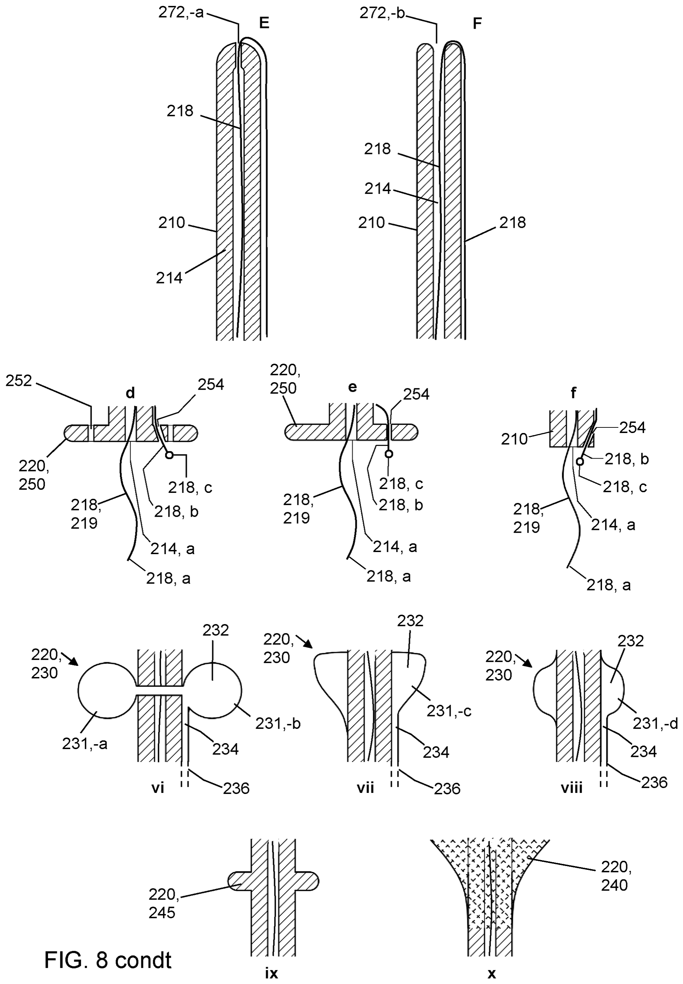

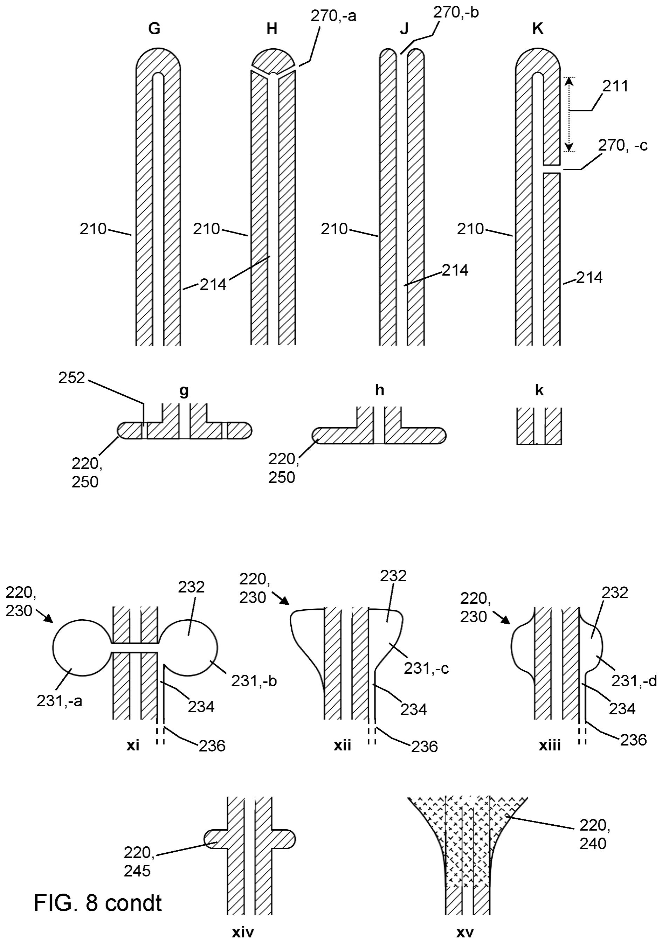

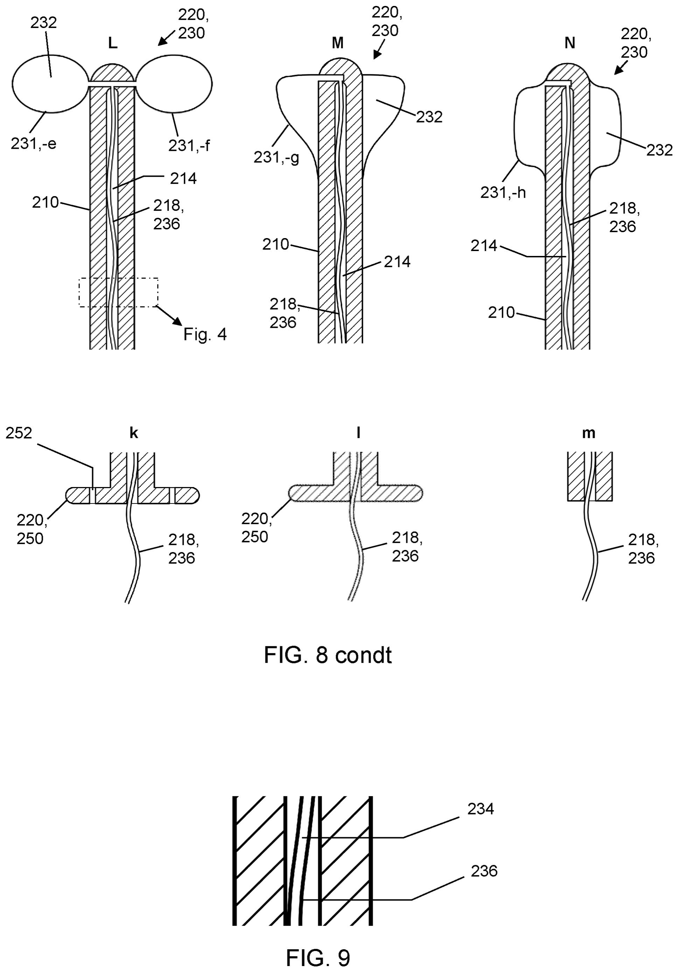

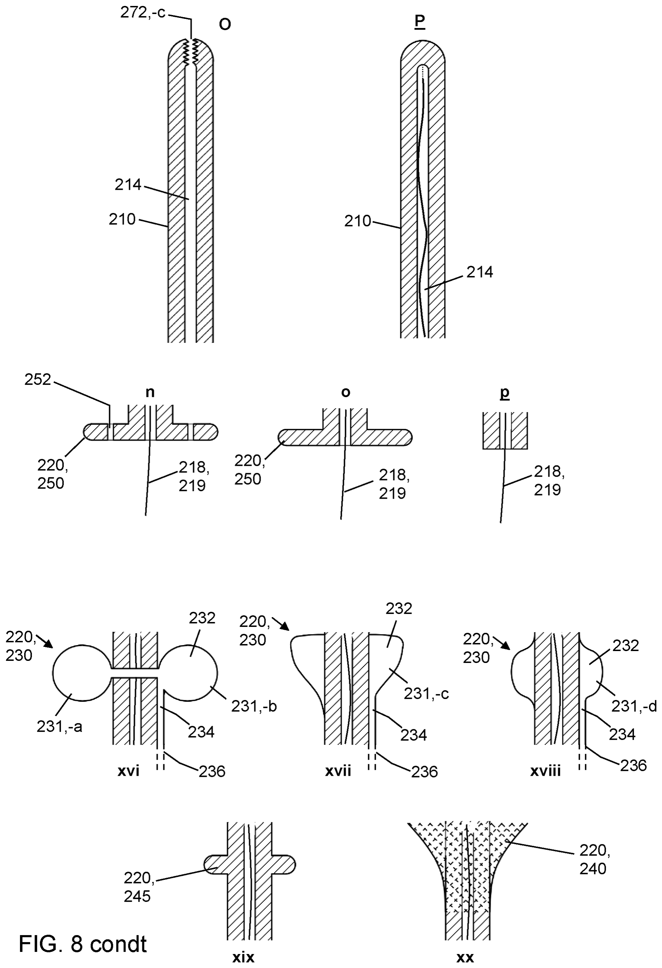

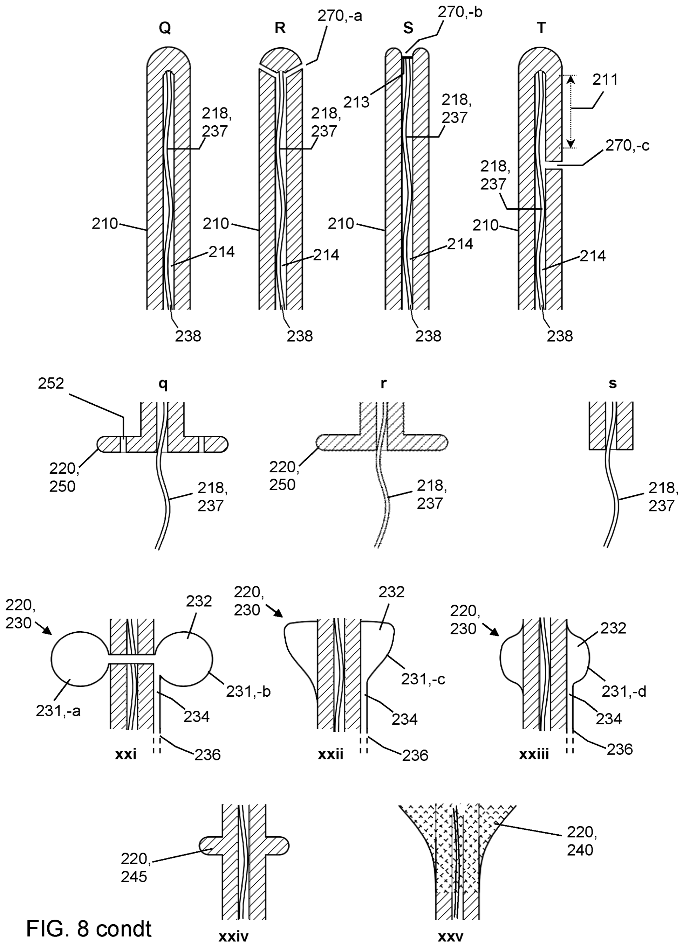

**FIG. 8** depicts different elements of an inserter that are combinable including elongated member (A to D), proximal slide restrictor (a to b) or none (c), distal slide restrictor (i to v) or none, wherein the guiding strand is a flexible cord; elongated member (E to F), proximal slide restrictor (d to e) or none (f), distal slide restrictor (vi to x) or none, wherein the guiding strand is dismountable; elongated member (G to K), proximal slide restrictor (g to h) or none (j), distal slide restrictor (xi to xv) or none, wherein the guiding strand is absent; elongated member (L to N), proximal slide restrictor (k to m), wherein the guiding strand is an inflation tube; and elongated member (O to P), proximal slide restrictor (n to o) or none (p), distal slide restrictor (xvi to x), wherein the guiding strand is dismountably attached to the elongated member; elongated member (Q to T), proximal slide restrictor (q to r) or none (s), distal slide restrictor (xxi to xxv), wherein the guiding strand is a flaccid tube.
**FIG. 9** shows an enlarged view of an inflation tube (236) within an inflation lumen (234).
**FIG. 10** shows a positioning tool (200) described herein with an inserter (204) and steering guide (300); the inserter is located in the cervical canal (602').
**FIGs. 11** and **12** each depict a configuration of a steering guide with a one-piece polymeric handle and transmission, and each having a different configuration of guiding strand passage exit of the steering guide.
**FIG. 13** depicts a steering guide provided with an inflatable transmission balloon.
**FIG. 14** depicts a steering guide where the guiding strand passage is a slot; detail of an entrance to the slot is shown in FIG. 14A.
**FIGs. 15** to **18** depict an alternative configuration handle portion (316) of the steering guide having a grip locator comprising notches or corner.
**FIG. 19** shows a configuration of a steering guide with a one-piece polymeric handle and transmission, and with a handle portion (316) having a grip locator containing a corner.
**FIGs. 20****, A to C** shows detailed views of the handle portion (316) of FIG. 19 having a grip locator containing a notch and corner.
**FIG.21** shows a configuration of a steering guide as similar to that of FIG. 19, additionally provided with a transmission balloon.
**FIG.22** shows a longitudinal cross-section of the steering guide of FIG. 21, having a fitting (329) integrated into the steering guide body.
**FIG.23** shows a longitudinal cross-section of the steering guide similar to FIG. 21, wherein fitting (329) is an inline fitting.
**FIG.24** shows a configuration of a steering guide for anal or vagina insertion, additionally provided with an effector shaft balloon (angle alpha = 180 deg).
**FIG.25** shows a detail of the effector shaft balloon of FIG. 23.
**FIG.26** shows a configuration of a steering guide for anal or vagina insertion, additionally provided with an effector shaft balloon.
**FIG.27** shows a longitudinal cross-section of the steering guide of FIG. 26, having a fitting (326) integrated into the steering guide body.
**FIG.28** shows a longitudinal cross-section of the steering guide similar to FIG. 27, wherein the fitting (326) is an inline fitting.
**FIGs.29 to 31** each show a steering guide provided with a different docking beacon.
**FIGs. 32 to 35** show different inserters located in the cervical canal.
**FIG. 36** and **37** - illustrations of a positioning tool inserted into the canal, where the different poses **(A** and **B)** of the positioning tool change the position of bodily tissue.
**FIG. 38** shows a positioning tool comprising an inserter provided herein located in the cervical canal, and mounted on the steering guide, and movement of the steering guide changing the position of the cervix and uterus.
**FIG. 39** shows an isometric view of a robotic arm, integrated into a cushion, and attached to a steering guide. Both apex parts have the same height.
**FIG. 40** shows a side view of the robotic arm of FIG. 39.
**FIG. 41** shows an isometric view of a robotic arm, integrated into a cushion, and attached to a steering guide. Both apex parts have a different height.
**FIGs. 42A** and **42B** each show a composite medical image illustrating a change of positioning tool (inserter) pose recorded during simulation and during a treatment session and prior to commencement of exposure. **FIG. 42A** is a side view of positioning tool (inserter), **FIG. 42B** is an axial view of positioning tool (inserter).
**FIG. 43** is a Dose-Volume graph indicating dose/volume distribution received by a structure for rectum, bladder and cervix when the cervix is not immobilised (a, b, c respectively) or when the cervix is immobilised (a', b', c').
**FIG. 44** is a view of a steering guide having ability to capture images and provide illumination. **Panel A** depicts the steering guide, **Panel B** is an enlargement of the distal tip of the effector shaft with light outlet ports and image entry port, **Panel C** shows an optional ancillary unit housing electrics, and optionally an image sensor and optionally a light source.
**FIG. 46** shows a first part of a two part knee support.

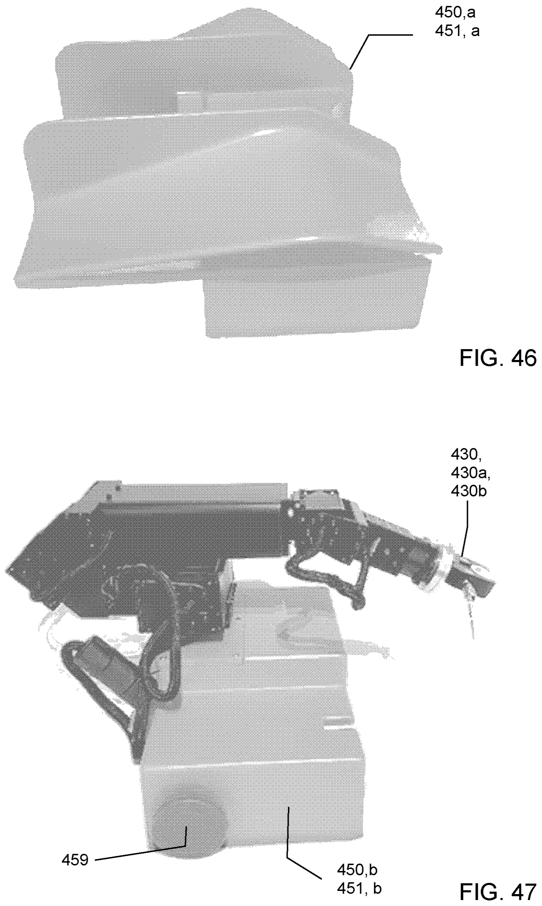

**FIG. 46** shows a second part of a two part knee support containing the base support of the robotic arm.
**FIG. 47** shows the first and second parts of the knee support mutually attached.

### Detailed description

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal", or "distal side", or "distal to" and "proximal", or "proximal side", or "proximal to" are used through the specification, and are terms generally understood in the field to mean towards (proximal) or away (distal) from the practitioner's side of the apparatus. Thus, "proximal" means towards the practitioner's side and, therefore, away from the subject's side. Conversely, "distal" means towards the subject's side and, therefore, away from the practitioner's side.

The term "longitudinal" is generally understood in the field to mean along the longer length of the treatment or simulation table. It may be used to refer to the radiotherapy treatment or simulation table itself, or to a device attachable to the radiotherapy treatment or simulation table, or to a subject lying on the radiotherapy treatment or simulation table.

The term "lateral" is generally understood in the field to mean along a shorter length of the treatment or simulation table i.e. from side-to-side or left to right. It may be used to refer to the radiotherapy treatment or simulation table itself, or to a device attachable to the radiotherapy treatment or simulation table, or to a subject lying on the radiotherapy treatment or simulation table.

The term "superior" is understood to mean towards a head of a subject. It may be used to refer to the radiotherapy treatment or simulation table, or to a device attachable to the radiotherapy treatment or simulation table, or to a subject lying on the radiotherapy treatment or simulation table. The term "inferior" is understood to mean towards the feet of a subject. It may be used to refer to the radiotherapy treatment or simulation table, or to a device attachable to the radiotherapy treatment or simulation table, or to a subject lying on the radiotherapy treatment or simulation table.

Provided herein is a system and method for assisting an external radiotherapy programme applied to a subject. The external radiotherapy programme comprises a least one session or fraction of external radiotherapy treatment and/or a simulation of treatment. It is applied to a subject, in particular to a body of the subject.

By external radiotherapy treatment programme, it is meant one or more sessions of radiotherapy treatment provided to a treatment site of the subject by an external radiotherapy source (e.g. through a linear accelerator optionally equipped with a multileaf collimator, or a tomotherapy system, or by any radiation source moving around the patient such as in the Cyberknife system). The treatment may be composed of one or more sessions or fractions. Where there is a plurality of fractions, the overall dose is divided into a plurality of smaller doses delivered at a number of intervals over time (fractionation) e.g. separated by a period of, hours, days or weeks. Typically duration is 28-30 fractions for treatment of a cervical tumour, each fraction delivering a dose of up to 2.6 Gy to the tumour. By fractioning treatment, healthy cells surrounding the site of treatment are less damaged, and the time interval allows recovery. It is also possible to use the system to deliver the treatment in a few fractions as a boost, or as a palliative therapy in case of bleeding, with high doses per fraction, for instance, 5 fractions of 6 Gy, or 5 fractions of 4 Gy delivered to the tumor as a boost or as a palliative therapy.

A radiotherapy programme typically comprises a simulation part and a treatment part. The simulation part involves acquiring internal medical images (e.g. by CT, MRI) of the subject usually in three-dimensions while the subject is accurately aligned on a moveable simulation table in relation to an imaging device. These medical images are used to plan the subsequent external radiotherapy treatment. The radiologist determines from the images which tissues structures are to receive higher doses, lower doses, sensitive structures, the number of sessions or fractions, and the like.

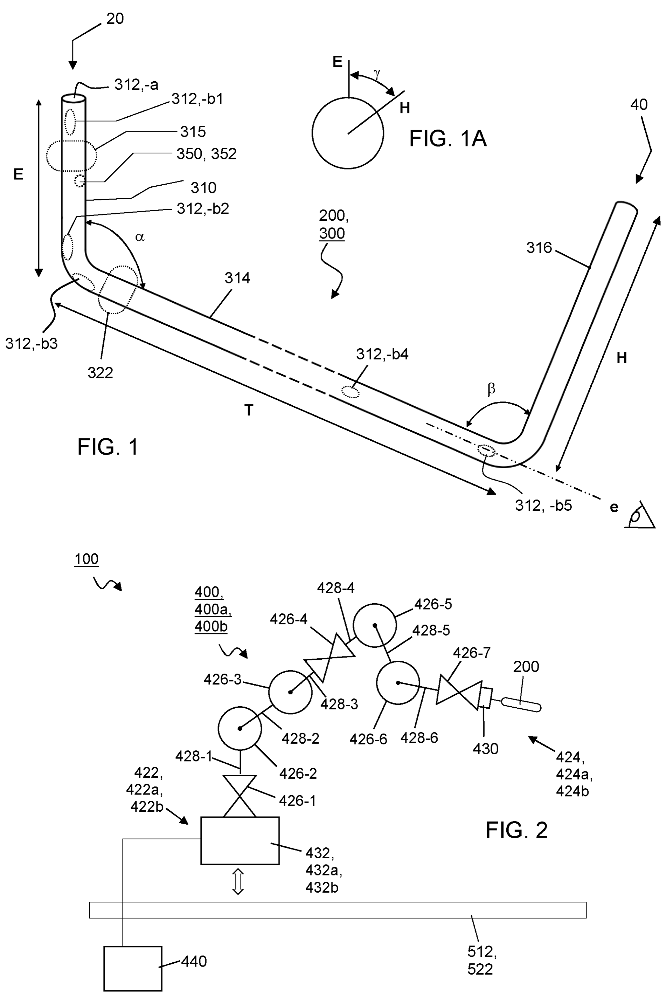

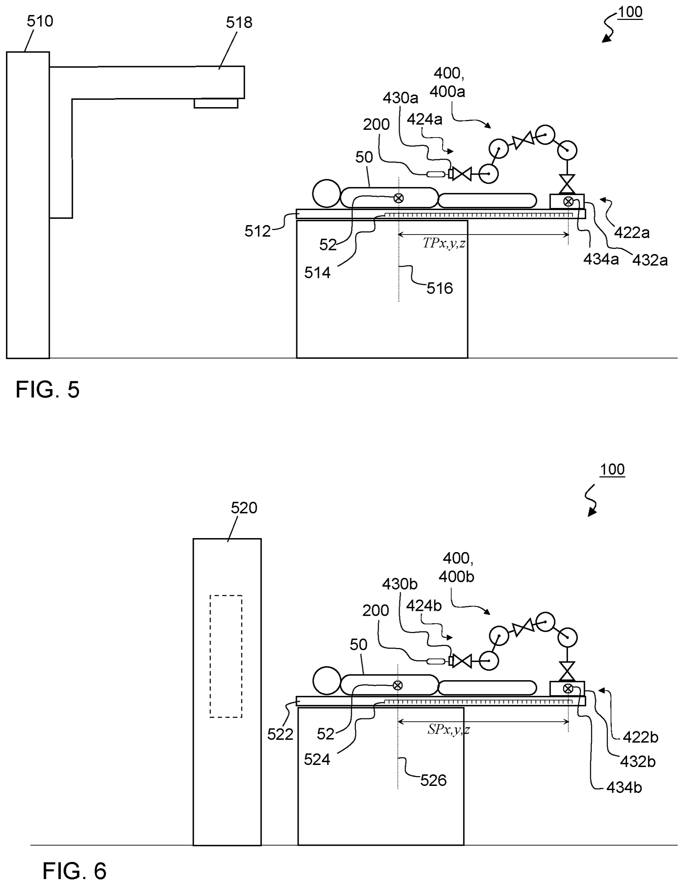

The system (100) (e.g. **FIGs. 2****,** **5, 6****)** comprises a robotic arm, RA, (400, 400a, 400b) having a base end (422, 422a, 422b) and an effector end (424, 424a, 424b). The base end (422, 422a) of the RA (400, 400a) is mountable in fixed and known relation to a radiotherapy treatment table (512) for treatment of a subject (50) **(****FIG. 5****).** Alternatively the base end (422, 422b) of the RA (400, 400b) is mountable in fixed and known relation to a simulation table (522) for a simulation of treatment a subject (50) **(****FIG. 6****).** The effector end (424, 424a, 424b) is disposed with a RA fitting (430, 430a, 430b) for dismountable attachment to a positioning tool (200) for insertion into a canal of the subject (50). Using the RA (424, 424a, 424b), the positioning tool (200) may be set at the same or similar one or more treatment poses relative to the subject (50). During the treatment phase **(****FIG. 5****)** the canal (602) (*e.g.* cervical canal) is moved by the positioning tool (200) during the external radiotherapy treatment session. During the treatment phase **(****FIG. 5****)** the canal (602) (*e.g.* cervical canal) is moved by the positioning tool (200) in relation to an ionising radiation beam emitted by an ionising-radiation treatment head (518) during the external radiotherapy treatment session, in particular in relation to a beam intersection volume of the external ionising radiotherapy beam. Movement of the canal also causes movement of bodily tissue of the subject in relation to the external ionising radiotherapy beam, in particular in relation to a beam intersection volume of an external ionising radiotherapy beam. The canal can be accurately moved and fixed by the positioning tool (200) in order to change and fix the position of canal and bodily tissue relative to the external ionising radiotherapy beam. The bodily tissue may be in the canal, or may be a structure that moves when the canal is moved (*e.g.* prostate that moves with the rectal canal). The bodily tissue (608) may be tissue that is a target of treatment (*e.g.* a tumour) and is to be brought into the external ionising radiotherapy beam for exposure to the beam; accordingly, the target(s) for receiving treatment is (are) highly accurately positioned, allowing receipt of maximal dose and reducing damage to healthy structures. Alternatively, the bodily tissue may be tissue that is to be moved out of the external ionising radiotherapy beam thereby avoiding exposure to the beam; accordingly, healthy tissue can be displaced away from a target, allowing a more isolated exposure.

During simulation **(****FIG. 6****)** under medical imaging, the canal can be accurately placed or moved by the positioning tool (200) in order to align the bodily tissue relative to an external ionising radiotherapy beam, in particular relative to a positional reference, and the pose of the positioning tool (200) recorded which pose is used for the subsequent treatment.

Radiotherapy is delivered to the subject while the canal is held at one or more of the treatment poses. At one or more subsequent sessions, the treatment pose(s) may be reproduced. The system allows bodily structures that can change their shape or position between sessions to be brought into a known, well defined position and stabilised during each session.

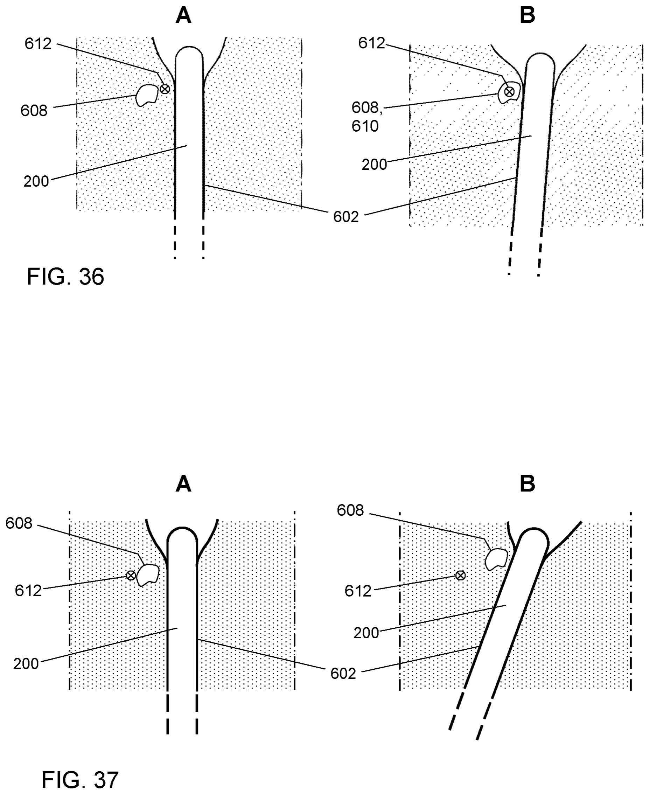

In **FIG. 36****, Panel A** depicts a positioning tool (200) inserted in a canal (602) of the subject and bodily tissue (608) connected to the canal (602). By adjusting the pose of the positioning tool (200) **(Panel B),** the position of bodily tissue (608) can be stabilised, adjusted and fixed relative to a beam intersection volume (612) (e.g. isocentre) of the ionising-radiation beam emitted by an ionising radiation head. The beam intersection volume (612) has a constant position in Panels A and B. In the case of **FIG. 36** **Panel B,** bodily tissue (608) is also a treatment target (610) that is brought into the beam intersection volume (612) for exposure to ionising-radiation. The pose of the positioning tool (200) can be recorded and re-applied in subsequent sessions of fractionated treatment.

**In** **FIG. 37** **Panel A** depicts a positioning tool (200) inserted in a canal (602) of the subject and bodily tissue (608) connected to the canal (602). By adjusting the pose of the positioning tool (200) **(Panel B),** the position of bodily tissue (608) of can be stabilised, adjusted and fixed relative to a beam intersection volume (612) (*e.g.* isocentre) of the ionising-radiation beam emitted by an ionising radiation head. The beam intersection volume (612) as a constant position in Panels A and B. In the case of **FIG. 37** **Panel B,** bodily tissue (608) is not a treatment target and it is brought outside the beam intersection volume (612) for avoiding exposure to ionising-radiation. The pose of the positioning tool (200) can be recorded and re-applied in subsequent sessions of fractionated treatment.

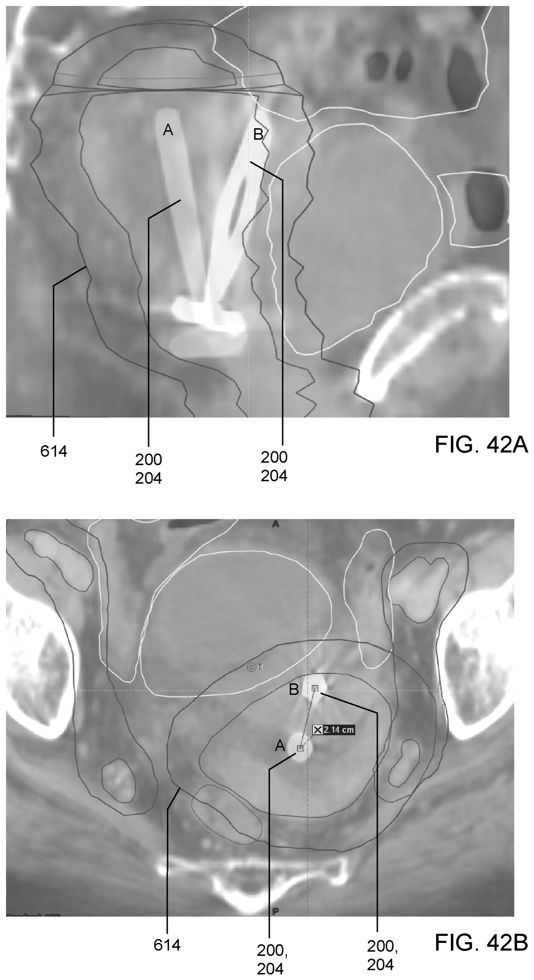

The extent of movement of internal organs or tissue in a subject between simulation and treatment, or between treatment sessions is illustrated in **FIGs. 42A** to **42B****.** Each figure is a fused X-ray image of a subject undergoing simulation and treatment for a cervical tumour in which the positioning tool (200) inserter (204) presently described has been inserted into the cervical canal, wherein the images taken during simulation and the subsequent 1^{st} treatment session were superimposed. The external margin (614) of the irradiated volume is indicated. A first pose (A) of the positioning tool (200) inserter (204) adopted in simulation corresponded to a natural placement of tissues and organs and there was minimum of active pose adjustment by the robotic arm. A second positioning tool (200) inserter (204) pose (B) was recorded in a subsequent 1^{st} treatment session prior to adjustment by the robotic arm. There was a significant difference between the first (A) and second (B) poses due to the internal movements of the tissues and organs between the sessions that caused displacement of the inserter (204) tip by around 2.1 cm, and a corresponding shift in the position of the treatment target. The new pose (B) was on the external margin of the irradiated volume, and would have received a much lower dose than calculated. The internal movement was corrected by the present systems and methods by an adjustment to the pose of the positioning tool (200) inserter (204) by the robotic arm during the treatment session to correspond with the pose recorded during simulation, leading to a significant reduction in toxicity.

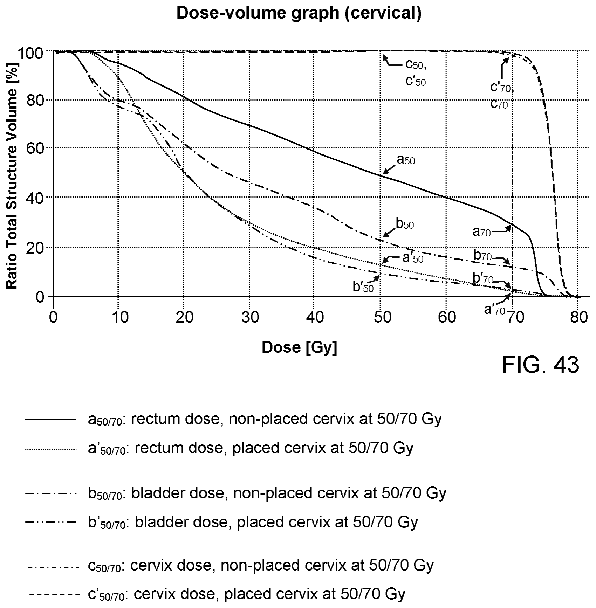

The effect of the positioning tool on toxicity reduction in adjacent structures is illustrated in **FIG. 43** which is a dose-volume histogram chart illustrating the relationship between the percentage of irradiated volume of each organ (rectum, bladder, cervix) and the dose received by said irradiated volume of rectum (a, a'), bladder (b, b') and cervix (c, c'), that was calculated for an external radiotherapy treatment of the cervix when the cervix was not placed (natural position) (a, b, c) and when the cervix was placed (a', b', c') using the positioning tool. The rectum (a, a') and bladder (b, b') are recipients of radiation toxicity when the cervix is the treatment target. The margins in the present calculation were 16 mm when the cervix was not placed (tight margins) and 5 mm when the cervix was placed using the positioning tool.

For the rectum (a, a'), when the cervix was not placed using the positioning tool, 50 % of the rectum volume received a dose of 50 Gy (a₅₀); when the cervix was placed with the positioning tool 13 % of the rectum volume received a dose of 50 Gy (a'₅₀). When the cervix was not placed, 30 % of the rectum volume received a dose of 70 Gy (a₇₀); when the cervix was placed with the positioning tool 2.5 % of the rectum volume received a dose of 70 Gy (a'₇₀). Hence, with the positioning tool placement of the cervix, the dose volume received by the rectum was reduced from 50 % to 13 % at 50 Gy (3.9 fold reduction) and from 30 % to 2.5 % at 70 Gy (12 fold reduction).

For the bladder (b, b'), when the cervix was not placed 22 % of the bladder volume received a dose of 50 Gy (b₅₀); when the cervix was placed with the positioning tool 10 % of the bladder volume received a dose of 50 Gy (b'₅₀). When the cervix was not placed 12 % of the bladder volume received a dose of 70 Gy (b₇₀); when the cervix was placed with the positioning tool 3 % of the bladder volume received a dose of 70 Gy (b'₇₀). Hence, with the positioning tool placement of the cervix, the dose volume received by the bladder was reduced from 22 % to 10 % at 50 Gy (2.2 fold difference) and from 12 % to 3 % at 70 Gy (4 fold reduction).

For the cervix (c, c'), 100 % of the cervix volume received a dose of 50 Gy (c₅₀) when the cervix was not placed; when the cervix was placed with the positioning tool 100 % of the cervix volume also received a dose of 50 Gy (c'₅₀). When the cervix was not placed ~98 % of the cervix volume received a dose of 70 Gy (c₇₀); when the cervix was placed with the positioning tool, ~99.5 % of the cervix volume received a dose of 70 Gy (c'₇₀). Hence, the positioning tool did not affect the cervix volume/dose, and lead to a slight increase in volume of cervix receiving the higher 70 Gy dose.

The results in **FIG. 43** demonstrate that high doses can be delivered externally to the cervix while reducing complications caused by irradiation of the rectum and bladder. Conventionally, such higher doses would be need delivered by brachytherapy (internal radiation source) which is more targeted for internal structures but is more complex The majority of patients with cervical cancer, who are not operable because of vaginal or parametrial invasion, will need an implant of invasive structures with brachytherapy needles, with the need of anaesthesia and hospitalization. This is feasible in only a limited number of radiotherapy centers and this makes this treatment not accessible in all patients. This complex brachytherapy intervention could be avoided by immobilising and placing the cervix using the present positioning tool (200). The immobilization and placement of the cervix allows doses to the cervix to be boosted without excessive toxicity on surrounding tissues and would suppress the need for brachytherapy.

The position and orientation of the RA fitting (430, 430a, 430b) determines the pose of the positioning tool (200). The positioning tool (200) pose refers to the position and direction of the positioning tool (200). The pose may be determined relative to a positional reference.

The positional reference refers to a reference point in three-dimensional space that is known relative to a beam intersection volume (*e.g.* isocentre) of the ionising-radiation beam emitted by the ionising radiation head, or to the simulation thereof. Typically the ionising radiation head moves such that different ionising-radiation beam directions intersect during a treatment session, thereby minimising damage to surrounding tissue. A beam intersection volume is the volume in which different ionising-radiation beam directions intersect during an external radiotherapy treatment session. A simulated beam intersection volume is the volume in which different simulated ionising-radiation beam directions intersect during a simulation of an external radiotherapy treatment session. The (simulated) beam intersection volume usually coincides with the tissue target (*e.g.* tumour) in the subject.

The positional reference in both treatment and simulation is the same or equivalent; both treatment and simulation rooms are constructed so that positions and orientation adopted in one room can be exactly replaced in the other, based on the common positional reference.

Where the radiotherapy device (510) is provided with a ionising-radiation treatment head (518) that rotates around a single axis, the beam intersection volume is also known as an isocenter that is a centre of rotation of the ionising-radiation beam emitted by the ionising-radiation treatment head (518) during the external radiotherapy treatment session. The isocentre is well known in the art, see for example, http://ozradonc.wikidot.com/isocentre-of-the-linac. Devices having an isocentre (linac) are manufactured for instance by Varian. A simulated isocentre is centre of rotation the ionising-radiation beam emitted by the ionising-radiation treatment head (518) during a simulation of an external radiotherapy treatment session.

In other systems (*e.g.* Cyberknife), the radiotherapy device (510) is provided with a ionising-radiation treatment head (518) mounted on a robotic arm having three or more degrees of freedom of movement that offers directional control of the ionising-radiation beam around more than one axis. The beam intersection volume is the volume in which the different directions of the ionising-radiation beam emitted by the ionising-radiation treatment head (518) during an external radiotherapy treatment session intersect. The simulated beam intersection volume is the volume in which the different directions of the ionising-radiation beam emitted by the ionising-radiation treatment head (518) during a simulation of external radiotherapy treatment session intersect.

The positional reference may in practice be the radiotherapy treatment table (512) whose position is known (*e.g.* by encoders) relative to the aforementioned beam intersection volume, the ionising-radiation treatment head (518) whose position is known relative to the aforementioned beam intersection volume, the base end (422, 422a) of the robotic arm (400, 400a) whose position on the radiotherapy treatment table (512) is known, the projected laser reference lines (516) whose position is known relative to the beam intersection volume, the subject (50) who is accurately positioned on the radiotherapy treatment table (512), or the bony structure of the subject (50) such as pelvis.

The positional reference may in practice be the simulation table (522) whose position is known (*e.g.* by encoders) relative to the aforementioned simulated beam intersection volume, a simulation of the ionising-radiation treatment head (518) whose position is known relative to the aforementioned simulated beam intersection volume, the base end (422, 422b) of the robotic arm (400, 400b) whose position on the simulation table (522) is known, the projected laser reference lines (526) whose position is known relative to the simulated beam intersection volume, the subject (50) who is accurately positioned on the simulation table (522), or the bony structure of the subject (50) such as pelvis.

The RA (400, 400a, 400b) comprises a base end (422), an effector end (424) and a plurality of intervening linkages (428-1 to -6 or -7) connected by joints (426-1 to -6 or -7), wherein the arrangement of linkages and joints provides at least 3, 4, 5 or 6 degrees of freedom (DOF) of movement to the effector end, preferably 6 DOF. Exemplary arrangement of joints and linkages are illustrated in **FIGs. 2****,** **39,** and **40** where each joint (426-1 to -6 or -7) is a revolute joint. The joints are actuatable, typically by motors, hydraulics, or pneumatics allowing control of the position and direction of the effector end by electronic signals. Each joint, also known as a kinematic pair, may offer 1 or 2 degrees of freedom (DOF) of movement, preferably 1 DOF. A joint may be a revolute or prismatic joint. A revolute joint has one degree of freedom of movement that is rotational. A prismatic joint has one degree of freedom of movement that is a linear displacement *i.e.* slidable. Typically a robotic arm comprises 6 joints each having 1 DOF to generate 6 DOF of movement to the effector end. Where a robot arm contains more than 6 joints, the position and direction of the effector can be attained using a plurality of different combinations of joint positions, offering redundancy that is useful for instance where the path of the robotic arm is restricted. The joints include any integrated into a RA, and any joints added by way of an adapter added to the effector end of the robotic arm.

One or more, preferably each and every joint of the RA may be provided with a force sensor, to detect an application of an external force to the joint. Where the joints are revolute joints, a joint torque sensor may be embedded in each joint so as to measure the joint torque. The external force may be applied to a link, or to the effector end. Detection of a force allows the detection of collision of a link against another object such as an adjacent instrument, the linear accelerator head, or with the subject or medical professional. Detection of an external force allows the detection of force applied to the steerable tool. Joint torque sensors are already provided in some commercially available robotic arms, such as those manufactured by Kuka (Kuka LBR Med, Germany).

The robotic arm may be commercially provided, for instance, as manufactured by Kuka or may be an adapted commercially available robotic arm. An adaptation to an existing robotic arm includes, for instance, a replacement of one or more joints or linkage, or an addition of one or more controllable degrees of freedom using an adapter attached to the effector end thereby creating a new effector end, by adding a force sensor and/or a gripper at the effector end.

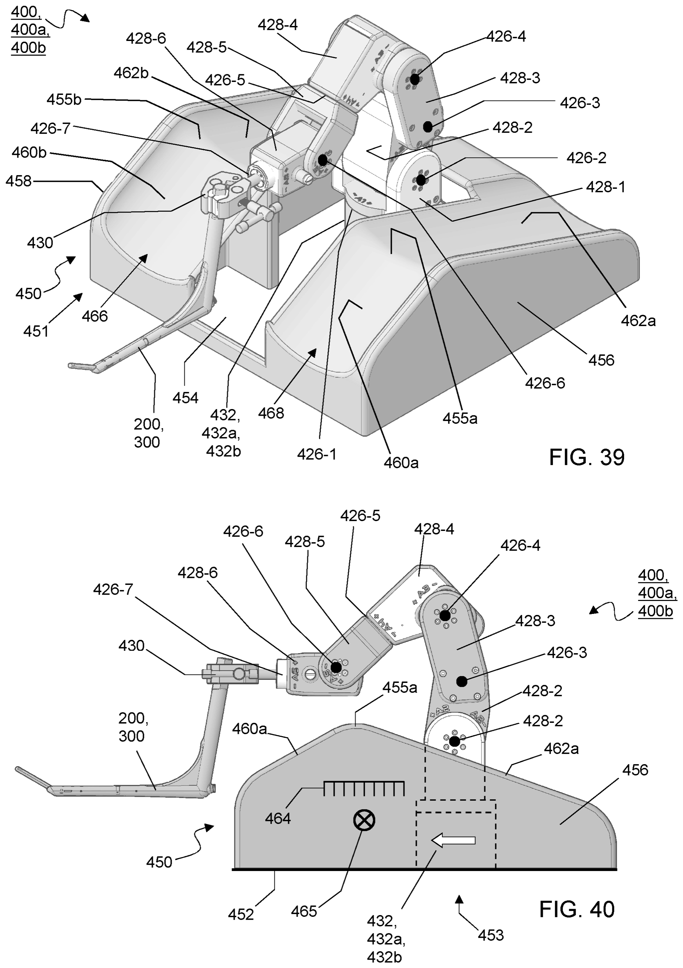

The RA (400, 400a, 400b) may be custom-made, in order to comply with size requirements restrictions imposed by the simulation table, radiotherapy treatment table, ionising-radiation treatment head (518), the medical imaging device (520), etc. An operational height of the RA (400, 400a, 400b) as measured from the upper surface of the radiotherapy treatment table (512) and simulation table (522) to a maximal operational height of its linkages may be less than 50 to 55 cm. Typically, a distance between the simulation table (522) and the upper part of the CT scanner (520) bore vault is equal or inferior to 50 to 55 cm. Typically a distance between treatment table (512) and a linac (518) collimator outer surface is maximum 50 cm. The RA (400, 400a, 400b) may be medical imaging (*e.g.* CT, MRI or X-ray) compatible. The RA (400, 400a, 400b) may be compatible with ionising radiation beam emitted by an ionising-radiation treatment head (518). The RA (400, 400a, 400b) may be compatible with a linear accelerator. An exemplary low height profile RA (400, 400a, 400b) is illustrated in **FIGs. 39** and **40****.** The RA (400, 400a, 400b) contains shortened linkages (428-1 to -7) that limit a maximum operational height of the RA.

The operational height of the RA (400, 400a, 400b) as measured from an upper surface of the radiotherapy treatment table (512) or simulation table (522) to a maximal operational height of its linkages may be less than 50 to 55 cm.

The processing unit may be configured to limit the operational height of the RA (400, 400a, 400b) as measured from an upper surface of the radiotherapy treatment table (512) or simulation table (522) to a maximal operational height of its linkages is less than 50 to 55 cm.

The RA (400, 400a, 400b) may have a switchable zero gravity mode. In zero-gravity-on (weightless) mode, the joints of the robotic arm may or may not be supported (e.g. by the servos) to prevent collapse of the arm. The pose of the RA fitting (430, 430a, 430b) may be guided manually *e.g.* by the medical staff. This allows ease of connection between the positioning tool (200) and the RA fitting (430, 430a, 430b) when the positioning tool (200) has already been inserted into the subject. It also allows a manual fine-tuning of the pose of the steering guide (300) for simulation and treatment. Once the connection between end effector fitting and handle portion (316) has been made, the zero-gravity mode may be deactivated and the actions performed as described elsewhere herein. The RA operating in zero-gravity-on mode may continue to register the pose of the RA fitting (430, 430a, 430b), so that when zero gravity mode is deactivated (zero-gravity-off mode), the RA can continue to be controlled by the controller processing unit (440) and the steering guide (300) to the treatment or simulation pose without an intervening calibration maneuver. Thus, upon entering the zero-gravity-off mode, the pose of the RA fitting (430, 430a, 430b) is initially determined from a last registration of the pose of the RA fitting (430, 430a, 430b) upon exiting the zero-gravity-on mode.

Force applied by the positioning tool (200) may be limited to prevent harm to the subject. The force may be measured based on at least one sensor disposed in the RA fitting (430, 430a) and/or in the RA arm joints, or based on current consumption by one or more RA motors. The processing unit may be configured to limit the force. The force by the positioning tool (200) may be 100 g to 2000 g force (0.980665 N to 19.6133 N). According to one example, the RA (400, 400a, 400b) may give a feedback on the current consumption by one or more, preferably all of its joint motors. Each of the motors of the RA (400, 400a, 400b) may constantly transmit its current consumption. A limit can be set on the current consumed by the motor, thus allowing a limitation of the maximum effort of the motor and hence of the RA (400, 400a, 400b) when the limit is exceeded. By constantly monitoring the current consumption on the motor of the robot arm, abnormal current surges (increases in the force on the motors) can be detected (*e.g.* due to an external force or obstacle) and movement of the RA can be interrupted.

The RA (400, 400a, 400b) may be integrated in a knee support (450) configured to support the knees of the subject lying in a semi-supine position on the radiotherapy treatment table (512) or simulation table (522). An exemplary knee support (450) is depicted in **FIGs. 39** to **41****,** **46** to **48**. The knee support (450) comprises a body (451) having a base end (453) for placement on the table (512, 522), an opposing supporting side (457) configured for supporting posterior of each knee of the subject lying in the semi-supine position.

At the supporting side (457), the body (451) is formed into two knee rests (466, 468), one supporting each knee posterior. Each knee rest (466, 468) comprises an apex part (455a, 455b) that each supports a knee under the joint and raises it upwards. The apex part (455a, 455b) runs laterally with respect to the radiotherapy treatment table (512) or simulation table (522). The apex part (455a, 455b) may be rounded in a longitudinal direction and/or lateral direction *e.g.* it may be concave to match a form of the knee posterior. Each knee rest (466, 468) further comprises a pair of descending guides (460a, 462a; 460b, 462b), each descending guide extending longitudinally from each apex part (455a, 455b) in a superior (460a, 460b) or inferior direction (462a, 462b) towards the base end (453). Each descending guide (460a, 462a; 460b, 462b) is configured to support the leg of the subject superior and posterior to the knee.

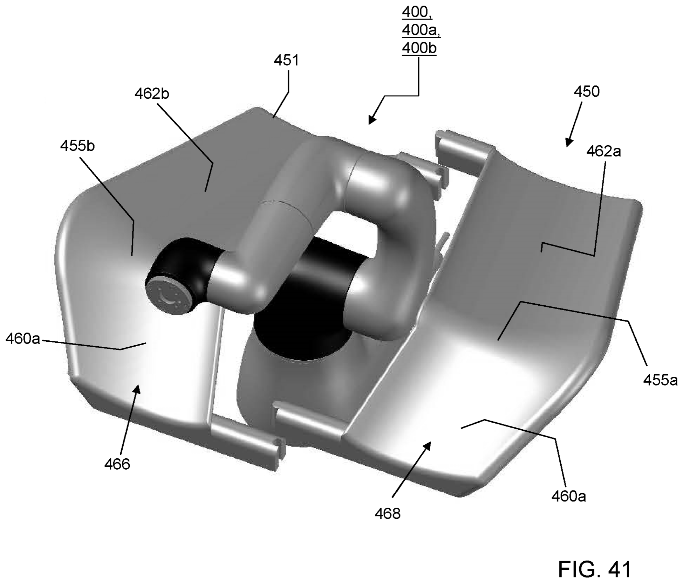

The height of each apex part (455a, 455b) *i.e.* the distance between each apex part (455a, 455b) and the base end (453) may be the same or different. Where the height of each apex part (455a, 455b) is the same, the apex parts (455a, 455b) raise both knees to an equal height; this is illustrated for instance in **FIGs. 39** and **40****,** **46 to 48****.** Where the height of each apex part (455a, 455b) is different, the apex parts (455a, 455b) raise both knees to an unequal height; this is illustrated for instance in **FIG. 41****.** An unequal height can be implemented when a part of the robotic arm would otherwise cross an apex part (455a, 455b).

The knee support (450) is repeatably dismountably attachable to the radiotherapy treatment table (512) or simulation table (522) at the same position; this may be achieved by mounting directly or using a rigid frame using one or more couplings standardly provided on a radiotherapy treatment table (512) or simulation table (522). Hence, the knee support (450) is attachable in fixed (non-adjustable) relation to the radiotherapy treatment table (512) or simulation table (522). The knee support 450 may be provided with a rigid base (452) for repeatable dismountable attachable to the radiotherapy treatment table (512) or simulation table (522).

The base support (432, 432a, 432b) of the RA may be disposed in the knee support (450) in particular in the body (451). The base support (432, 432a, 432b) of the RA may be disposed in fixed relation to the knee support (450) or body (451).

The base support (432, 432a, 432b) of the RA may be slidable and its position lockable relative to the knee support (450). The base support (432, 432a, 432b) of the RA is slidable relative to the knee support (450), in particular to the rigid base (452) of the knee support (450). As such, the base support (432, 432a, 432b) of the RA is slidable relative to a longitudinal axis of the radiotherapy treatment table (512) or simulation table (522).

The position of the base support (432, 432a, 432b) of the RA relative to the knee support (450) may be indexed. In other words, the base support (432, 432a, 432b) may be disposed and lockable at two or more discrete positions relative to the knee support.

The knee support (450) may be provided with a slot (454) into which the base support (432, 432a, 432b) of the RA can lockably slide. The slot may be disposed in a longitudinal direction. The slot (454) may be disposed between both knee rests (466, 468). Accordingly, when the patient is lying on the radiotherapy treatment table (512) or simulation table (522) with knees supported by the knee support (450), the position of the RA can be adjusted to match the length of the subject's femur.

The knee support (450) may be provided with a rail onto which the base support (432, 432a, 432b) of the RA is slidably mounted. The rail may be fixed to the rigid base (452) of the knee support (450).

The knee support (450) body (451) may house a battery pack configured to electrically power the robotic arm (400). The battery pack is preferably rated to provide sufficient electrical power for at least one session. The battery pack is preferably rechargeable. The battery pack allows the parts of the system in contact with the table (512, 522) to function with fewer or without electrical or data cables. The reduction in cables makes the setting up easier, and/or is more compliant with safety and regulatory practices. The knee support (450) body (451) may house the processing unit (440).

The knee support (450) may be formed from 1 part or from 2 or more parts (450,a; 450,b). In particular, the knee support (450) body (451) may be formed from 1 part or from 2 or more parts (451,a; 451,b). The 2 or more parts connect together to form the knee support (450) or body (451). An example of a 2 part knee support is shown in **FIGS. 41****,** **46** to **48.** The parts (not labelled) shown in **FIGs. 46** to **48** correspond to the parts shown in **FIGs. 39** to **41** including, for example, two knee rests (466, 468) and apex parts (455a, 455b). The knee support (450) may be formed from 2 or more parts allowing for easier carrying and transport, for instance, to partition the total weight of the knee support (450).

One part of the knee support (450,a; 451,a) may contain the two knee rests (466, 468), and the apex part (455a, 455b) (*e.g.* **FIG. 46****).** It may also contain a battery pack. It may also contain a processing unit. Another part of the knee support (450,b; 451,b) may contain the robotic arm (*e.g.* the base support (432, 432a, 432b) of the RA) (*e.g.* **FIG. 47****).** It may also contain a processing unit. It may have a pair of wheels (459) for easier mobility.

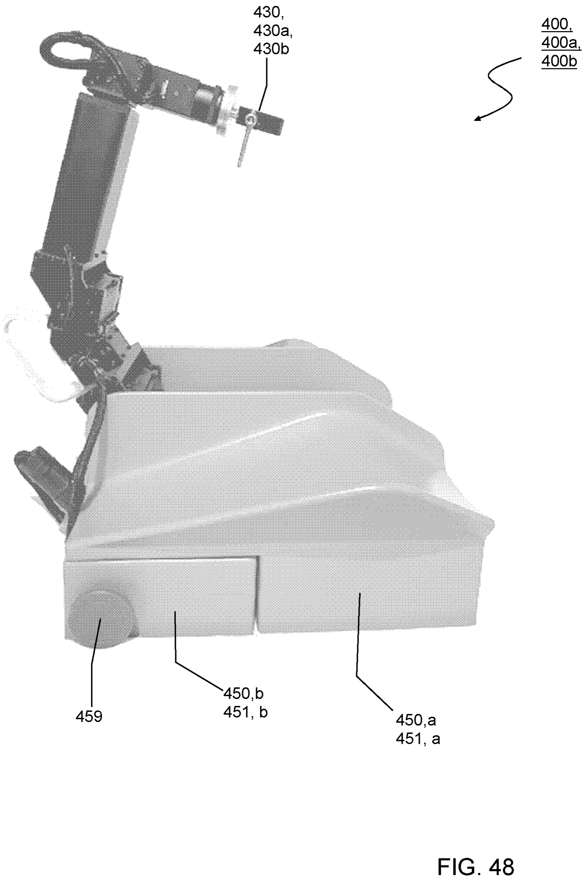

Both parts join together to form the complete robotic arm (400, 400a, 400b) (*e.g.* **FIG. 48****).** The parts (450,a; 450,b; 451,a; 451,b) may be dismountably attachable to each other. The dismountable attachment may be position-repeatable. The attachment may be achieved by means of a clip, a screw, a bolts, a pin, and other fixation elements. One or more connectors for transmission of power and/or electrical signals may be disposed on the respective parts (450,a; 450,b; 451,a; 451,b), that align and engage when the parts are mutually attached.

One or both lateral sides (456, 458) of the knee support (450) may be markable (*e.g.* by a marker pen) or be pre-disposed with a reference marking (464, 465) to indicate a position where a projected laser reference line (516, 526) crosses the one or both two lateral sides (456, 458). The reference marking may be a gauge (464) or an alignment indicator (465) (such as a line or cross).

One or both two lateral side (456, 458) may be marked with, or
the reference marking (gauge (464)) may be read where, or
the reference marking (alignment indicator (465)) may be aligned with
   the position where the projected laser reference line crosses the one or both two lateral sides (456, 458) while the treatment or simulation table (512, 522) is in a set up (withdrawn) position.

At least one projected laser reference lines (526) described earlier, whose position is known with respect to the intersection volume or isocentre or simulation thereof, may cross one or both two lateral sides (456, 458) of the knee support (450).

By
- marking (464) or noting from the reference marking (gauge (464)) the position of the least one projected laser reference lines (526) on the one or both two lateral sides (456, 458) while the knee support (450) is on the simulation table (522),
   or
- by aligning the reference marking (alignment indicator (465))) on one or both two lateral sides (456, 458) of knee support (450) on the simulation table (522) with the least one projected laser reference lines (526),
the same position of the knee support (450) on the radiotherapy treatment table (512) can be reproducing by lining up the marking, or reference marking (464, 465) on the one or two lateral sides (456, 458) with the least one projected laser reference lines (516) on the radiotherapy treatment table (512).

The same projected laser reference line (526) crossing a lateral side (456, 458) of the knee support (450) also crosses the lateral side of the subject (*e.g.* leg or knee). Hence, the tattoo marking that is placed on the subject (*e.g.* on the leg or knee) while on the simulation table (522) may indicate the position of the projected laser reference line (526) that also crosses a lateral side (456, 458) of the knee support (450) at the marking or reference marking (464, 465). The tattoo marking may be a line or a series of lines.

The same tattoo marking, while the subject is placed on the radiotherapy treatment table (512), is aligned with the corresponding projected laser reference line (516) in the treatment room. The same projected laser reference line (516) is also used to correctly position the knee support (450) by aligning the marking or reference marking (464, 465) on a lateral side (456, 458) of knee support (450) with the projected laser reference line (516).

The knee support (450) forces the subject into the semi supine position; the bent legs form a natural shape which co-operates with knee support (450) and locates the subject at the same position on the radiotherapy treatment table (512) at each treatment session. The tattoo on the side of the leg further refines the position of the subjection on the radiotherapy treatment table (512).

According to one aspect, a radiotherapy treatment system (100) as described herein is provided, comprising a knee support (450) configured to support the knees of the subject lying in a semi-supine position on the radiotherapy treatment table (512) or simulation table (522), wherein the knee support (450):
- comprises a body (451) having a base end (453) and an opposing apex edge (455), which body is configured to abut both posterior side of both legs at the knees of the subject on the radiotherapy treatment table (512) or simulation table (522),
- is configured for dismountable attachment to and in fixed relation to the radiotherapy treatment table (512) or simulation table (522),
and the body (451) comprises a slot (454) into which a base support (432, 432a, 432b) of the RA can lockably slide in one to more positions defined in relation to the radiotherapy treatment table (512) or simulation table (522). One or both lateral sides (456, 458) of the knee support (450) may be markable or be pre-disposed with a reference marking (464, 465) to indicate a position where a projected laser reference line (516, 526) crosses the one or both two lateral sides (456, 458).

The system (100) or method described herein may employ one or two RAs. One RA (400, 400a, 400b) may be provided configured for repeatable dismountable attachment in relation to each of the treatment table (512) and the simulation table (522). The same RA may be portable, and may be moved between the simulation room and treatment room, attached to the relevant table, and configured according to the function. Alternatively, two RAs (400a, 400b) may be provided, a treatment RA, TRA, **(****FIG. 5****,** 400, 400a), configured for repeatable dismountable attachment in relation the radiotherapy treatment table (512), and a separate simulation RA, SRA, **(****FIG. 6****,** 400, 400b) configured for repeatable dismountable attachment in relation the simulation table (522). Thus, two separate RAs may be provided, one (SRA (400b)) performing tasks related to simulation, and the other (TRA (400a)) performing tasks related to treatment.

The radiotherapy treatment system (100) may be provided, wherein:
- one RA (400, 400a, 400b) is provided configured for repeatable dismountable attachment to each of the treatment table (512) and the simulation table (522), or
- two RAs (400a, 400b) are provided,
   - a treatment RA, TRA, (400, 400a), configured for repeatable dismountable attachment to the treatment table (512), and
   - a simulation RA, SRA, (400, 400b) configured for repeatable dismountable attachment to the simulation table (522).

The system (100) further comprises processing unit (440) comprising a processor and a memory. The processing unit may be implemented in a computing device such as a desktop PC, laptop, dedicated programmable controller, as a collection of connected computing devices, as a collection of computing devices. The processing unit may be provided in part or entirely by a processor and a memory disposed within the RA, or radiotherapy device (510), or medical imaging device (520). The processing unit may be configured for performing one of more of the methods, or parts thereof, as described herein

The processing unit (440) is configured to control movement of the RA (400, 400a, 400b). The processing unit (440) may configured to control movement of the positioning tool (200) in accordance with the simulation and treatment tasks described herein. It may be configured for positioning of the positioning tool (200) at the same or similar one or more treatment poses relative to the positional reference (e.g. subject (50) and/or radiotherapy treatment table (512)) during at least 1 or 2 and preferably all of the treatment sessions.

The effector end (424) is provided with an RA fitting (430, 430a, 430b) for dismountable attachment of the positioning tool (200) steering guide (300) to the last (distal-most) link of the RA. Typically, the link of the last joint (426-7) of the RA (400, 400a, 400b) is attached to the RA fitting (430, 430a, 430b). The RA fitting (430, 430a, 430b) may be a standard fitting such as already provided by the robotic arm, or may be customised according to the parameters of the positioning tool (200). The RA fitting (430, 430a, 430b) may comprise a gripper e.g. a set of jaws, chuck. The gripper clamps one end of the positioning tool (200).

The RA (400, 400a, 400b), in particular the RA fitting (430, 430a, 430b) may automatically dock with the positioning tool (200), using for instance one or more optical location markers attached to the positioning tool (200) and an imaging sensor. The one or more optical location markers may be reflective markers, or markers having a particular shape or form. The "handle" of the positioning tool may have a particular shape which can be recognized by the camera. The imaging sensor may be incorporated into a camera including a lens assembly. The imaging sensor may be attached in relation to the RA fitting, for instance, in fixed relation. The processing unit (440) may be configured to control movements of the RA fitting (430, 430a, 430b) responsive to signals received from the imaging sensor for repeatable docking of the RA fitting (430, 430a, 430b) to the positioning tool (200).

The RA fitting (430, 430a, 430b) may comprise at least one force sensor (e.g. a 6-DOF force sensor), configured to measure force applied by the positioning tool (200) on the subject (50). This allows a limit on the quantity of force that may be applied by positioning tool (200) on the subject (50). The processing unit (440) may be configured to limit the force applied by the RA (400, 400a, 400b) *via* the positioning device (200) on the subject (50) responsive to signals received from the at least one force transducer, preferably to not more than 100 g to 1000 g force (0.980665 N to 9.80665 N), more preferably to not more than 700 g (6.864655 N).

The RA fitting (430, 430a, 430b) may comprise at least one linkage fuse. A linkage fuse is a breakable connection disposed between the last link of the robotic arm and the RA fitting (430, 430a, 430b). The linkage fuse tolerates a working load without breaking, and breaks with an application of an abusive load, releasing mechanical connection between the RA fitting and the remainder of the robotic arm. An abusive load may arise, for example, if the subject falls from the table (earthquake). The linkage fuse may be one-time breakable or repeatably-breakable (resettable). Examples of linkage fuses include magnetic, electro-magnetic, frangible connection, latch (*e.g.* press-stud). The linkage fuse may be broken by an emergency button accessible to the user and/or subject. Such may be achieved where the connection is electro-magnetic, or using a Bowden cable to open a latch, or using a small explosive charge.

The base end (422, 422a, 422b) of the RA (400, 400a, 400b) may be mountable in fixed relation to the radiotherapy treatment table (512) or to the simulation table (522). By fixed, it is meant that the position and orientation of the base end (422, 422a, 422b) does not change relative to the radiotherapy treatment table (512) or to the simulation table (522) for a duration it is mounted. The position on the radiotherapy treatment table (512) or to the simulation table (522) may be the same or different between sessions. The base end (422, 422a, 422b) may be mountable on the radiotherapy treatment table (512) or simulation table (522), or to a support disposed in relation to the radiotherapy treatment table (512) or simulation table (522), for instance, to an extension piece. The RA (400, 400a, 400b) may be mountable using a dismountable attachment or using a permanent attachment. The dismountable attachment may be realised using, for instance, a clamp, one or more screw-fittings, one or clip screw-fittings, a push fitting, and the like. At the base end (422, 422a, 422b) the first joint (426-1) may be attached to a base support (432, 432a, 432b); the base support (432) may be mountable in fixed relation to the radiotherapy treatment table (512) or to the simulation table (522) as exemplified in **FIG. 5****.**

It is within the scope of the invention that the base end (422, 422a, 422b) of the RA (400, 400a, 400b) is permanently attached to the radiotherapy treatment table (512) or to the simulation table (522). Typically, a permanent fixing is non-releasable e.g. using non-releasable bolts.

The base end (422, 422a, 422b) of the RA (400, 400a, 400b) may be mounted or mountable in relation to the radiotherapy treatment table (512) or to the simulation table (522) between the lower limbs of the subject. It may be mounted or mountable at a position corresponding to between the feet, between the ankles, between the crus, between the knees or between the thighs. The base end (422, 422a, 422b) of the RA (400, 400a, 400b) may integrated inside the cushion located or locatable under the knees of the subject (Civco, QFix, etc. cushions).

The simulation table (522) is steerable, allowing movement between a "set-up position" and an "acquisition position". In the set-up position, simulation table (522) is withdrawn from the medical imaging device (520) so the subject can mount the simulation table (522) and be placed in a comfortable position. In the acquisition position the simulation table (522) is moved into the medical imaging device (520)for image acquisition. The position of the simulation table (522) relative to the simulated beam intersection volume or isocentre is known.

A positional relationship (SPx,y,z) of the RA base end (422, 422b) or base support (432, 432b) with respect to the positional reference *e.g.* simulation table (522) may be known. The positional relationship may be determined using a position measurement gauge (524) such as a marked grid or ruler provided (*e.g.* attached to or drawn) on the simulation table (522). The positional relationship may be determining using an overseeing camera that observes both the simulation table (522) and the RA base end (422, 422b) or base support (432, 432b). Since the position of the RA base end (422, 422b) or base support (432, 432b) with respect to the positional reference *e.g.* simulation table (522) is known, the optimal pose of the positioning tool (200) as controlled by the RA (400, 400a) may be determined clinically and by imaging, and its exact position in relation to the RA base end (422, 422b) or base support (432, 432b) and to the simulation table (522) can be calculated. The RA base end (422, 422b) or base support (432, 432b) may be attached to the simulation table (522) at the same position or at a different position prior to imaging where simulation is done more than once ; the position measurement gauge (544) and/or overseeing camera allows the position to be determined.

The medical imaging device (520) is typically disposed in a room (see **FIG. 6****)** having a position controllable simulation table (522) on which the subject is accurately aligned (see later below) that introduces into or withdraws from the subject a measurement volume of the medical imaging device (520). The medical imaging device (520) is typically a computed tomography scanner that generates a three-dimensional image containing the bodily tissue for radiotherapy.

The positioning tool (200) may be moved (by the specialist) during simulation such that the canal is displaced from its natural position when the subject is lying on the simulation table. Movement of the canal by the specialist also causes movement of bodily tissue of the subject in relation to the simulated external ionising radiotherapy beam. The bodily tissue (608) may be tissue that is a target of treatment (*e.g.* a tumour) and is to be brought into the simulated external ionising radiotherapy beam for exposure to the beam. Alternatively, the bodily tissue may be tissue that is to be moved out of the simulated external ionising radiotherapy beam thereby avoiding exposure to the beam; accordingly, healthy tissue can be displaced away from a target, to reduce side effects.

For instance, the positioning tool may be lifted upwards (towards the "ceiling") in order to distance the target prostate from the rectal wall which is more sensitive to radiation. Alternatively, the positioning tool may pull the target downwards (to the "floor") in order to distance it from the bladder and uterus. This more optimal position may be reproduced for the treatment by the positioning tool at each treatment session *i.e.* it is a more refined treatment pose.

Hence, during simulation, the processing unit (440) may configured to position the positioning tool (200) responsive to an input (of the specialist) and to record one or more simulation poses relative to the positional reference (*e.g.* to subject (50) on the simulation table (522)) as the one or more empirically-determined simulation poses.

According to one embodiment:
- the position of the bony pelvis of the subject on the simulation table (552) relative to the positional reference (*e.g.* simulated beam intersection volume or isocentre) is known. This is usually known by precisely aligning the subject on the simulation table (552) as described elsewhere herein;
- the position of the base end (422, 422b) of the RA mounted on the simulation table (522) is known. This is usually known by using a repeatable mount, or a measurement gauge (524) as mentioned elsewhere. The position can be ultimately referenced to the positional reference (*e.g.* simulated beam intersection volume or isocentre);
- the pose of the positioning tool in relation to the bony pelvis of the subject is known. This is usually known because encoders in the RA can be used to determine the pose of the positioning tool in relation to the RA base end (422, 422b);
- the position of the RA base end (422, 422b) in relation to the bony pelvis of the subject are known. This is known because the position of the base end (422, 422b) of the RA and of the bony pelvis of the subject relative is known.

From the known positional relation of the bony pelvis to the positioning tool, the known pose of the positioning tool in relation to the RA base end, and the known positional relation of the RA base end to the bony pelvis, the pose of the positioning tool that fixes the bodily tissue of the subject in relation to the simulated beam intersection volume or isocentre during simulation can be accurately determined. This information can be recorded and used during treatment on the radiotherapy treatment table (512).

The radiotherapy treatment table (512) is steerable, allowing movement between a "set-up position and a "treatment position". In the set-up position, the radiotherapy treatment table (512) is withdrawn from the treatment beam area so the subject can mount the radiotherapy treatment table (512) with ease and be moved accurately into position. In the treatment position the treatment table (512) is moved into the intersection volume or isocentre for treatment. The position of the treatment table (512) relative to the beam intersection volume or isocentre is known.

A positional relationship (TPx,y,z) of the RA base end (422, 422a) or base support (432, 432b) with respect to the positional reference *e.g.* radiotherapy treatment table (512) may be known. The positional relationship may be determining using a position measurement gauge (514) such as a marked grid or ruler provided (*e.g.* attached to or drawn on) the radiotherapy treatment table (512). The positional relationship may be determining using an overseeing camera that observes both the radiotherapy treatment table (512) and the RA base end (422, 422a) or base support (432, 432a). Since the position of the RA base end (422, 422a) or base support (432, 432a) with respect to the positional reference *e.g.* radiotherapy treatment table (512) is known, the optimal pose of the positioning tool (200) as controlled by the RA (400, 400a) can be calculated from stored simulation data. The RA base end (422, 422a) or base support (432, 432a) may be attached to the radiotherapy treatment table (522) at the same position or at different position per fractioned treatment session; the position measurement gauge (544) and/or overseeing camera allows the position to be determined.

The radiotherapy device (510) is typically disposed in a room (see **FIG. 5****)** having a position-controllable radiotherapy treatment table (512) on which the subject is accurately aligned (see later below) that introduces the subject into or withdraws the subject from a treatment volume of a ionising-radiation treatment head (518). The ionising-radiation treatment head (518) moves (*e.g.* rotates or moves on a robotic arm) around the target tissue while delivering ionising radiation, more in particular around the beam intersection volume (isocentre) for radiotherapy. A revolute axis of the ionising-radiation treatment head coincides with the beam intersection volume, thereby irradiating the beam intersection volume at all angles of rotation. The target for treatment is aligned with the beam intersection volume, and receives a majority of the dose while healthy tissue receives a momentary exposure during beam rotation. The radiotherapy device (510) is typically a linear accelerator optionally provided with a collimator for dynamic beam shaping (conformal radiotherapy), or a tomotherapy machine, or any other radiotherapy device allowing for conformal radiotherapy.

From both
- the positional relationship (SPx,y,z) of the RA base end (422, 422b) or base support (432, 432b) with respect to the positional reference (*e.g.* simulation table (522)), and
- the positional relationship (TPx,y,z) of the RA base end (422, 422b) or base support (432, 432b) with respect to the positional reference (*e.g.* radiotherapy treatment table (512)),
the pose of the positioning tool (200) on the simulation table (522) may be reproduced by the RA (400, 400a) mounted on the radiotherapy treatment table (512).

Combined with the aforementioned positioning of RA base end or support (422, 422b, 432, 432b), when the patient is placed in the same position, or an exactly known position on both the simulation table (522) and the radiotherapy treatment table (512), a pose of the positioning tool (200) inserted into a canal of the subject (50) on the simulation table (522) can be precisely reproduced on radiotherapy treatment table (512).

According to one embodiment:
- the position of the bony pelvis of the subject on the radiotherapy treatment table (512) relative to the positional reference (*e.g.* beam intersection volume or isocentre) is known. This is usually known by precisely aligning the subject on the radiotherapy treatment table (512) as described elsewhere herein;
- the position of the base end (422, 422a) of the RA mounted on the radiotherapy treatment table (512) is known. This is usually known by using a repeatable mount, or a measurement gauge (514) as mentioned elsewhere. The position can be ultimately referenced to the positional reference (*e.g.* simulated beam intersection volume or isocentre);
- the pose of the positioning tool in relation to the bony pelvis of the subject on the radiotherapy treatment table (512) is known. This is usually known because encoders in the RA can be used to determine the pose of the positioning tool in relation to the RA base end (422, 422a);
- the position of the RA base end (422, 422a) in relation to the bony pelvis of the subject on the radiotherapy treatment table (512) is known. This is known because the position of the base end (422, 422b) of the RA and of the bony pelvis of the subject relativeis known.

From the known positional relation of the bony pelvis to the positioning tool, the known pose of the positioning tool in relation to the RA base end, and the known positional relation of the RA base end to the bony pelvis, the pose of the positioning tool that fixes the bodily tissue of the subject in relation to the simulated beam intersection volume or isocentre during simulation can be accurately reproduced on the radiotherapy treatment table (512). When these positions are exactly known, it all makes the placement of the patient and of the system much faster and more reproducible for each radiotherapy session. Once the patient is placement in the right position, and once the positioning tool is moved by the RA in the predetermined position, some fine tuning may be performed, for instance, with transponders and/or imaging.

The subject may be placed at the same position or in a known position on both the simulation table (522) and the radiotherapy treatment table (512). The position of the subject on the table is known with respect to the positional reference. The position reference may ultimately be the simulation of a beam intersection volume or the simulation of an isocentre during simulation, or may ultimately be the beam intersection volume, the isocentre during treatment. In practice, the positional reference may be, for instance, a gauge provided on the simulation table (522) and the radiotherapy treatment table (512) whose position is known relative to the intersection volume or isocentre or simulation thereof.

More usually, the positional reference may be one preferably two or three projected laser reference lines (516, 526) who positions are known with respect to the intersection volume or isocentre or simulation thereof. The project laser reference lines (516, 526) are projected by lasers fixed to the wall and/or ceiling of the simulation room and treatment room.

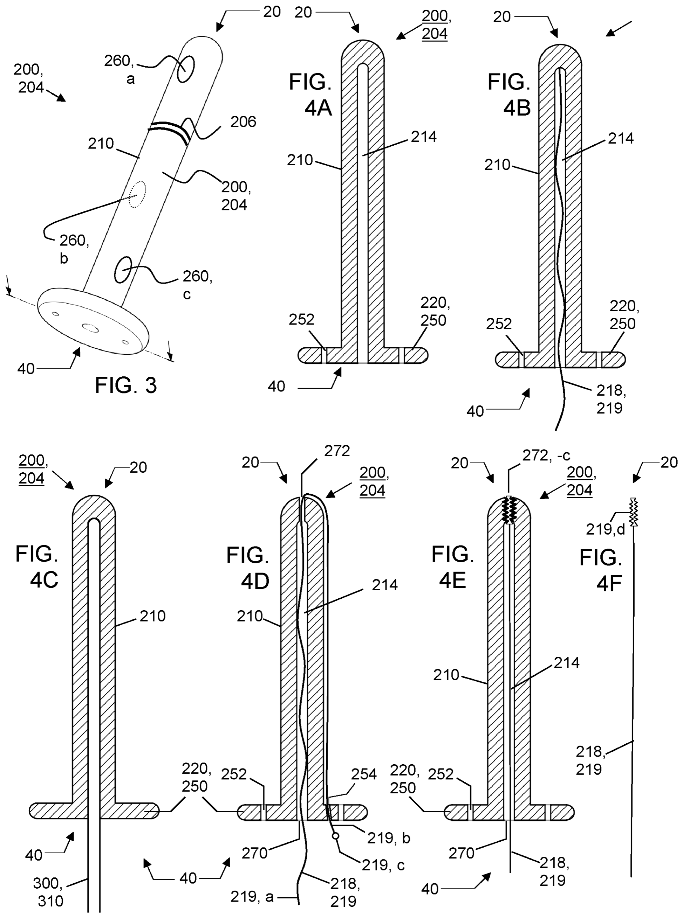

The subject may be placed at the same position or in a known position on both the simulation table (522) and the radiotherapy treatment table (512) by aligning the subject, in particular at least one tattoo marking thereon, with the at least one preferably two or three projected laser reference lines (516, 526). One set of projected reference lines **(****FIG. 5****,** 526) is projected at an exact position onto the simulation table (522). Another set of projected reference lines **(****FIG. 4****,** 516) is projected at an exact position onto the radiotherapy treatment table (512). The positions and directions of the projected reference lines are also known with respect to the positional reference, which is typically the beam intersection volume (*e.g.* isocentre) of simulation thereof. The position of the medical imaging device (520) is known with respect to the positional reference. By aligning the subject (50) with the at least one projected laser reference line (526) the subject (50) is positioned precisely on the simulation table (522) and in known relation to the RA base (434b). Similarly, the position of the radiotherapy treatment table (512) is known with respect to the positional reference, which is typically the beam intersection volume (*e.g.* isocentre). By aligning the subject (50) with the at least one projected laser reference line (516) the subject (50) is positioned precisely on the radiotherapy treatment table (512) and in known relation to the RA base (434a) and to the beam intersection volume (*e.g.* isocentre).

Both the simulation table (522) and the radiotherapy treatment table (512) are typically identical in shape, and the arrangement of projected laser reference lines (516, 526) is also identical in both simulation room and radiotherapy treatment room. The positional reference (isocentre or simulation thereof) is the also the same in both rooms. Accordingly, the subject accurately positioned with respect to a projected laser reference line (526) on the simulation table (522) can be reproducibly positioned respect to a projected laser reference line (526) on the treatment table (512).

Most modern external radiotherapy machines have a built-in medical imaging unit that can take (*e.g.* X-ray, low quality CT scan performed) images of the subject on same table used to treat the patient. This allows, among other things, to ensure a fine-tuning of the position of the bodily tissue with respect to beam intersection volume of the ionising radiation beam emitted by the radiotherapy device. After a first positioning the subject using projected laser reference lines (516, 526) that cross one or more tattoos, a second more accurate positioning may be carried using the built-in medical imaging unit; it is usually the bony structures (e.g. pelvis) or implanted radio-opaque markers that are accurately visualized and used to precisely position the subject on the treatment table. The subject will always be placed in a reproducible position on both treatment and simulation tables. This positioning of subject, however, does not take into account the displacement of internal structures.

The simulation table (522) is steerable, allowing movement between the set-up position and the acquisition position described earlier. In the set-up position, wherein simulation table (522) is withdrawn from the image detector so the subject can mount the simulation table (522), at least one projected laser reference line (526) is projected onto the simulation table (522) from a fixed position in relation to the wall and/or ceiling of the room containing the simulation table (522). Once the subject (50) is comfortable on the simulation table (522) in the set-up position, the position of the subject relative to the at least one projected laser reference line is marked by applying one or more tattoo marks on the subject (50); the tattoos allow the subject positioned be reproducibly on the treatment table (512).

The simulation table (522) may be provided with a measurement gauge (524) that may be visual such as a marked grid or ruler provided (*e.g.* attached to or drawn on) the simulation table (522). Preferably, the measurement gauge (524) (*e.g.* ruler) is disposed in relation to a long edge of the simulation table (522). Preferably it is attached or drawn in fixed relation to the long edge of the simulation table (522). The measurement gauge (524) is positioned so that it crosses at least one projected laser reference line (526) and extends towards the expected position of an RA base end (422, 422b) or base support (432, 432b) of the RA (400, 400b) on the simulation table (522). The position of the measurement gauge (524) ("gauge position") relative to the simulation table (522) is recorded. The position indicated by the measurement gauge (524) ("laser position") where the at least one projected laser reference line (526) crosses it is also recorded.

Then the RA (400b) is placed on the simulation table (522). Preferably it is positioned preferably such that a revolute axis of the RA base end (422, 422b) crosses the longitudinal midline of the simulation table (522). It is also placed such that an RA fitting (430, 430b) of the RA (400, 400b) is positioned for dismountable attachment to the positioning tool (200) for simulation of treatment. Then the position indicated by the measurement gauge (524) of the RA base end (422, 422b) or base support (432, 432b) ("base position") on the simulation table (522) is recorded. Subsequently the simulation table (522) is steered into the detector volume and into the "acquisition" position, in which the medical images are recorded for the simulation.

The recorded "gauge position", "laser position", and "base position" are transferred to the radiotherapy treatment table (512) disposed with an identical measurement gauge (514) to ensure that the subject (50) is always at the same position on the radiotherapy treatment table (512) relative to the RA base end (422, 422a) and/or to ensure that the distance between subject (50) and RA base end (422, 422a) is exactly known.

The radiotherapy treatment table (512) is steerable, allowing movement between the set up position" and the treatment position described earlier. In the set-up position, wherein the radiotherapy treatment table (512) is withdrawn from the treatment beam area so the subject can mount the radiotherapy treatment table (512) with ease and be moved accurately into position, at least one projected laser reference line (516) projected onto the radiotherapy treatment table (512) from a fixed position in relation to the wall and/or ceiling of the treatment room. The projected laser reference line allows the subject to be placed more accurately on the treatment table. The laser lines cross one or more tattoo marks already applied the subject (50) during simulation. Once the subject (50) is aligned with the at least one projected laser reference line (516), the radiotherapy treatment table (512) is steered into the source volume and into the treatment position.

Prior to radiotherapy treatment, the radiotherapy treatment table (512) is moved to the set-up position. The radiotherapy treatment table (512) may be provided with a position measurement gauge (514) that may be visual such as a marked grid or ruler provided (*e.g.* attached to or drawn on) the radiotherapy treatment table (512). Preferably, the position measurement gauge (514) (*e.g.* ruler) is disposed in relation to a long edge of the radiotherapy treatment table (512). Preferably it is attached or drawn in fixed relation to the long edge of the radiotherapy treatment table (512). The measurement gauge (514) is preferably same or identical to the measurement gauge (524) attached the simulation table (514). The measurement gauge (514) is preferably positioned on the radiotherapy treatment table (512) at the same "gauge position" recorded at simulation table (514). The longitudinal position of the treatment table (512) is adjusted in the set-up position such that the at least one projected laser reference line (526) crosses the measurement gauge (514) on the treatment table (512) at the "laser position" recorded at simulation table (514).

The subject may be placed such that a midline of the subject aligned with a longitudinal midline of the radiotherapy treatment table (512) using laser beams. The subject (50) may be moved such that that tattoo marks (52) on the subject (50) are aligned with the at least one projected laser reference line (516). The RA (400a) is placed such that a revolute axis of the RA base end (422, 422a) crosses the longitudinal midline of the radiotherapy treatment table (512). The RA (400, 400a) may be placed on the treatment table (512) such that an RA fitting (430, 430b) of the RA (400, 400a) is positioned for dismountable attachment to the positioning tool (200) for radiotherapy treatment. The position of the RA base end (422, 422a) or base support (432, 432a) is adjusted such that it is equal to the "base position" (recorded on the simulation table) as indicated by the measurement gauge (514) on the treatment table (512).

By using the position measurement gauge (514) on the radiotherapy treatment table (512), the position of the subject can be quickly and simply aligned with both the table and the RA base end (422, 422a) reproducibly, without the need supply additional positional information.

The radiotherapy treatment system (100) described herein may further comprise:
- the radiotherapy treatment table (512), provided with
   - one or more attachment points for repeatable reproducible attachment of the RA (400, 400a), or
   - one or more attachment points for repeatable reproducible attachment of the a knee support (450) as described herein,
      in fixed positional relation the treatment table (512),
      and/or
   - the simulation table (522), provided with:
      - one or more attachment points for repeatable reproducible attachment of the RA (400, 400a), or
      - one or more attachment points for repeatable reproducible attachment of a knee support (450) as described herein,
         in fixed positional relation the simulation table (522),

- optionally wherein the one or more attachment points are positioned on the radiotherapy treatment table (512) and on the simulation table (522) in geometrically-identical positions such that a pose of the RA (400, 400a) in relation to the simulation table (522) is reproducible on the radiotherapy treatment table (512),
- optionally wherein the one or more attachment points on the radiotherapy treatment table (512), and on the simulation table (522) are positioned non-adjustably and identicaly.

The radiotherapy treatment system (100) described herein is provided, wherein:
- the radiotherapy treatment table (512) is disposed with a measurement gauge (514) for determining a position of the RA (400, 400a) base end (422, 422a) with respect to the subject (50), in particular with respect to the subject aligned with at least one preferably two projected laser reference lines,
   and/or
- the simulation table (522) is disposed with a measurement gauge (524) for determining a position of the RA (400, 400b) base end (422, 422b) with respect to the subject (50), in particular with respect to the subject aligned with at least one preferably two projected laser reference lines.

- optionally wherein both measurement gauges (514, 524) have an identical scale.

A pivot point may be assignable to the positioning tool (200) that is a point or region of the positioning tool (200) around which movements of positioning tool (200) are pivoted. Once the positioning tool (200) has been inserted into the canal, the pivot point may be assigned to the positioning tool (200), and movements of the positioning tool (200) become limited to pivoting rotations centred on the pivot point. A pivoting rotation is a yaw or pitch movement. The pivot point does not displace during pivoting rotations. The position of pivot point may be spatially fixed, optionally with respect to the positional reference. The pivot point may be disposed on the effector shaft (310) or on the transmission (314) of the steering guide, or on the inserter (200). The pivot point may be positioned corresponding to an entrance to the canal of the subject. This pivotal point may correspond to the introit of the vagina, or slightly (e.g. 1 to 10 mm) inside the introit of the vagina, or the introit of the anus, or slightly (*e.g.* 1 to 10 mm) inside the introit of the anus. The position of the pivot point may be determined by measuring a distance between a point on the positioning tool (200) and the entrance to the canal of the subject. The positioning tool (200) may be disposed with a scale for reading off a distance for measuring a position of the pivot point. The position of the pivot point may be measured by reading off a scale (*e.g.* graduated) disposed (*e.g.* printed or engraved) on the effector shaft (310), inserter (204) or on the transmission (314). The value of the distance may be determined during simulation, and reproduced during treatment. The controller may be configured to control the robotic arm such that movements of positioning tool (200) are pivoted around the pivot point. The pivot point allows for pose fine tuning, for instance, based on transponders and/or markers on the positioning tool (200). The pivot point allows the steering guide to be moved with less effort exerted by the robotic arm and less pain for the patient because the introit, which is more tonic, if moved significantly, may be painful. It further avoids pushing on the entrance of the canal (*e.g.* vagina or anus) in any direction. The introit position is relatively fixed on the body. The inner part of the vagina, for instance, is more moveable than the introit.

The subject returning for further radiotherapy treatment sessions may be aligned with the position measurement gauge (514) to allow a fast a reproducible set-up relative to the RA base end (422, 422a).

Prior to radiotherapy treatment, the radiotherapy treatment table (512) is moved to the set-up position so that the measurement gauge (514) at the "gauge position" on the radiotherapy treatment table (512) crosses the at least one projected laser reference line (516) at the same place as in the previous radiotherapy treatments ("laser position"). The subject (50) is placed such that a midline of the subject (50) is aligned with a longitudinal midline of the radiotherapy treatment table (512). The subject (50) is further moved on the radiotherapy treatment table (512) until the tattoo marks (52) on the subject (50) are aligned with the at least one projected laser reference line (516) that cross the midline.

Ideally, the RA (400a) is placed such that a revolute axis of the RA base end (422, 422a) crosses the longitudinal midline of the radiotherapy treatment table (512). The RA base end (422, 422a) is further positioned to correspond with the position at the measurement gauge (514) where it was during the previous radiotherapy treatments ("base position"). By using the position measurement gauge (514) on the radiotherapy treatment table (512), the position of the subject can be quickly reproducibly aligned with both the table and the RA base end (422, 422a), without the need supply additional positional information, for instance from an overseeing camera.

With simulation and treatment tables the exact height of the table is known very precisely and may be accurately reproduced for each simulation or treatment session.

As mentioned earlier a medical imager (e.g. X-ray) built into the radiotherapy device (510) allows further precise alignment of the subject as it reveals bony structures such as the pelvis and the positioning tool (200). Knowing the distance between the RA base end (422, 422a) and the subject (50) is useful when the positioning tool is to be accurately positioned using the built-in medical imager. Knowing the distance between the RA base end (422, 422a) and the subject (50) is less important when the positioning tool is equipped with one or more transponders (see below). However, using transponders are not always possible because of technical limitations (obese subjects) or because of the cost of the transponder system. Hence, access to a built-in medical imager (e.g. X-ray) is useful also in order to position the treatment tool accurately inside the subject.

The speed of movement of the robotic arm may be adjustable to allow the subject to accommodate to a change in pose of the positioning tool. It is noted that a tumor is highly painful because it contains hardened tissue, and the radiotherapy causes inflammation and tenderness. Using a robotic arm, motions can be slowed down to provide accommodation time to the subject. Movement of the positioning tool (effector shaft or inserter) may be such a few mm per minute (1-10 mm/min, preferably 1 to 5 mm/min).

Further provided herein is a positioning tool (200). The positioning tool (200) is configured for insertion into a canal of the subject (50). It has a proximal end and a distal end. The distal end is configured for insertion into a canal of the subject (50). The proximal part is configured for attachment to the RA fitting (430, 430a, 430b). Examples of positioning tools (200) are provided in **FIGs. 1****,** **3, 4A to 4E****,** **8****,** **11 to 38****,** **44**.

Movement of the canal by the positioning tool (200) moves the bodily tissue of a subject relative to the ionising radiation beam or positional reference for treatment. The bodily tissue may be in the canal, or may be a structure that moves when the canal is moved (*e.g*. prostate that moves with the rectal canal). The bodily tissue may be positioned in the beam by the position tool (200) in the beam (*e.g.* for treatment), or outside the beam (*e.g.* to reduce damage). A position and/or orientation of the positioning tool is adjustable and fixable.

One or more parts of the positioning tool (200) may be made from any suitable biocompatible material such as medical grade non-ferromagnetic stainless steel, tantalum, titanium, polycarbonate, PEEK, carbon fibre, fibreglass, aluminium (coated).

Usually the treatment is first simulated under a CT scan, or a PET CT scan, or under MRI. Later the treatment may involve acquisition of one or more X-rays in the treatment room. It is preferred that the positioning tool (200) is made from a material compatible with medical imaging, such as with CT or MRI or X-ray. The material may or may not be visible on a medical image.

Where a part of the positioning tool (200) is visible on a medical image, its pose may be determined directly from the medical image of the effector shaft (310). Wherein the treatment is simulated under MRI, the part visible by imaging may be manufactured from a low density material, and from a MR compatible (non-magnetic) material, such as aluminium coated with a layer of biocompatible metal (titanium) or from titanium. For the CT scan simulation, coated aluminium or titanium would also be an advantage, because it allows for less artefacts compared with using a high density metal such as stainless steel. It is as aspect that coated aluminium part is used for simulation and the stainless steel for the treatment. It is as aspect that an aluminium part is used for simulation and for treatment. Most modern imaging devices that are combined with radiotherapy treatment devices give a good visibility of any metal structure such as the effector shaft (310). In this case, the effector shaft may be visible by itself and could not necessarily need the presence of imaging markers to be seen by the imaging device.

Where it a part of the positioning tool is not visible or not sufficiently visible to determine the position and/or orientation it may be may be disposed with one or more imaging markers. This is of assistance when performing images using the imaging tools of the linear accelerator. The positioning tool may be provided with one or more imaging markers that can be identified by a medical image. An imaging marker may be provided in fixed relation to the positioning tool for instance on an inner surface, outer surface or within a body of the effector shaft (310) or inserter (204). An imaging marker may be made from a material different from the positioning tool for instance a heavy metal such as platinum, platinum iridium, tantalum, tungsten, etc.

The positioning tool (200) may comprise a steering guide (300) having a proximal (40) and distal (20) end (*e.g.* **FIGs. 1****,** **10** to **31, 38).**

The steering guide (300) comprises at the proximal end a handle portion (316). The handle portion (316) is configured for attachment to the RA fitting (430, 430a, 430b). The steering guide (300) comprises at the distal end an effector shaft (310). The effector shaft (310) is configured for insertion into the canal of the subject (50), or for attachment to an inserter (204) configured for insertion into the canal of the subject (50).

Where the inserter (204) (described later below) is present, the effector shaft (310) may be permanently or removably (dismountably) attached to the inserter (204). Where the effector shaft (310) is dismountably attached to the inserter (204), the inserter comprises an elongated member lumen (214) that engages with the effector shaft (310). To assist with insertion of the effector shaft (310) into the elongated member lumen (214), the elongated member lumen (214) may be disposed with a guiding strand (218) at least partially within the elongated member lumen (214), and which exits at a proximal end therefrom. The effector shaft (310) of the steering guide (300) may comprise a guiding strand passage (312) that receives the guiding strand (218). The effector shaft (310) guiding strand passage (312) can be threaded through a proximal end of the guiding strand trailing outside the body and guided reliably into the elongated member lumen (214).

The effector shaft (310) may be provided in fixed (position and orientation) relation to the handle portion (316). Accordingly directional and/or positional movements of the handle portion (316) cause corresponding directional and/or positional movements of the effector shaft (310). The effector shaft (310) and the handle portion (316) may be connected by a transmission (314). The transmission may be provided in fixed connection (*i.e.* directional and/or positional) with both the effector shaft (310) and the handle portion (316).

The handle portion (316) is preferably rigid. It is preferably non-flexible. It may be substantially formed of a rigid rod.

The handle portion (316) may have a length (H) of 2 - 50 cm. Preferably it may have a length (H) of 15 to 25 cm for cervix/uterus/anal/vagina application, or preferably of 15 to 40 cm for rectal application (see **FIG. 1****).** In very obese subjects, the handle portion (316) may have a length of up to 40 or 50 cm. The maximum outer diameter may be 0.3 - 3 cm, preferably of 0.5 - 2 cm. The handle portion may adopt an angle beta with respect to the transmission (*e.g.* **FIG. 1****).** The angle beta is measured in a plane formed between the transmission and handle portion. The angle beta is less than 180 deg when the handle and effector shaft are on the same side (cis) of the transmission; the angle beta is greater than 180 deg when the handle and effector shaft are on opposite sides of the transmission (trans). See **Tables 1** and **1a** for preferred dimensions, angles for various medical applications.

The handle portion (316) may be made from any suitable biocompatible material such as medical grade non-ferromagnetic stainless steel, tantalum, titanium, polycarbonate, PEEK, carbon fibre, fibreglass, polyarylamide resin reinforced with fibers (e.g. Ixef (Solvay)), polyphenylsulfone (PPSU), bioceramics such as aluminosilicates, styrene acrylonitrile, bioceramic-polymer materials. The handle portion (316) may be made from the same material as the transmission (314). The handle portion (316) may have the same diameter as the proximal (40) end of the transmission (314).

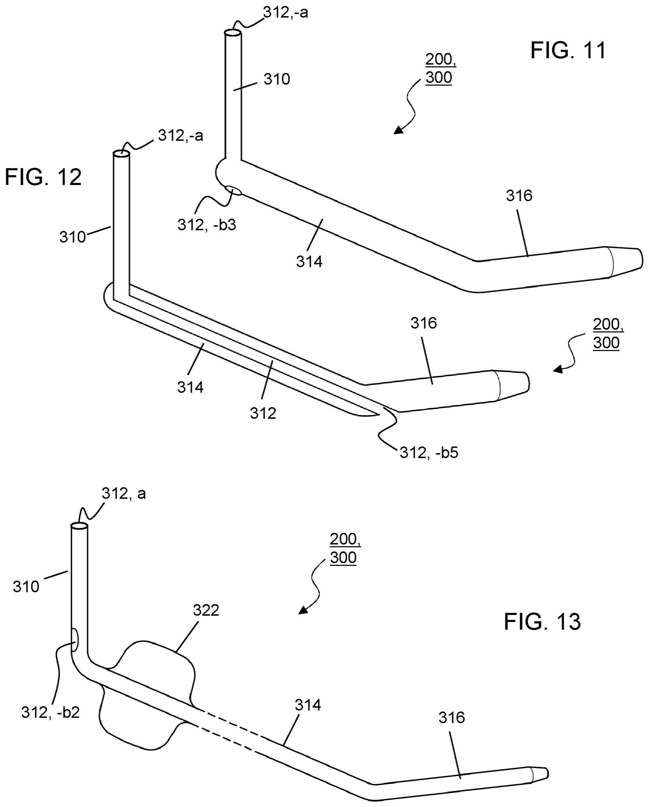

The handle portion (316) may be formed from an imaging-transparent material such as a polymeric rod or tube. The same material may be used to form the transmission (314), for ease of manufacture; this can reduce imaging artefacts caused by the transmission (314) *e.g.* in the vaginal regional (606) - this is described in more detail below. Examples of suitable polymers include polycarbonate. Other materials that may be used for the handle portion are fiberglass, carbon fiber, polyarylamide resin reinforced with fibers (*e.g.* Ixef (Solvay)), polyphenylsulfone (PPSU), bioceramics such as aluminosilicates, styrene acrylonitrile, bioceramic-polymer materials, etc. **FIGs. 11, 12****,** **18** to **28** depict examples of a steering guide (300) formed from a polymeric handle portion (316) and transmission (314) and both having a larger diameter (*e.g*. 0.8 - 1cm) compared with the effector shaft (310) which may be formed from a rigid metal such as titanium or a hard polymer (polycarbonate, PEEK, polyarylamide resin reinforced with fibers).

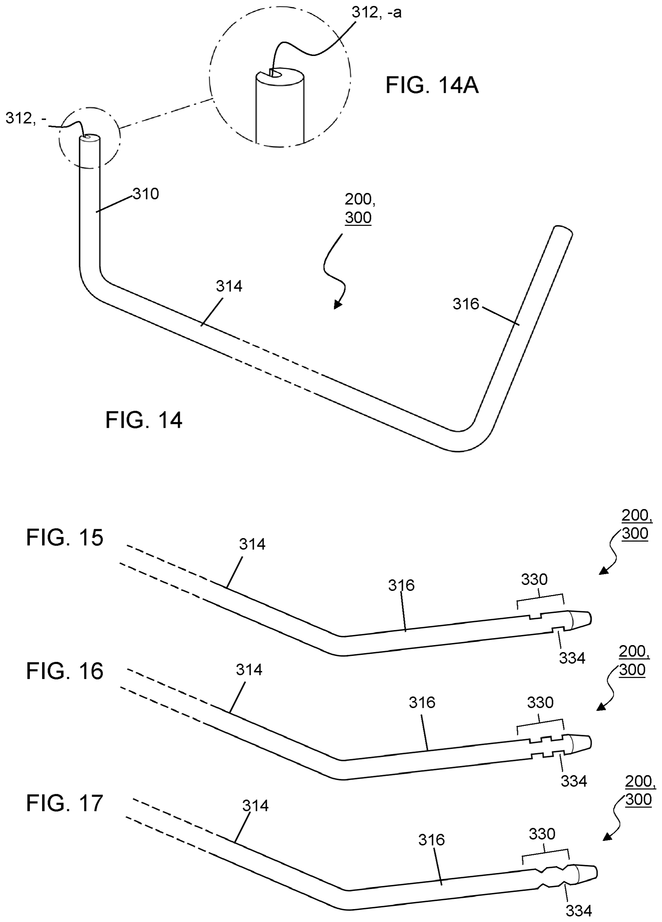

To facilitate attachment to the RA, handle portion (316) may be provided with a grip locator (300) comprising one or more notches (334) and/or one or more protrusions and/or one or more corners (332) that co-operate with an RA fitting (430) that comprises a gripper *e.g*. a set of jaws, chuck. The grip locator (300) becomes seated firmly within the closed gripper, restricting and preventing rotations and/or movements between the gripper and handle portion (316). Exemplary grip locators are illustrated in **FIGs. 15** to **21****,** **24****,** **26****,** **29** to **31**. The grip locator (300) may be disposed at the proximal end of the handle portion (316). The base of the notch (334) may be pointed, flat or linear (*e.g.* a long apex). The notch (334) may have straight (*e.g*. radial) or bevelled sides. The grip locator (330) allows very accurate and reproducible mountable-dismountable attachment of the RA fitting (430) to the handle portion (316). The grip locator allows gripping by the gripper of the handle portion (316) with high positional repeatability and reduced play or backlash. The RA fitting (430) coupled to the grip location (300) attaches the RA fitting (430) in fixed relation with the grip location (300).

The gripper jaws may be provided with one or more protrusions that engage with the grip locator (*e.g*. one or more notches) on the handle portion (316) when the gripper is closed. When the notch has bevelled sides, bevelled protrusions on the gripper bring the steering guide (300) into alignment upon closing.

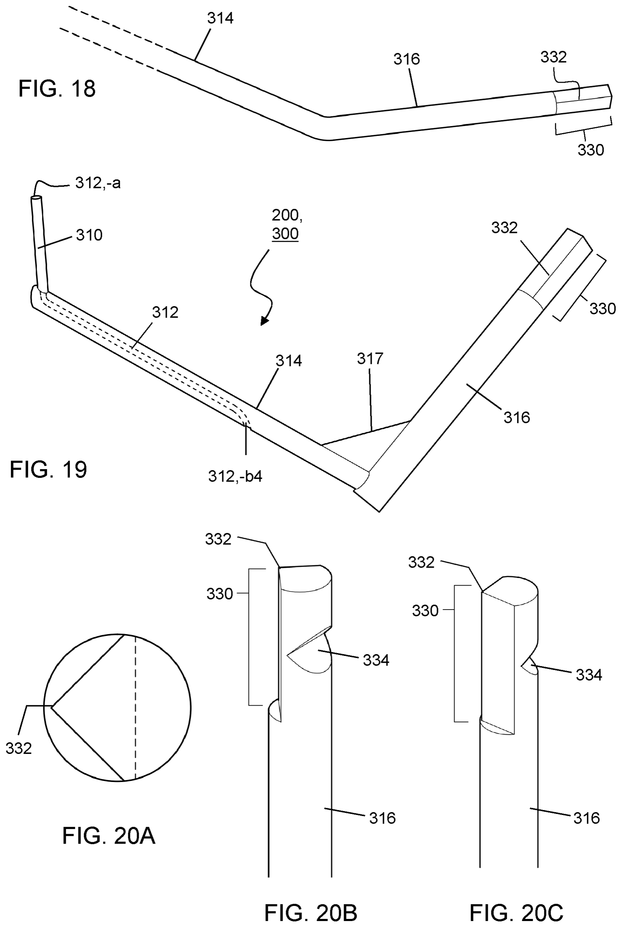

Examples of notches (334) of a grip locator (330) are given in **FIGs. 15** to **17****,** **20A** to **20C****,** **21****,** **24****.** In **FIG. 15** straight-sided notches (334) each with a flat base are provided separated longitudinally and at different radial positions, in **FIG. 16** a proximal set of straight-sided notches each with a flat base is provided having the same longitudinal position but different radial positions and a distal set of notches is provided having a different longitudinal position from the proximal set bit the same different radial positions, and in **FIG. 17** the notches are arranged similarly to **FIG. 16** but they have bevelled sides (V-shaped) and a linear base. In **FIGs. 20** **B** and **C, 21, 24** a bevelled-side (V-shaped) notch (334) is provided having a linear base.

One or more corners (332) of a grip locator (330) may be disposed along an axial direction of the proximal end of the handle portion (316). The corner (332) may be square. There may only be one corner. A grip locator (330) that is a combination of the notch (334) and corner (332) allows for a stable grip by the RA fitting (430) when it is a gripper having a pair of jaws. At least one notch and the corner may have different mutual directions, preferably perpendicular directions. At least one notch may be provided within the longitudinal span of the corner. For instance, the corner may run in an axial direction at proximal end of the handle portion (316) while the base of a notch may run perpendicular to the axial direction; this ensures an absolutely reproducible fixation of the steering guide to the RA fitting and removes an additional uncertainty of steering guide position in relation to RA base end.

**FIG. 20** **B** and **C**, **21, 24** the proximal end of the handle portion (316) is disposed with grip locator (330) comprising a single corner (332) along an axial direction of the proximal end of the handle portion (316), and a bevelled-side (V-shaped) notch (334) having a linear base is provided within the longitudinal span of the corner. **FIG. 20A** shows an end view of the handle portion (316) depicting the corner (332).

The gripper jaws may close to form a profile similar in profile to a transverse cross-section of the grip locator (330); the profile in particular may complement the corner (332) of the grip locator (330). When the jaws close, the corner (332) seated in the jaw profile ensures that the steering guide (300) is correctly and stably aligned with the RA.

The handle portion (316) may be provided with a docking beacon (340) configured to provide information as to the position and optionally orientation of the steering guide (300) relative to the RA fitting (430). The docking beacon (340) allows manual, semi-automatic or automatic guidance of the RA fitting (430) that comprises a gripper (e.g. a set of jaws, chuck) to the handle portion (316) in particular to the grip locator (330). The position and optionally orientation of the steering guide (300) relative to the RA fitting (430) can be determined and tracked in real-time.

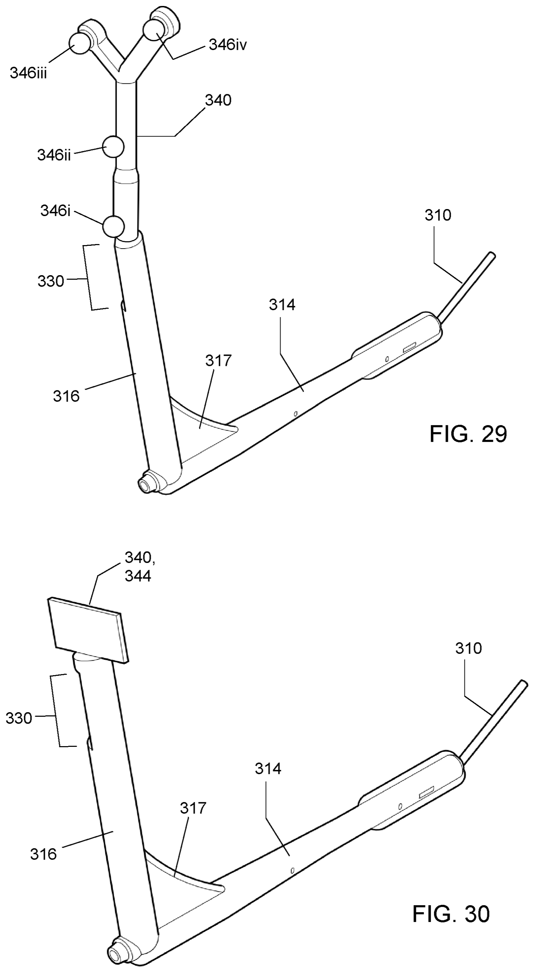

Exemplary docking beacons (340) are illustrated in **FIGs. 29** to **31****.** The pose of the RA fitting (430) can be adjusted in real-time as it approaches the handle portion (316) based on the relative pose of the docking beacon (340) to the RA fitting (430), thereby allowing coupling without disturbing the pose of steering guide (300) already inserted in the subject canal. A closed feedback loop may be used to guide the RA fitting (430) towards the target docking beacon (340); where the approaching RA fitting (430) deviates from the target handle portion (316) a correction to the approach direction is applied until the approach is on target. The closed feedback loop continuously checks and corrects the approach direction. The docking beacon (340) may be provided at the proximal tip of the handle portion (316). The docking beacon may be disposed on the handle portion (316) proximal to the grip locator (330). The docking beacon (340) may be passive or active, or a combination of passive and active. The docking beacon (340) may be detachable from the handle portion (316). The docking beacon (340) may be non-detachable from the handle portion (316).

A passive docking beacon comprises a body of a predefined geometric shape that is recognisable by a vision guided system of the RA (*e.g.* one or more cameras, laser scanner).

Vision-guided robotic systems are well known in the art. The shape of the body and its orientation allows identification of the pose of the handle portion (316). The body of the passive docking beacon may be positioned at the proximal end of the handle portion (316), preferably at the proximal tip. It may be positioned proximal of the grip locator (330). The optical recognition system may be provided attached to the RA fitting (430).

The body of the passive docking beacon may comprise a plurality of spheres (361i to iv) as shown, for instance, in **FIG. 29****.** The number of sphere may be at least 3. The positions and spacing of the spheres are pre-defined. The orientation of the handle portion (316) can be determined from a three-dimensional image of the spheres and their mutual distances. The distance of the RA fitting (430) from the handle portion can be determined from a three-dimensional image of the spheres and their diameter which will appear the same in any orientation.

The body of the passive docking beacon may comprise a two-dimensional shape (344) (*e.g.* rectangular form) as shown, for instance, in **FIG. 30****.** The rectangular form is of a pre-defined size and shape. The orientation of the handle portion (316) can be determined from a three-dimensional image of the rectangular form which exhibits a sheered structure depending on the orientation. The distance of the RA fitting (430) from the handle portion can be determined from a non-contact distance measurement device (*e.g*. laser or ultrasonic range finder); where the optical recognition system is a laser scanner, it may incorporate a laser range finder.

An active docking beacon wirelessly emits information that allows the position and/or orientation of the handle portion (316) to be determined. It may comprise a solid state gyroscope (3-axis). It may comprise a solid state gyroscope (3-axis), wireless transmitter (*e.g*. Bluetooth), a controller and a replaceable or rechargeable power source. The angle of approach of the RA fitting (430) may be adapted according to the pose of the handle portion (316) as transmitted by the active docking beacon. Distance between the RA fitting and the handle portion (316) may be determined by a non-contact distance measurement device (*e.g*. laser or ultrasonic range finder).

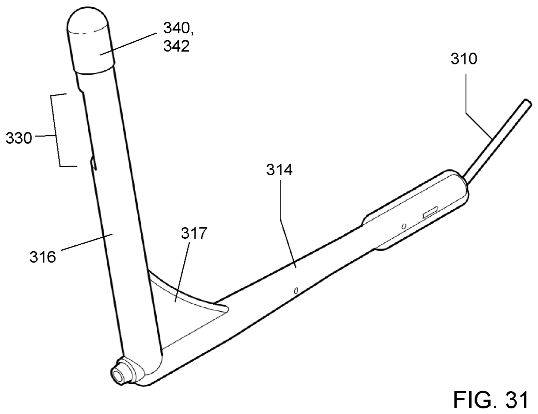

An exemplary active docking beacon (342) is shown, for instance, in **FIG. 31****.**

Another example of an active docking beacon is an array of position-determining radio transponder as described elsewhere herein. The positions of the transponders are trackable in real-time a spatial transponder detector that can typically delivers sub-millimetric, sub-degree accuracy. The transponder may receive power inductively or from a built-in power source, for instance, by a battery located on the handle of the steering guide.

The RA may be docked manually to the positioning tool (200) already inserted into the subject using the RA in zero gravity mode. The handle portion (316) may be connected or connectable to the RA fitting in the zero gravity mode described above. In zero gravity-on (weightless) mode, the joints of the robotic arm are supported (e.g. by the servos) to prevent collapse of the arm, and the pose of the RA fitting (430, 430a, 430b) can be guided manually, by the medical staff, to the vicinity of the handle portion (316') when then distal end of the positioning tool (200) has already been inserted into the subject. Once the connection between RA fitting (430, 430a, 430b) and handle portion (316) has been made, the zero gravity mode may be deactivated (zero gravity-off mode) and the actions performed as described elsewhere herein.

The RA may be docked manually to the positioning tool (200) already inserted into the subject and using the RA set to a treatment pose. Docking may realized by setting the pose of the RA fitting (430) on the radiotherapy treatment table to one of the treatment poses, and attaching the RA fitting (430) to the handle portion (316) of the steering guide (300) that has been inserted in the patient. The steering guide (300) is introduced inside the patient, the patient being placed in the same position as during simulation using laser lights and imaging. The steering guide (300) is moved slightly to the left or to the right to move the handle portion (316) out of the way of the RA fitting (430, 430a, 430b). Then, the pose of the robotic arm (400) is adjusted to adopt the same pose as the one reached during simulation (treatment pose); in other words the RA fitting (430, 430a, 430b) is moved to a treatment pose. The treatment pose is maintained while the steering guide (300) is connected manually to the RA fitting (430, 430a, 430b) (e.g. effector end (gripper)) of the robotic arm. The steering guide would thus have the same pose inside the patient as during simulation.

The effector shaft (310), disposed at the distal end (20) of the steering guide is configured for insertion into the canal of the subject (50), and/or for attachment to an inserter (204) configured for insertion into the canal of the subject (50).

Where the inserter (204) is present, the effector shaft (310) may be dismountably or permanently attached to the inserter (204) or elongated member (310). The effector shaft (310) may be configured for (repeatable) slidable and removable insertion into the elongated member lumen (214) of the inserter (204). The effector shaft (310) may be configured for slidable and removable insertion into the elongated member lumen (214) along the guiding strand (218) where present in the inserter (204).

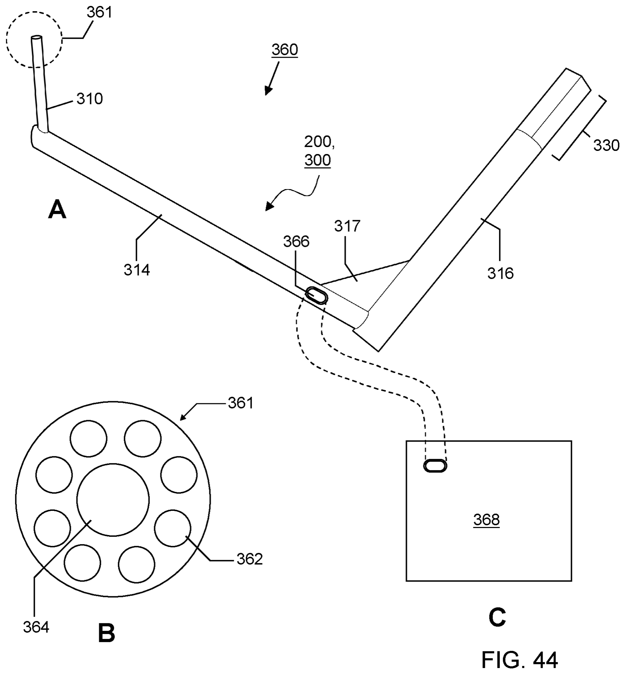

Where the steering guide (300) has no guiding strand passage (312), effector shaft (310) may be introduced into the elongated member lumen (214) of an *in situ* inserter (204) using a speculum to provide a line of sight to the practitioner to the elongated member lumen (214). Examples where the steering guide (300) has no guiding strand passage (312) are shown, for instance, in **FIG. 44A****.** The image capture system (360) exemplified in **FIG. 44A** shows the distal tip (361) of the effector shaft (310) provided with an image inlet port (364) for receiving object light and a plurality of light outlet ports (362) for illuminating the object from a light source. Realtime images captured by the image capture system (360) assist the practitioner in guiding the effector shaft (310) to the elongated member lumen (214).

When steering guide is configured for insertion into the canal of the subject (50), the inserter (204) does not need to be attached. This is known as direct insertion. Where the inserter (204) is absent, the effector shaft (310) may be configured for entry into the cervix, uterus, anus, or vagina. The effector shaft (310) may contact the tissue of the canal directly, or through a thin (e.g. <1 mm) protective sheath, or through an inflatable effector shaft (310) balloon (315) described later below. The inflatable effector shaft balloon (315) serves to dilate the canal (e.g. rectal, cervical) and to centre the effector shaft (310)..

The effector shaft (310) may have a circular cross-sectional outer profile perpendicular to its longitudinal axis. The outer profile may have the same size in an axial direction. The outer profile may be tapered in an axial direction; the small profile may be at the distal end.

The effector shaft (310) may be disposed with one or more indentations on the surface that co-operate with complementary protrusions in the inner surface of the elongated member lumen (214). The arrangement allows the effector shaft (310) to latch within the elongated member lumen (214). The effector shaft (310) may click into position in the inserter (204). Removal of the effector shaft (310) is by pulling to overcome the force of the latch.

The effector shaft (310) is preferably rigid. It is preferably non-flexible. It may be substantially formed of a rigid rod. It may have a straight form for instance for use with the cervix, uterus, anus, vagina. It may have a curved form for use with the rectal ampulla.

The effector shaft (310) may be provided with the guiding strand passage (312) for slidable movement thereof along the guiding strand (218). The guiding strand passage (312) may be a lumen within the effector shaft (310) or a longitudinal groove (e.g. **FIG. 14****,** and detail **FIG. 14A****)** on the surface of the effector shaft (310). The guiding strand passage (312) may be provided at least partially along a longitudinal length of the effector shaft (310). An entrance (312, -a) to the guiding strand passage is disposed at the distal end of the effector shaft (310), preferably at the distal tip. An exit (312, -b1 to -b5) from the guiding strand passage is disposed proximal to *(i.e.* at a proximal side of) the entrance (312, -a). **FIG. 1** shows different possible positions for the guiding strand passage (312) entrance (312, -a) and exit (312, -b) where the guiding strand passage is a lumen. An exit (312,-b1) to the guiding strand passage (312) may be disposed at a distal end of the effector shaft (310). An exit (312,-b2) to the guiding strand passage (312) may be disposed at a proximal end of the effector shaft (310). An exit (312,-b3) to the guiding strand passage (312) may be disposed at a distal end of the transmission (314). An exit (312,-b4) to the guiding strand passage (312) may be disposed in a mid-portion of the transmission (314). An exit (312,-b5) to the guiding strand passage (312) may be disposed at a proximal end of the transmission (314).

In **FIG. 11****,** the guiding strand passage (312) entrance (312, -a) is at the effector shaft (310) distal tip, and exit (312, -b3) provided towards a distal end of the transmission (314). In **FIG. 12** the exit (312, -b5) is provided where the proximal end of the transmission (314) joins with a distal end of the handle portion (316). In **FIG. 13****,** the guiding strand passage (312) entrance (312, -a) is at the effector shaft (310) distal tip, and exit (312, -b2) provided towards a proximal end of the effector shaft (310). The further the distance of the exit (b) from the entrance (a), the longer the guiding strand (218). In **FIG. 19****,** **22** and **23****,** the guiding strand passage (312) entrance (312, -a) is at the effector shaft (310) distal tip, and the exit (312, -b4) is provided towards on a mid-section the transmission (314).

The effector shaft (310) may be made from any suitable biocompatible material such as medical grade non-ferromagnetic stainless steel, tantalum, titanium, polycarbonate, PEEK, carbon fibre, fibreglass, polyarylamide resin reinforced with fibers (e.g. Ixef (Solvay)), aluminium (coated).

Usually the treatment is first simulated under a CT scan, or a PET CT scan, or under MRI. Later the treatment may involve acquisition of one or more X-rays in the treatment room. It is preferred that the effector shaft (310) is made from a material compatible with medical imaging, such as with CT or MRI or X-ray. The material may or may not be visible on a medical image.

Where the effector shaft (310) is visible on a medical image, the pose of the effector shaft (310) may be determined directly from the medical image of the effector shaft (310). When the treatment is simulated under MRI, the effector shaft (310) may be manufactured from a low density material, such as PEEK or polycarbonate, polyarylamide resin reinforced with fibers (e.g. Ixef (Solvay)) or from a MR compatible (non-magnetic) material, such as aluminium coated with a layer of biocompatible metal (titanium) or from titanium. For the CT scan simulation, coated aluminium or titanium, PEEK, polycarbonate or polyarylamide resin reinforced with fibers (e.g. Ixef (Solvay)) (mixed with barium sulfate) would also be an advantage, because it allows for less artefacts compared with using a high density metal such as stainless steel. It is an aspect that a coated aluminium, PEEK, polycarbonate, polyarylamide resin reinforced with fibers (e.g. Ixef (Solvay)) steering guide (300) (mixed with barium sulfate) is used for simulation and the stainless steel for the treatment. It is as aspect that an aluminium steering guide (300) or PEEK, polycarbonate or polyarylamide resin reinforced with fibers (e.g. Ixef (Solvay)) (mixed or not with barium sulfate) is used for simulation and for treatment. Most modern imaging devices that are combined with radiotherapy treatment devices allow a good visibility of any metal structure or radio-visible polymer such as the effector shaft (310). In this case, the effector shaft may be visible by itself and could not necessarily need the presence of imaging markers to be seen by the imaging device.

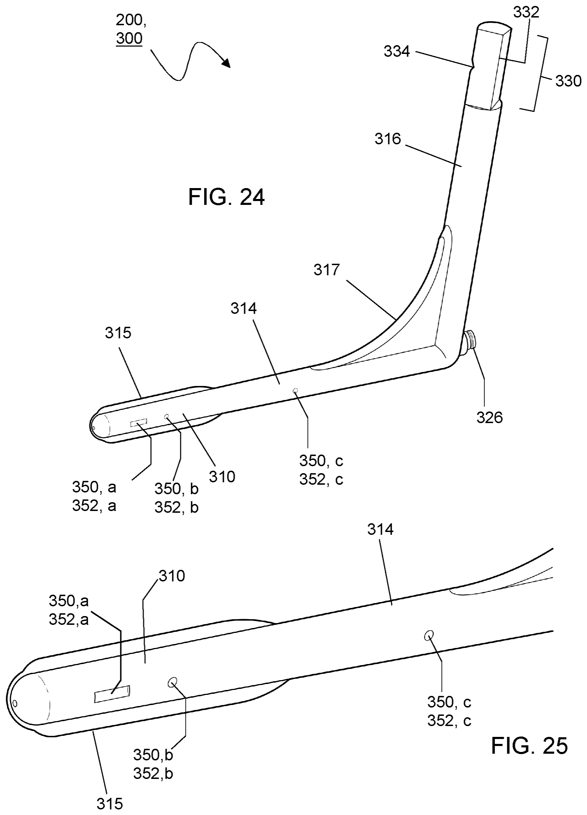

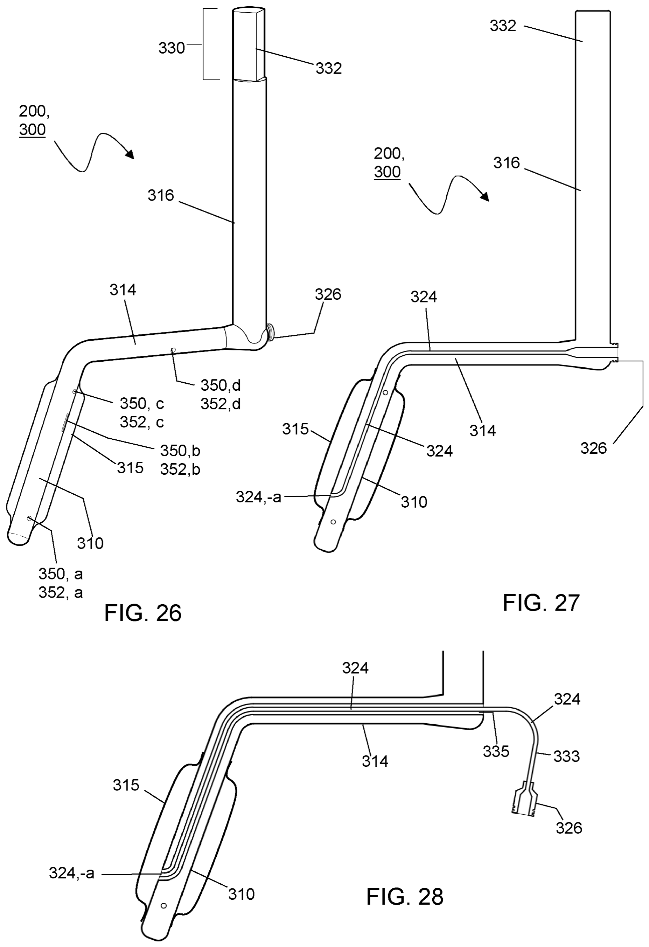

Where the effector shaft (310) is not visible or not sufficiently visible to determine the position and/or orientation of the inserter, the effector shaft (310) may be disposed with one or more imaging markers (350, a, b, c). This is of assistance when performing images using the imaging tools of the linear accelerator. The steering guide (300), in particular the effector shaft (310) may be provided with one or more imaging markers that can be identified by a medical image. An imaging marker may be provided in fixed relation to the effector shaft (310), for instance on an inner surface of, outer surface of or within the effector shaft (310). An imaging marker may be made from a material different from the effector shaft (310) for instance a heavy metal such as platinum, platinum iridium, tantalum, tungsten, etc. **FIGs. 24** to **28** show one or more imaging markers (350, a, b, c) disposed on the effector shaft (310) (FIG. 24: 350, a,b; **FIG. 25****:** 350, a,b; **FIG. 26****:** 350, a,b,c).

An inflatable effector shaft balloon (315) may be provided on the effector shaft (310), for example as shown in **FIGs. 1****,** **24****,** **25****,** **26** to **28.** The inflatable effector shaft balloon (315) may be present when the inserter (204) is absent. The inflatable effector shaft balloon (315) may be used to dilate the cervical canal or rectal canal, preferably to a known or fixed diameter, for radiation treatment. The inflatable effector shaft balloon (315) is repeatably inflatable and deflatable. It has a deflated state for repeatable insertion into and removal from the canal (602), and inflated state for repeatable dilation of the canal (602). The inflatable effector shaft balloon (315) having deflated state considerably reduces pain for the subject during insertion and withdrawal at each treatment session. In particular for cancer patients, the canal may be tender from the radiotherapy, and the subject may not be able to withstand invasion. The canal may be dilated slowly by gradual inflation at the start of each treatment session thereby reducing pain and discomfort. At the end of treatment the dilation force may be released and the effector shaft (310) withdrawn from the subject.

The inflatable effector shaft balloon (315) may further centre the effector shaft (310) *e.g.* within the canal. In the inflated condition, it assists in placing the cervical canal or rectal canal in a defined position and/or orientation and/or diameter for radiotherapy treatment. The diameter of the effector shaft (310) is small at a distal end (20), the inflatable effector shaft balloon (315) allows entry into the canal with a effector shaft (310) which is less painful. Inflation of the inflatable effector shaft balloon (315) dilates the canal wall so that the wall becomes positioned. A wall of the inflatable effector shaft balloon (315) may be made from any suitable expandable or non-expandable material. Examples of expandable materials include latex, any elastic polymer, thin film polymers (PET, nylon, polyamide, polyurethane or others) or other elastomers. The inflatable effector shaft balloon (315) may have a limited inflation size, whereby inflation at or above the maximum inflation size is resisted (semi-compliant or non-compliant balloon). In other words, the inflatable effector shaft balloon (315) may be expansion limited, wherein expansion reproducibly stops at the limited inflation size. Continued inflation by application of hydraulic pressure at or above the limited inflation size will not result in further expansion. The limited inflation size is reproducible, for instance, in one or more further treatment sessions. The reproducible limited inflation size, ensures that the target is in the correction position for a given treatment pose, since expansion will stop once the limited size has been reached. The distance between the inflated balloon wall and the effector shaft is known and/or reproducible. In particular for repeated treatments in a fractionated treatment programme, the limited inflation size reduces placement errors in subsequent treatment sessions. Limited maximum inflation size may be achieved by forming the balloon wall from a non-expandable material such as PET, non-compliant or semi-compliant polyamide. Alternatively, it may be achieved by applying a sheath of the non-expandable material over a balloon of expandable material. The inflatable effector shaft balloon (315) may have a maximum inflation diameter of 2.0 to 6 cm. Examples of inflatable effector shaft balloon (315) dimensions and medical applications are provided in **Tables 1** and **1a.**

In fluid connection with the inflatable effector shaft balloon (315) may be an inflation lumen (324) that extends in a proximal (40) direction of the steering guide (300) *(e.g.* **FIGs. 27, 28****).** This inflation lumen (324) may be a channel within a body of at least a part of the effector shaft (310) and/or of the transmission portion (314); it may terminate in a port (324,-a) within the balloon lumen *(e.g.* **FIG. 27****).** This inflation lumen (324) may be within a tubing (333) disposed within a passage (335) within a body of at least a part of the transmission portion (314) *(e.g.* **FIG. 28****);** it terminates in a port (324,-a) within the balloon lumen. This inflation lumen (324) may be outside and lie parallel to at least a part of the effector shaft (310) and/or transmission portion (314). A fitting (326) (e.g. Luer fitting) may be disposed at the proximal end of the inflation lumen (324) for connection to a pump. The fitting (326) may be integrated into the steering guide (300), for instance, it may be provided in fixed relation to the transmission (314) *(e.g.* **FIG. 27****).** The fitting (326) may be an inline fitting attached to the proximal end of the tubing (333) (*e.g*. **FIG. 28****).** The inflatable effector shaft balloon (315) may be deflated after simulation and/or after each session or fraction of radiotherapy treatment by release of inflation fluid (*e.g*. saline) thereby allowing the steering guide (300) to be withdrawn.

The inflatable effector shaft balloon (315) may be inflated with saline or sterile water. It may optionally contain 1-4 % of contrast medium which allows effector shaft balloon to be visible on CT simulation images and/or on images made before the radiotherapy session or fractions. Alternatively, the inflatable effector shaft balloon (315) may be provided with one or more imaging markers (e.g. imaging visible wires (longitudinal, helicoidal, circular)).

The effector shaft (310) may have a length (E) of 1 - 15 cm, preferably of 4-10 cm for cervical/uterus/anal/vaginal insertion, or preferably of 5 to 15 cm or 7 to 11 cm for rectal insertion (see **FIG. 1****).** The maximum outer diameter of the effector shaft may be 0.1 to 4 cm.

When the effector shaft balloon is present, the effector shaft (310) may have a maximum outer diameter of 0.1 to 0.8 cm for smaller canals (e.g. cervical canal, vaginal vault, uterus canal) or 0.5 to 4 cm for larger canals (e.g. vagina, rectum, anus).

When the effector inserter is present, the effector shaft may have maximum outer diameter of 0.1 to 0.8 cm for smaller canals (e.g. cervical canal, vaginal vault, uterus canal) or 0.5 to 4 cm for larger canals (e.g. vagina, rectum, anus).

When the effector shaft balloon and inserter are absent, the effector shaft may have maximum outer diameter of 0.1 to 0.8 cm for smaller canals (e.g. cervical canal, vaginal vault, uterus canal) or 0.5 to 4 cm for larger canals (e.g. vagina, rectum, anus).

The outer diameter of effector shaft (310) may be uniform from proximal to distal end or may vary. For instance, the diameter may be larger towards the proximal part and smaller towards the distal part. The change in diameter may be gradual. The change in diameter may be gradual across the length of the effector shaft (310).

The effector shaft may adopt and angle alpha with respect to the transmission (see **FIG. 1****).** The angle alpha is measured in a plane formed between the transmission and effector. The angle alpha is less than 180 deg when the handle and effector shaft are on the same side (cis) of the transmission; the angle alpha is greater than 180 deg when the handle and effector shaft are on opposite sides of the transmission (trans). The angle alpha is 180 deg when the handle and effector shaft mutually coaxial or linear. See **Tables 1** and **1a** for preferred dimensions, angles for various medical applications.

The effector shaft (310) and the handle portion (316) may be connected by a transmission (314). The transmission (314) is typically a rigid rod. The transmission may be provided in fixed connection and relation (*i.e.* directional and/or positional) with both the effector shaft (310) and the handle portion (316). It may be a straight, curved, or contain a one or more angular bends. It may be substantially formed of a rigid rod. The rod may be hollow or solid.

The transmission (314) may have a length (T) of 1 - 30 cm, preferably of 10 - 25 cm for cervical/uterus/ vaginal vault resection insertion, and preferably of 4 to 25 cm for anal/vaginal/rectal insertion (see **FIG. 1****).** The transmission (314) may have a maximum outer diameter of 0.3 - 3 cm, preferably of 0.3 - 1.2 cm. The handle portion and plane formed between transmission and effector shaft may adopt an angle gamma with respect to each other. See **Tables 1** and **1a** for preferred dimensions, angles for various medical applications.

The diameter may be uniform from proximal to distal end or may vary. For instance, the diameter may be larger towards the proximal part and smaller towards the distal part of the transmission. The change in diameter may be gradual. The small distal diameter is more atraumatic when entering the anus or vagina, and a larger diameter towards the proximal part improves rigidity of the steering guide (300).

The handle portion (316) may be in continuation with the transmission (314). The transmission (314) may have the same diameter (e.g. 0.3 - 3 cm, preferably 0.5 - 2 cm) as the handle portion (316) for a part of its proximal length, for instance for 3-6 cm of the proximal length of the transmission (314). Having the larger diameter of the handle portion (316) continued into a proximal part of the transmission (314) allows stiffening of the steering guide (300). A strengthening strut (317) may be disposed in a corner between the handle portion (316) and transmission (314) *(e.g.* **FIGs. 19****,** **21****,** **22****,** **24****).**

The distal part (20) of the transmission (314) may have a smaller diameter (0 - 0.8 cm) in order to be atraumatic by its diameter (atraumatic when entering the vagina or anus).

The guiding strand passage (312) where present may continue from the effector shaft (310) into the transmission (314). The guiding strand passage (312, a-b) may be a lumen within the transmission (314) or a longitudinal groove on the surface of the transmission (314). The guiding strand passage (312) may continue at least partially along a longitudinal length of the transmission (314). In **FIG. 1** possible guiding strand passage (312) exits on the transmission are at the distal end (312,-b3), mid-section (312,-b4), or proximal end (312,-b5). In **FIG. 11** a guiding strand passage (312) exits on the transmission at the distal end (312,-b3). In **FIG. 12** a guiding strand passage (312) exits on the transmission are at the proximal end (312,-b5). In **FIGs. 19****,** **22** and **23** possible guiding strand passage (312) exit on the transmission is at the mid-section (312,-b4) in the lower part of the transmission.

An inflatable transmission balloon (322) may be provided towards a distal (20) end of the transmission (314), for example as shown in **FIGs. 1****,** **13****,** **21, 22, 23****,** **35****.** The inflatable transmission balloon (322) may be used to dilate the vagina or anus, preferably to a known or fixed diameter, for radiation treatment. The inflatable transmission balloon (322) is repeatably inflatable and deflatable. It has a deflated state for repeatable insertion into and removal from the canal (602), and inflated state for repeatable dilation of the canal (602). The inflatable transmission balloon (322) having deflated state considerably reduces pain for the subject during insertion and withdrawal at each treatment session. In particular for cancer patients, the canal may be tender from the radiotherapy, and the subject may not be able to withstand invasion. The canal may be dilated slowly by gradual inflation at the start of each treatment session thereby reducing pain and discomfort. At the end of treatment the dilation force may be released and the transmission withdrawn from the subject.

The inflatable transmission balloon (322) may further centre the transmission (314) e.g. within the anal or vaginal passage (606). In the inflated condition, it assists in placing the anal or vaginal passage (606) in a defined position and/or orientation and/or diameter for radiotherapy treatment. The diameter of the transmission (314) is small at a distal end (20), the inflatable transmission balloon (322) allows entry into the anal or vaginal passage (606) with a narrower transmission (314) which is less painful. Inflation of the inflatable transmission balloon (322) dilates the anal or vagina wall so that the wall becomes positioned. A wall of the inflatable transmission balloon (322) may be made from any suitable expandable or non-expandable material. Examples of expandable materials include latex, any elastic polymer, thin film polymers (polyurethane or others) or other elastomers. The inflatable transmission balloon (322) may have a limited maximum inflation size, whereby inflation at or above the maximum inflation size is resisted (semi-compliant or non compliant balloon). In other words, the inflatable transmission balloon (322) may be expansion limited, wherein expansion reproducibly stops at a limited inflation size. Continued inflation by application of hydraulic pressure at or above the limited inflation size will not result in further expansion. The limited inflation size is reproducible, for instance, in one or more further treatment sessions. The reproducible limited inflation size, ensures that the target is in the correction position for a given treatment pose, since expansion will stop once the limited size has been reached. The distance between the inflated balloon wall and the effector shaft is known and/or reproducible. In particular for repeated treatments in a fractionated treatment programme, the limited inflation size reduces placement errors in subsequent treatment sessions. Limited maximum inflation size may be achieved by forming the balloon wall from a non-expandable material such as PET, non-compliant or semi-compliant polyamide. Alternatively, it may be achieved by applying a sheath of the non-expandable material over a balloon of expandable material. The inflatable transmission balloon (322) may have a maximum inflation diameter of 2.0 to 5 cm. Examples of transmission balloon (322) dimensions and medical applications are provided in **Tables 1** and **1a.**

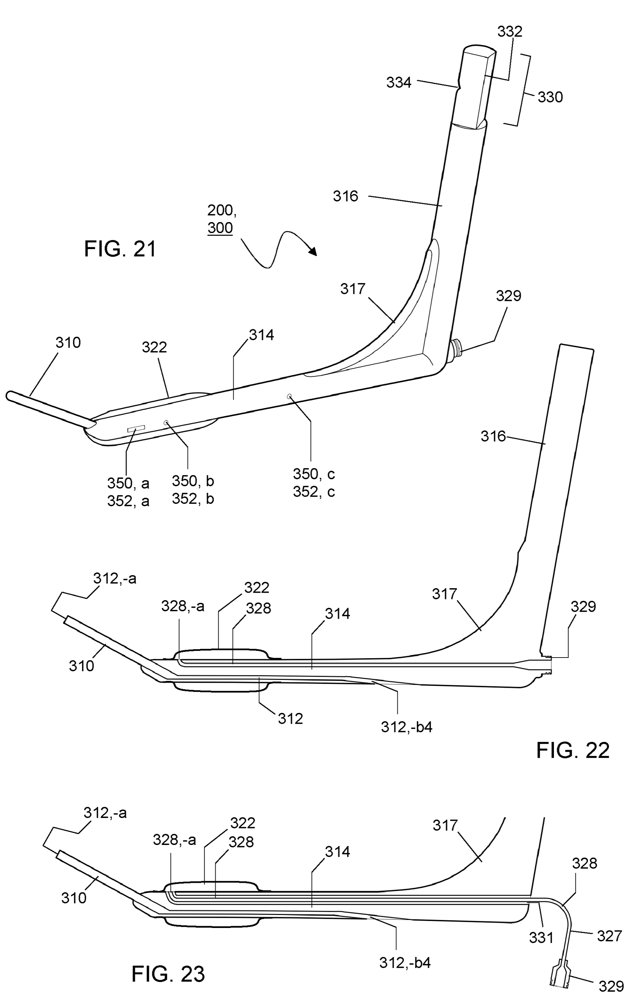

In fluid connection with the inflatable transmission balloon (322) may be an inflation lumen (328) that extends in a proximal (40) direction of the steering guide (300) This inflation lumen (328) may be a channel within a body of at least a part of the transmission portion (314) *(e.g.* **FIG. 22****);** it terminates in a port (328,-a) within the balloon lumen. This inflation lumen (328) may be within a tubing (327) disposed within a passage (331) within a body of at least a part of the transmission portion (314) *(e.g.* **FIG. 23****);** it terminates in a port (328,-a) within the balloon lumen. This inflation lumen (328) may be outside and lie parallel to at least a part of the transmission portion (314). A fitting (329) (e.g. Luer fitting) may be disposed at the proximal end of the inflation lumen (328) for connection to a pump. The fitting (329) may be integrated into the steering guide (300), for instance, it may be provided in fixed relation to the transmission (314) *(e.g.* **FIG. 22****).** The fitting (329) may be an inline fitting attached to the proximal end of the tubing (327) (e.g. **FIG. 23****).** The inflatable transmission balloon (322) may be deflated after simulation and/or after each session or fraction of radiotherapy treatment by release of inflation fluid (e.g. saline or sterile water) thereby allowing the steering guide (300) to be withdrawn.

The transmission balloon (322) may be inflated with saline or sterile water. It may optionally contain 0.5-4 % of contrast medium which allows transmission balloon to be visible on CT simulation images and/or on images made before the radiotherapy session or fractions. Alternatively, the transmission balloon (322) may be provided with one or more imaging markers (e.g. imaging visible wires (longitudinal, helicoidal, circular)). One or more radio position-determining transponders may be located on the inflatable transmission balloon (322).

An inflatable transmission balloon (322) may provided towards a distal (20) end of the transmission (314), wherein:
- optionally the inflatable transmission balloon (322) has a fixed maximum inflation diameter *i.e.* a limited maximum inflation size, and/or
- optionally, the inflatable transmission balloon (322) bears one or more imaging markers visible by medical imaging, and/or
- optionally, the inflatable transmission balloon (322) bears one or more radio transponders for determining a position and/or orientation of the transmission (314) and/or of the effector shaft (310) by a spatial transponder detector.

The transmission (314) may be made from any suitable biocompatible material such as medical grade non-ferromagnetic stainless steel, tantalum, titanium, polycarbonate, PEEK, carbon fibre, polyarylamide resin reinforced with fibers (e.g. Ixef (Solvay)), polyphenylsulfone (PPSU), fibreglass, aluminium (coated) fiberglass, bioceramics such as aluminosilicates, styrene acrylonitrile, bioceramic-polymer materials. The transmission (314) may be made from the same material as the handle portion (316). The transmission (314) at the proximal end may have the same diameter as the distal end of the handle portion (316).

The transmission (314) may be formed from an imaging-transparent material such as a polymeric rod or tube. The same material may be used to form the handle portion (316), for ease of manufacture; this can reduce imaging artefacts caused by the transmission (314) *e.g.* in the vaginal regional (606) - this is described in more detail below. Examples of suitable polymers include polycarbonate, polyphenylsulfone (PPSU), and polyarylamide resin reinforced with fibers (e.g. Ixef (Solvay)). Other materials that may be used for the handle portion are fiberglass, carbon fiber, bioceramics such as aluminosilicates, styrene acrylonitrile, bioceramic-polymer materials, etc. **FIGs. 11, 12** and **19** depict examples of a steering guide (300) formed from a polymeric handle portion (316) and transmission (314) and both having a larger diameter (e.g. 0.8 - 2.5 cm) compared with the effector shaft (310) which may be formed from a rigid metal such as titanium.

Where the transmission (314) is not visible or not sufficiently visible to determine the position and/or orientation of the inserter, the transmission (314) may be disposed with one or more imaging markers (350, c, d). This is of assistance when performing images using the imaging tools of the linear accelerator. The steering guide (300), in particular the transmission (314) may be provided with one or more imaging markers that can be identified by a medical image. An imaging marker may be provided in fixed relation to the transmission (314), for instance on an inner surface of, an outer surface of or within the transmission (314). An imaging marker may be made from a material different from the transmission (314) for instance a heavy metal such as platinum, platinum iridium, tantalum, tungsten, etc. **FIGs. 21****,** **24****,** **25****,** **26** show one or more imaging markers (350, c, d) disposed on the transmission (314) **(****FIG. 24****:** 350, c; **FIG. 25****:** 350, c; **FIG. 26****:** 350, d).

The polymeric rod or tube for a transmission (314) may have a larger diameter (e.g. 1 cm) compared with a transmission (314) made from a stronger material such as titanium or stainless steel. A polymeric transmission (314) may significantly contribute to reducing artifacts and obtaining superior images of the diseased structures. Some imaging artefact may arise from any imaging markers present on the transmission (314), from the effector shaft (310) which may be made from titanium which corresponds to the intra-cervical part, and from any imaging markers disposed on the inserter (204) but will be less important than if whole steering guide (300) is made from metal (titanium, non ferro-magnestic steel, coated aluminium, etc.).

When made from a polymeric or ceramic material, the transmission portion (314) material may be mixed with radio-visible material such as barium sulfate, in order to render it radio-visible on simulation images as well as on control images performed before each radiation therapy session. It order to render it radio-visible, the surface of the transmission portion may also be covered with radiovisible longitudinal, circular, helicoidal markers made from metal (thin titanium or lead wires) or from material mixed with barium sulfate for instance. When made from a polymeric material, the transmission portion may also contain radio-visible markers inside the structure.

An angle (alpha) (e.g. **FIG. 1**) may be formed between the effector shaft (310) and the transmission (314) of 90 deg to 260 deg, depending on the position of the canal e.g. the cervix, uterus, vagina, rectum and the ease of access. An angle (beta) (e.g. **FIG. 1**) may be formed between the handle portion (316) and the transmission (314) of 40 deg to 150 deg, depending on ease of access. An angle (gamma) (e.g. **FIG. 1A****)** may be formed between the handle portion (316) and a plane formed by the transmission (314) and handle portion (316) 0 or -30 to +30, depending on ease of access. See **Tables 1** and **1a** for preferred dimensions, angles for various medical applications.

When the canal is the cervical canal, the steering guide typically has a geometry as shown in **FIGs. 1****,** **10** to **14A, 19, 21, 22, 35.**

When the canal is the anal or vaginal canal, the steering guide (300) typically has a geometry as shown in **FIGs. 24** and **25****.**

When the canal is the rectal canal, the steering guide (300) typically has a geometry as shown in **FIGs. 26** to **28****.**

**Table 1: Exemplary dimensions of elongated member and parts of steering guide. Dimensions may exceed ranges for some presenting subjects and are not intended to be limiting.**

| **Type of tumor → Information ↓** | **Primitive tumor of cervix uteri** | **Vaginal Vault recurrence (Uterus absent)** | **Primitive tumor of Vagina or recurrence located in the vagina (from cervical, uterine, rectal cancer) Uterus Present** | **Primitive tumor of corpus uteri if inoperable** | **Primitive tumor of Vagina or recurrence located in the vagina (from cervical, uterine, rectal cancer) Uterus Absent** | **Primitive tumor of anus** | **Primitive tumor of the rectum** |
|---|---|---|---|---|---|---|---|
| **Insertion position** | Cervical canal | Vaginal vault resection | Cervical canal | Cervical/uterus canal | Vaginal canal | Anal canal | Rectal canal |
| **Length of elongated member (cm)** | 1 to 8 | 1 to 5 | 1 to 8 | 1 to 10 | Inserter absent | Inserter absent | Inserter absent |
| **Length of effector shaft (E)(cm)** | 1 to 8 | 1 to 5 | 1 to 8 | 1 to 10 | 1 to 10 | 1 to 10 | 5 to 15 |
| | | | | | | | pref: 7 to 11 |
| **Length of transmission part (T) (cm)** | 10 to 25 (up to 30 cm if obese) | 10 to 25 (up to 30 cm if obese) | 10 to 25 (up to 30 cm if obese) | 10 to 25 (up to 30 cm if obese) | 4 to 25 | 4 to 25 | 4 to 25 |
| **Length of handle part (H) (cm)** | 15 to 25 | 15 to 25 | 15 to 25 | 15 to 25 | 15 to 25 | 15 to 25 | 15 to 40 |
| | 40-50 in obese | 40-50 in obese | 40-50 in obese | 40-50 in obese | 40-50 in obese | 40-50 in obese | 40-50 in obese |
| **Angle E - T parts (°) Alpha** | 90 to 240 | 90 to 210 | 90 to 240 | 90 to 240 | 170 to 190 ideallv 180 | 170 to 190 ideallv 180 | 90 to 260, ideallv 220 to 240 |
| **Angle T - H parts (°) Beta** | 70 to 150 | 70 to 150 | 70 to 150 | 70 to 150 | 70 to 150 | 70 to 150 | 40 to 130 |
| **Angle H wrt plane (T-E) (°) Gamma** | 0 | -30 to +30 | 0 | 0 | 0 | 0 | 0 |

**Table 1a: Exemplary diameter and length of effector shaft, elongated member, transmission balloon. Dimensions may exceed ranges for some presenting subjects and are not intended to be limiting.**

| **Type of tumor → Information ↓** | **Primitive tumor of cervix uteri** | **Vaginal Vault recurrence (Uterus absent)** | **Primitive tumor of Vagina or recurrence located in the vagina (from cervical, uterine, rectal cancer) Uterus Present** | **Primitive tumor of corpus uteri if inoperable** | **Primitive tumor of Vagina or recurrence located in the vagina (from cervical, uterine, rectal cancer) Uterus Absent** | **Primitive tumor of anus** | **Primitive tumor of the rectum** |
|---|---|---|---|---|---|---|---|
| **Insertion position** | Cervical canal | Vaginal vault resection | Cervical canal | Cervical/uterus canal | Vagina | Anus | Rectal canal |
| **Effector shaft (mm) max outer diameter when used with inserter** | 0.05 to 2 | 0.05 to 2 | 0.05 to 2 | 0.05 to 2 | Not applicable | Not applicable | Not applicable |
| **Inserter elongated member (cm) max outer diameter** | 0.1 to 0.8 | 0.1 to 0.8 | 0.1 to 0.8 | 0.1 to 0.8 | Not applicable | Not applicable | Not applicable |
| **Effector shaft (cm) max outer diameter when used without inserter or balloon** | Not applicable | Not applicable | Not applicable | Not applicable | 1 to 4 | 0.5 to 3 | 0.5 to 3 |
| **Effector shaft (mm) max outer diameter when used with balloon** | Not applicable | Not applicable | Not applicable | Not applicable | 0.3 to 3 | 0.3 to 2 | 0.3 to 2 |
| **Effector shaft balloon max outer diameter inflated (option) (cm)** | Not applicable | Not applicable | Not applicable | Not applicable | 2 to 6 | 2 to 6 | 2 to 15 |
| **Effector shaft balloon length inflated (option) (cm)** | Not applicable | Not applicable | Not applicable | Not applicable | 4-10 | 4-10 | 4-10 |
| **Transmission balloon** | 1-4, pref. 1.5-3.5 | 1-4, pref. 1.5-3.5 | 1-4, pref. 1.5-3.5 | 1-4, pref. 1.5-3.5 | Not applicable | Not applicable | Not applicable |
| **- max diameter** | | | | | | | |
| **- inflated (option)** | | | | | | | |
| **Transmission balloon** | 3-12, pref. 6 to 8 | 3-12, pref. 6 to 8 | 3-12, pref. 6 to 8 | 3-12, pref. 6 to 8 | Not applicable | Not applicable | Not applicable |
| **- length** | | | | | | | |
| **- inflated (option) (cm)** | | | | | | | |

The effector shaft (310) with or without inserter (204) moves responsive to movements of the effector shaft (310), which in turn moves responsive to movements of the transmission (314) and ultimately of the handle (316). By positioning the effector shaft (310) the position of the canal, of the tissue around the canal can be adjusted and maintained in a fixed position. By inflating the inflatable transmission balloon (322) the tissue around the canal can also be adjusted and maintained in a fixed position.

The steering guide (300), in particular the effector shaft (310) and/or transmission (314) and/or handle portion (316) and/or transmission balloon where present may be provided with one or more (e.g. 2, 3 or more) position-determining radio transponders (352, a, b, c, d; ) whose positions can be determined and tracked using a spatial transponder detector.

The terms position-determining radio transponder and transponder are used interchangeably herein. A transponder is sometimes known as beacon transponder. In **FIG. 21** three transponders (352, a, b, c) are provided fixed to an outer surface or inside of the transmission (314) at different positions. In **FIGs. 24** and **25** two transponders (352, a, b) are provided fixed to an outer surface of the effector shaft (310) at different positions, and one transponder (352,c) is provided on the transmission (314). In **FIGs. 26** three transponders (352, a,b,c) are provided fixed to an outer surface of the effector shaft (310) at different positions, and one transponder (352,d) is provided on the transmission (314). The same transponders (352 a-d) also act as imaging markers (350 a-d) because they are visible on a medical image.

A transponder is a device that emits electromagnetic pulses at a certain radio frequency that is detectable by the spatial transponder detector - typically comprising a number of spatially separated receivers (coils). The timing of the pulse as detected by a number of spatially separated receivers in a positional transponder reader (see later below) allow the location of the transponder to be accurately determined. A transponder is sometimes known as beacon transponder. The transponder may receive power inductively. The transponder may be powered by built-in power source, for instance, by a battery located on the handle of the steering guide. Where more than one transponder is present, each transponder may emit a signal at a different radio frequency. When at least three separately-identifiable transponders are disposed on the steering guide (300) at different positions, the orientation of the effector shaft (310) may also be determined. Examples of such systems are described, for example, in US 9,248,003 B2, and US 9,072,895. The use of transponders reduces the need to align the effector shaft (310) and/or transmission (314) prior to radiotherapy treatment using medical imaging several times which reduces exposure to imaging radiation.

The transponders allow realtime capture of the effector shaft (310) and/or transmission (314) position during simulation. The transponders also allow realtime and automated guidance (e.g. by a robotic arm) of the position and/or orientation of the effector shaft (310) and/or transmission (314) during treatment so that it aligns with a reference pose determined during simulation.

The transponders also allow the position and/or orientation of the effector shaft (310) to be guided, changed and fixed manually in real-time according to the position and orientation information captured by the spatial transponder detector. For instance, a closed feed-back loop, wherein continuous input is the pose of the steering guide (300) and hence of the effector shaft (310) as determined by from the one or more (e.g. 2, 3 or more) position-determining radio transponders, may provide guidance to the operator to manually bring the pose of the steering guide (300) and hence effector shaft (310) into agreement with the pose determined during simulation. In this scenario, the steering guide (300) may be attached by the handle portion (316) to a positioning device that is manually controllable (e.g. a manually adjustable positioning device having lockable passive joints, or a positioning device that is a robotic arm operating in a manual zero-gravity mode). The same transponders and manual control may also allow capture and storage of the pose of the steering guide (300) and hence of the effector shaft (310) during simulation.

According to one aspect:
- at least a part of the effector shaft (310) and/or one or more imaging markers borne thereby is visible by medical imaging, in particular by X-ray medical imaging and/or MR medical imaging
   and/or
- at least a distal part the transmission (314) and/or or one or more imaging markers borne thereby, is visible by medical imaging, in particular by X-ray medical imaging or MR medical imaging
   and/or
- the transmission (314) and/or the effector shaft (310) is disposed with one or more radio transponders for determining a position and/or orientation of the transmission (314) and/or of the effector shaft (310) by a spatial transponder detector.

The steering guide (300) may be provided with an image capture system (360). The image capture system (360) captures images from the distal tip (361) of the effector shaft (310) so that the effector shaft (310) can be inserted into the bodily tissue or into the inserter (204) elongated member lumen (214) under guidance of the generated images. The images allow, for instance, guidance for manual insertion into the inserter (204). An example of a steering guide (300) provided with an image capture system (360) is shown in **FIG. 44A****.**

The distal tip (361) of the effector shaft (310) may be disposed with an image inlet port (364), through which light reflected from the object enters. An example of an image inlet port (364) is shown in **FIG. 44B****.** The image inlet port (364) may be covered or sealed with a transparent window. The transparent window may be a lens. An image sensor (e.g. CCD, CMOS) is provided onto which the captured image is projected, and converted into electronic signals. The image sensor may be provided in the distal tip (361) of the effector shaft (310), or in the body of the effector shaft (310), or in the body of the transmission (314), or in an ancillary unit (368). Where the image sensor is remote from the image inlet port (364), light may be transmitted using a fibre-optic bundle. Where the image sensor is in the ancillary unit, a connector (366) disposed in the steering guide (300) may be configured to dismountably couple to a cable (367) containing a fibre-optic bundle for transmitting image light from the steering guide to the ancillary unit (368). An example of an ancillary unit (368) unit is shown in **FIG. 44C****,** and the cable (367) attaching it via the connector (366) to the steering guide (300).

The distal tip (361) of the effector shaft (310) may be disposed with one or more light outlet ports (362), through which light emitted by a light source exits. An example of a light outlet port (362) is shown in **FIG. 44B****.** The light leaving the outlet port (362) is used to illuminate the object. The light outlet port (362) may be covered or sealed with a transparent window. The transparent window may be a lens. The light source may be provided in the distal tip (361) of the effector shaft (310), or in the body of the effector shaft (310), or in the body of the transmission (314), or in an ancillary unit (368). Where the light source is remote from the light outlet port (362), light may be transmitted using a fibre-optic cable. Where the light source is in the ancillary unit, a connector (366) disposed in the steering guide (300), may dismountably couple to a cable (367) containing the fibre optical cable for transmitting illumination light from the ancillary unit (368) to the steering guide. An example of an ancillary unit (368) unit is shown in **FIG. 44C****,** and the cable (367) attaching it via the connector (366) to the steering guide (300).

Electronic components of the image capture system (360) such as one or more of processor, memory, I/O ports, power supply, wireless interface, controls may be disposed partially or entirely within the steering guide. Components not present in the steering guide (300) may be disposed in the ancillary unit (368). Signals and/or electrical power may pass from the steering guide via a cable (367) to the ancillary unit (368). The images captured by the image sensor may be displayed on a display, such as a screen, virtual reality viewer, or any device capable of displaying images from electrical signals. The captured images may or may not be stored. Microcameras with a 1 mm outer diameter including illumination are commercially available (e.g. Scoutcam).

The steering guide (300), in particular the handle portion (316) and/or transmission (314) may be provided with one or more (e.g. 2, 3 or more) radiofrequency identification (RFID) tags. The RFID tag allows identification of the steering guide (300). The system may be provided with an RFID tag reading unit comprising an RFID tag reader and processor or interface to a processor configured to prevent operation of the robotic arm when the RFID tag does not match an expected RFID tag stored in the system. As a radiation oncology department may have a plurality of steering guides of different sizes (see e.g. **Tables 1** and **1a** herein) for use with different subjects, the provision of an RFID tag prevents a subject from being provided with the incorrect steering guide (300). The RFID tag may be disposed within a body of the steering guide (300). For instance, it may be provided within a slot provided in the strengthening strut (317) (see e.g. **FIGs. 19****,** **21 to 25****).** For instance, it may be placed within a slot provided in the lateral side of the handle (316). The RFID tag may be rewritable or non-rewritable. The non-rewritable RFID chip allows the steering guide to be attributed only to one single patient with a reduction in work-flow errors. The RA program would, for instance, not allow a steering guide RFID chip to be rewritten and be used in another patient.

The positioning tool (200) may further comprise an inserter (204) *(e.g.* **FIGs. 3, 4A, 4B, 4C****,** **8****,** **10****,** **32** to **35, 38)** having a proximal (40) and distal (20) end. The inserter (204) comprises an elongated member (210) configured for insertion through an entrance to a canal in connection with the bodily tissue. The elongated member (210) may be dimensioned for engaging with the wall of the canal so that movement of the positioning tool (200) causes movement of the canal. The effector shaft (310) may be configured to co-operate with the elongated member (210) such that movements of the steering guide are transferred to the elongated member (210).

The inserter (204) may be non-dismountably (e.g. permanently) attached to the steering guide *(e.g.* **FIG. 4A****).** The inserter (204) elongated member lumen (214) may be configured for repeatable dismountable attachment to the steering guide (300) effector shaft (310) *(e.g.* **FIG. 4B, 4C****).** The effector shaft (310) may be further configured to stiffen at least a substantial part of the elongated member (210) when elongated member (210) is flexible. The inserter (204) may further comprise a guiding strand (218) for guiding the effector shaft (310) into the lumen (214) from outside the entrance to the canal. A distal end (20) of the guiding strand (218) is attached in fixed relation to the lumen (214).

Movement of the canal by the positioning tool and hence inserter (204) moves the bodily tissue of a subject relative to the ionising radiation beam or positional reference for treatment. The bodily tissue may be in the canal, or may be a structure that moves when the canal is moved (e.g. prostate that moves with the rectal canal). The bodily tissue may be positioned in the beam by the inserter (204) in the beam *(e.g.* for treatment), or outside the beam *(e.g.* to reduce damage). A position and/or orientation of the positioning tool and hence inserter (204) is adjustable and fixable.

The elongated member (210) has a proximal (40) and distal (20) end. The elongated member (210) may be rigid (non-flexible). The elongated member (210) may be flexible which allows for better tolerance of the body towards the elongated member (210) which can remain in place in the canal up to 2-3 months during the course of fractionated treatment.

The elongated member (210) is dimensioned for insertion into the canal, in particular into the cervical canal and/or uterus and/or a vaginal vault resection. The elongated member (210) may have a length of 1 to 10 cm. Where the elongated member (210) is configured for positioning in the cervical canal and/or uterus, it may have a length of 1-8 cm and a maximum outer diameter of 3-8 mm. Where the elongated member (210) is configured for positioning in a vaginal vault resection, the elongated member (210) may have a length of 1-5 cm and a maximum outer diameter of 3-8 mm. The diameter of elongated member (210) may be uniform from proximal to distal end or may vary. For instance, the diameter may be larger towards the proximal part and smaller towards the distal part. The change in diameter may be gradual. The change in diameter may be gradual across the length of the elongated member (210). The small distal diameter is more atraumatic when entering the vagina, and a larger diameter towards the proximal part improves rigidity of the elongated member (210). See **Tables 1** and **1a** for preferred dimensions for various medical applications.

The elongated member (210) may be disposed with an elongated member lumen (214). The elongated member lumen (214) is open at the proximal end (40) to allow slidable insertion of the effector shaft (310) of the steering guide (300) prior to treatment. The elongated member lumen (214) may be open or closed at or towards the distal end (20). Where it is open, it can provide a drainage channel (270), or exit port (272) or threaded passage (272,-c) for a dismountable guiding strand (218). Where the elongated member (210) is permanently attached to the effector shaft (310) of the steering guide (300), the elongated member lumen (214) may be absent or occupied by the effector shaft (310) of the steering guide (300).

The steering guide (300) attached to the inserter (204) or elongated member (210) is rigidly attached. The rigid attachment minimises play or backlash between the inserter (204) and handle portion (316) of the steering guide (300).

The distal tip of the elongated member (210) may be atraumatic (e.g. have rounded edges, dome shaped, does not create an incision). The elongated member (210) may have a circular profile perpendicular to its longitudinal axis. The elongated member (210) may comprise an essentially cylindrical form. The elongated member (210) may be disposed with one or more fins. A fin is a protrusion that extends outwards from a surface of the elongated member (210). The fin extends also in a longitudinal direction. It functions to better affix the elongated member (210) to the inner walls of the canal (e.g. cervix), to prevent it from rotating during the manipulation with the steering guide. Preferably the one or more fins are provided within a proximal (40) half of the elongated member (210), for instance within the 2-4 cm of the proximal (40) end.

The elongated member (210) may be a rigid tube. The elongated member (210) may be a flexible tube. An advantage of a flexible tube is greater comfort to the subject while it is worn for the treatment duration (e.g. weeks). A wall of the elongated member (210) may be made from any biocompatible material such as a polymer. Examples of suitable substances include polycarbonate, PEEK, carbon fiber, polyamide, polyimide, polyurethane, or silicone. Examples of substances used to form a rigid elongated member (210) include polycarbonate, PEEK, carbon fiber, polyacrylamide resin reinforced with fibres (e.g. Ixef (Solvay)). Examples of substances used to form a flexible elongated member (210) include polyamide, polyimide, polyurethane, or silicone.

Usually the treatment is first simulated under a CT scan, or a PET CT scan, or under MRI. Later the treatment may involve acquisition of one or more X-rays in the treatment room. It is preferred that the elongated member (210) is made from a material compatible with medical imaging, such as with CT or MRI. The material may or may not be visible on a medical image. Examples of materials visible in CT or CT/PET scan include polycarbonate, PEEK, carbon fiber, polyamide, polyimide, polyurethane, or silicone. For visibility, the material may be mixed with a low percentage of barium sulfate. Examples of materials visible in MRI scan include polycarbonate, PEEK, carbon fiber, polyamide, polyimide, polyurethane, or silicone. The pose (position and/or orientation) of the elongated member (210) may be determined directly from the medical image of the elongated member (210) under MRI or opacified with barium sulfate (under CT or PET-CT). Examples of materials not visible in CT/PET scan include polycarbonate, PEEK, carbon fiber; they are visible under MRI, not visible when not mixed with barium sulfate under CT. Where it is not visible or not sufficiently visible to determine the position and/or orientation of the inserter, the elongated member (210) may be disposed with one or more imaging markers. This is of assistance when performing images using the imaging tools of the linear accelerator.

The inserter (204), in particular the elongated member (210), may be provided with one or more imaging markers (206) that can be identified by a medical image. In **FIG. 3** a pair of imaging markers (206) is provided fixed to an outer surface of the elongated member (210). An imaging marker (206) can be identified by a medical image. An imaging marker (206) may be provided in fixed relation to the elongated member (210), for instance on an inner surface, outer surface or within a body of the elongated member of the elongated member (210). An imaging marker (206) may be a protrusion or an indentation. It may be made from the same material as the elongated member (210), and may be visible on a medical image because of a size difference. An imaging marker (206) may be made from a material different from elongated member (210) for instance a heavy metal such as platinum, platinum iridium, tantalum, tungsten, etc.

The inserter (204), in particular the elongated member (210) may be provided with one or more *(e.g.* 2, 3 or more) position-determining radio transponders (260) whose positions can be determined and optionally tracked using a spatial transponder detector.

The terms position-determining radio transponder and transponder are used interchangeably herein. A transponder is sometimes known as beacon transponder. In **FIG. 3** three transponders (260, a, b, c) are provided fixed to an outer surface of the elongated member (210) at different positions. The same three transponders (260, a, b, c) also act as imaging markers because they are visible on a medical image.

A transponder (260) is a device that emits electromagnetic pulses at a certain frequency that is detectable by the spatial transponder detector - typically comprising a number of spatially separated receivers (coils). The timing of the pulse as detected by a number of spatially separated receivers in a positional transponder reader (see later below) allows the location of the transponder to be accurately determined. The transponder (260) may receive power inductively. Where more than one transponder is present, each transponder may emit a signal at a different frequency. When at least three separately-identifiable transponders (260, a, b, c) are disposed on the inserter (204) at different positions, the orientation of the inserter (204) may also be determined. Examples of such systems are described, for example, in US 9,248,003 B2, and US 9,072,895. The use of transponders reduces the need to align the inserter (204) prior to radiation treatment using medical imaging which reduces exposure to imaging radiation.

Transponders function well below the surface of the subject. All transponders do not need to be placed inside patient's body. For instance, 1 or 2 transponders may be located on the positioning tool (200) on a part that will be inside the patient's body (e.g. on the inserter (204), effector shaft (310) or distal part of the transmission (314) of the steering guide (300) - see later below), and 1 or 2 transponders may be located outside the patient's body (e.g. on the proximal part of the transmission (314) of the steering guide (300)).

According to one aspect:
- at least a part of the inserter (204) or one or more imaging markers borne thereby, is visible by medical imaging, in particular by X-ray medical imaging and/or by MR medical imaging, and/or
- at least a part of the elongated member (210) or one or more imaging markers borne thereby, is visible by medical imaging, in particular by X-ray medical imaging and/or by MR medical imaging,
   and/or
- the inserter (204) and/or elongated member (210) is disposed with one or more radio transponders for determining a position and/or orientation of the inserter (204) and/or elongated member (210) by a spatial transponder detector.

Where the inserter (204) is dismountable from the steering guide (300), a guiding strand (218) may or may not be present. Where the guiding strand (218) is present, it is disposed at least partially within the elongated member lumen (214), and exits at a proximal end therefrom. The effector shaft (310) of the steering guide (300) may comprise a guiding strand passage (312) that receives the guiding strand (218). The guiding strand passage (312) may be a lumen within the effector shaft (310) or a longitudinal groove on the surface of the effector shaft (310). The guiding strand (218) is restrained at or towards a distal end (20) of the guiding strand (218) to limit or prevent sliding of the guiding strand (218) in a proximal direction relative to the lumen (214). This allows tension to be applied to the guiding strand (218) in a proximal direction without release or displacement of the guiding strand (218). The effector shaft (310) guiding strand passage can be threaded through a proximal end of the guiding strand trailing outside the body and guided reliably into the elongated member lumen (214). The guiding strand allows repeatable mounting and dismounting of the steering guide prior to and after simulation and/or radiation treatments. Access to the elongated member lumen (214) is made available despite the elongated member lumen (214) being located *in situ,* for instant, in the cervix.

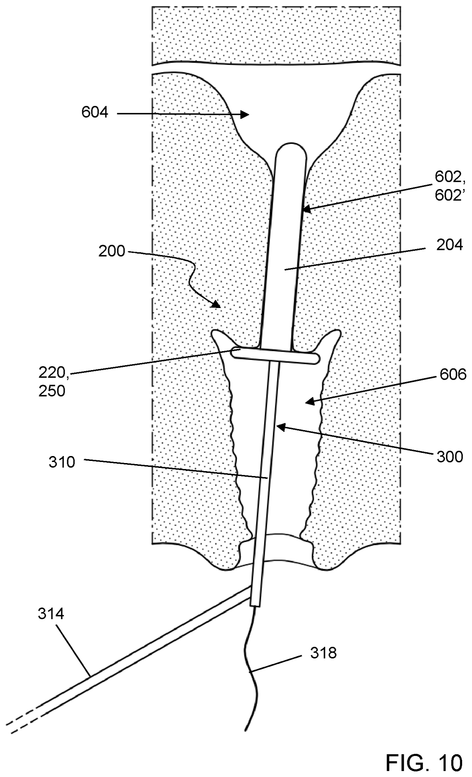

The guiding strand (218) may be a flexible cord (219) (e.g. made up of one more strands) or or an inflation tube (236) or a flaccid tube to be stiffened by a stiffening stylet. The external diameter of the guiding strand (218) is smaller than the internal diameter of the guiding strand passage (312). It is dimensioned to be threaded through the guiding strand passage (312). The guiding strand (218) may have a narrow cross-sectional profile, for instance, 0.1 to 2.5 mm (flexible cord), or 1 to 2.5 mm (inflation tube), or 1 to 5 mm (flaccid tube). It may have a tensile strength to resist tension placed thereon while the effector shaft (310) of the steering guide (300) is inserted into the elongated member lumen (214). It may be non-expandable in a longitudinal direction. Examples of a guiding strand (218) that is a flexible cord (219) is shown in **FIG. 4B****,** and in **FIG. 8** panels A, B, C, D, E, F, O, P, a, b, c, d, e, f, n, o, p and **FIG. 10****.**

The guiding strand (218) may be non-dismountably (e.g. permanently) attached to the inserter (204) or may be dismountably attached to the inserter (204). Where the guiding strand (218) is dismountably attached, preferably such guiding strand is a flexible cord (219).

A non-dismountable attachment to the inserter (204) may be achieved, for instance, during a moulded production of the elongated member (210), by a knotted attachment to a strut (213), by adhesive. A distal end of the guiding strand (218) is preferably attached in fixed relation to the elongated member lumen (214), preferably to a distal-most end of the elongated member lumen (214). Examples of a guiding strand (218) that is non-dismountably attached is shown in **FIG. 4B****,** and in **FIG. 8** panels A, B, C, D, L, M, N.

A dismountable attachment of the guiding strand (218, 219) to the inserter (204) allows both
- a reliable guidance of the effector shaft (310) into the elongated member lumen (214) when the guiding strand (218, 219) is present;
- an insertion of a brachytherapy applicator into the elongated member lumen (214) when the guiding strand (218, 219) is removed (dismounted).

There are situations when a treatment starts with an external radiotherapy programme, and subsequently a brachytherapy treatment is needed. Brachytherapy is well known in the art, and is a process of treatment of a target using an internal ionising radiation source. The radiation source is disposed in a sealed capsule at the end of a flexible cable spooled in an afterloader safe. When required, the cable, having a certain pushability, is fed out from the afterloader into a transfer tube connected to brachytherapy applicator at the treatment target. The radiation source is held in position by or within the brachytherapy applicator for a duration of the treatment, and subsequently withdrawn into the afterloader safe. The elongated member *(210) in situ* may be utilised as part of a brachytherapy applicator which avoids a step of inserting a separate brachytherapy applicator. The elongated member (210) *in situ* may be utilised as a catheter allowing the introduction of a brachytherapy applicator. Where elongated member (210) is already placed adjacent to the target, the guiding strand (218) is dismounted and removed and the applicator introduced into the elongated member lumen (214) and maintained therein in order to irradiate the target.

Where the guiding strand (218, 219) is dismountably attached to the inserter (204), it is typically a flexible cord (219).

According to one example, the dismountable guiding strand (218, 219) is a flexible cord (219) that has an anchoring end (219, b) provided with a stop anchor (219, c). The elongated member lumen (214) may be provided at a distal (20) end with an exit port (272, -a, -b). The guiding strand (218, 219) is disposed through a proximal entrance (214, a) to the elongated member lumen (214), along the elongated member lumen (214) in a distal direction, through the distal exit port (272, -a, -b), and returns in a proximal direction to a proximal end of the inserter (204). The guiding strand (218, 219) has an anchoring end (219, b) and a free end (219, a). The anchoring end (219, b) exits from the exit port (272, -a, -b). The anchoring end (219, b) is provided with a stop anchor (219, c). The stop anchor (219, c) is configured to engage with a reciprocating stop (254) on the inserter (204) for instance on the elongated member (210) or on the stop member (250). The reciprocating stop (254) may be a passageway. The reciprocating stop (254) is disposed at the proximal end of the inserter (204) that is accessible by the specialist when the inserter (204) is *in situ.* The free end (219, a) exits from the proximal entrance (214, a) to the elongated member lumen (214). The free end (219, a) is able to pass unrestricted through the proximal entrance (214, a) to the elongated member lumen (214), exit port (272, -a, -b) and reciprocating stop (254).

Tension applied to the free end (219, a) of the guiding strand (218) engages the stop anchor (219, c) with the reciprocating stop (254), preventing sliding movement of the guiding strand (218) within the elongated member lumen (214). Tension applied to the anchoring end (219, b) of the guiding strand (219) causes sliding movement the guiding strand (218) within the elongated member lumen (214); the free end (218, a) passes through the proximal entrance to the elongated member lumen (214), exit port (270, -a, -b, -c) and reciprocating stop (254) and ultimately dismounts the guiding strand (218) from the inserter (204). Examples of a guiding strand (218) that is dismountable with a stop anchor (219, c) is shown in **FIG. 4D****,** and in **FIG. 8** panels E, F, d, e, f.

According to another example, the dismountable guiding strand (218, 219) is a flexible cord (219) that has a threaded (219, d) distal end. The elongated member lumen (214) may be provided at a distal (20) end with a reciprocating threaded passage (272,-c). Axial rotation of the flexible cord (219) in one direction allows the threaded (219, d) distal end of the guiding strand (218, 219) to engage with the reciprocating threaded passage (272,-c) of the inserter (204) thereby attaching the elongated member (210). Rotation in the other direction releases the flexible cord (219) from the elongated member (210). Examples of a guiding strand (218) with a threaded portion (219, d) that is dismountable is shown in **FIG. 4E, 4F****,** and in **FIG. 8** panel O.

According to another example, the dismountable guiding strand (218, 219) is a flexible cord (219) that is removeable by exertion of a pulling force above a certain threshold. The pulling force may in the range of 1 to 3 kg. In one example, the flexible cord (219) has a breakable portion (219, e) at the distal end where it attaches to the inserter (204), in particular to the elongated member (210). In another example, the flexible cord (219) is attached to the inserter (204) by coupling that pulls apart from the inserter (204). An example of a guiding strand (218) with a breakable portion (219, e) is shown in **FIG. 8** panel P.

The guiding strand (218) that is a flexible cord (219) may be made from any suitable material, preferably non-ferromagnetic, such as nylon, or other polymeric material, or metal such as nitinol. The guiding strand (218) that is a flaccid tube may be made from any suitable material, preferably non-ferromagnetic, such as polymer such as polyamide. The guiding strand (218) that is an inflation tube may be made from any suitable material, preferably non-ferromagnetic, such as polymer such as polyamide, or metal, such as nitinol. The guiding strand (218) may be contain or be coated with an antibacterial agent; examples of antibacterial agent include silver particles, erythromycin, or other antibiotics. Preferably guiding strand (218) is made from a radio-transparent material. Preferably guiding strand (218) is made from a MRI compatible and biocompatible material. A distal end of the guiding strand is preferably attached in fixed relation to the elongated member lumen (214), preferably to a distal-most end of the elongated member lumen (214).

The guiding strand (218) may be an inflation tube (236) that is a tube having a lumen in fluid connection with a distal slide restrictor (220) that is an inflatable balloon assembly (230) - see later below. A lumen of the inflation tube (236) may allow passage of fluid (e.g. liquid, saline) for inflating one or more balloons of the inflatable balloon assembly (230). Examples of a guiding strand (218) that is an inflation tube (236) is shown in **FIG. 8** panels L, M, N k, l, m, **FIGs. 9****,** **34** to **35, 38.** The elongated member (200) may be provided with a crimpable or self-expandable cylindrical body (e.g. a metal mesh (240)) around an outside of a distal end. This allows the practitioner open this metallic mesh inside the canal (for instance inside the uterus (604)) by inflating a balloon (balloon expandable mesh) or by removing a slide member around the mesh (self-expanding stent). The inflatable balloon assembly (230) allows blocking movement of the elongated member inside the uterus. Stitches used to attach the inserter to the entrance to the cervix may become loose over time, when tumors shrink. The presence of distal slide restrictor (220) that is an inflatable balloon assembly (230) prevents the elongated member (210) slipping downwards out from the uterus (604).

The guiding strand (218) may be a flaccid tube (237) that is a tube having a lumen (238) configured to receive a stiffening stylet. The flaccid tube (237) is more flexible in the absence of the stiffening stylet, and is less flexible (more stiff, having more pushability) when the stiffening stylet in inserted in the lumen (238). Examples of a guiding strand (218) that is a flaccid tube (237) is shown in **FIG. 8** panels Q, R, S, T, q, r, t, xxi to xxv. The stiffening stylet is more flexible than the flaccid tube (237). The stiffening stylet may be a metallic or polymeric wire. While the inserter (204) is worn by the subject, the stiffening stylet is absent from the flaccid tube lumen (238). The flaccid tube (237) without stiffening stylet allows for a more comfortable wearing of the inserter as the flaccid tube (237) has increased flexibility, and is more conforming to the changes in shape of the subject during wearing. Prior to insertion of the steering guide, the stiffening stylet is inserter along the flaccid tube (237) lumen (238); this increases the stiffness, and allows the guiding strand passage (312) of the effector shaft (310) of the steering guide (300) to be pushed along the stiffened flaccid tube (237) with a reduction in buckling, and hence provides a faster and less uncomfortable experience for the subject. After the external radiotherapy treatment and/or a simulation of treatment at a certain pose of the inserter (204), the steering guide (300) is removed. The stiffening stylet may be removed after steering guide (300) has been inserter, or may be removed after the steering guide (300) has been removed.

Where the inserter (204) is for the cervical canal (602'), the guiding strand (218) is long enough to exit through the vagina (606). A trailing end of the guiding strand (218) may be fixed with an adhesive pad to the skin, for instance in the groin area of the subject between simulation and radiotherapy treatment and/or between radiotherapy treatment fractions.

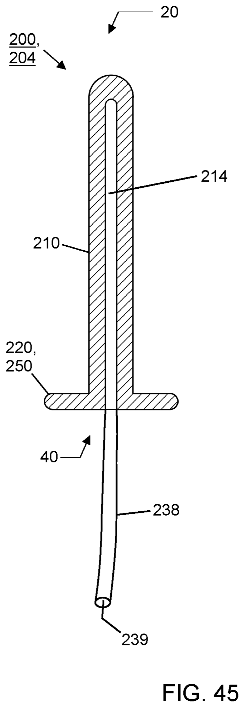

Where the inserter (204) is dismountable from the steering guide (300), the inserter may be provided with a guiding sleeve having a sleeve lumen, wherein the guiding sleeve lumen is configured to receive the steering guide effector shaft (310) and to guide said steering guide effector shaft (310) into the elongated member lumen (214). The guiding sleeve may be attached to the proximal end of the inserter (204) such that the elongated member lumen (214) and the guiding sleeve lumen are continuous. An exemplary guiding sleeve is shown in **FIG. 45****.**

The effector shaft (310) of the steering guide (300) can be inserted into the sleeve lumen of the guiding sleeve, thereby guiding the effector shaft (310) into the elongated member lumen (214).

The guiding sleeve allows repeatable mounting and dismounting of the steering guide prior to and after simulation and/or radiation treatments. Access to the elongated member lumen (214) is made available despite the elongated member lumen (214) being located *in situ,* for instant, in the cervix.

The guiding sleeve may be a thin-walled hollow tube, with flexible walls. The internal diameter of the guiding sleeve is greater than the external diameter of the effector shaft (310). It may have a tensile strength to resist tension placed thereon while the effector shaft (310) of the steering guide (300) is inserted into guiding sleeve. It may be non-expandable in a longitudinal direction. Where the inserter is for the cervix, the guiding sleeve is long enough to exit through the vagina. A trailing end of the guiding sleeve may be fixed with an adhesive pad to the skin, for instance in the groin area of the subject between simulation and radiotherapy treatment and/or between radiotherapy treatment fractions.

The inserter (204) may comprise one or more slide restrictors (220). The elongated member (210) may be provided with one or more slide restrictors (220) configured to reduced or prevent sliding of the elongated member (210) relative to the canal as shown, for instance in **FIGs. 3, 4A, 4B, 4C, 4D****,** **8****,** **10** and **33** to **35, 38.** The slide restrictor (220) may engage with a wall of the canal by friction, for instance, or abut with an entrance or exit to the canal. The slide restrictor (220) may be attached in fixed relation to the elongated member (210). The slide restrictor (220) may be disposed at a discrete longitudinal position on the elongated member (210). Examples of a slide restrictor include an inflatable balloon assembly (230), an expandable stent (240), and a stop member (250).

There may be two slide restrictors (220) each disposed at a different longitudinal position on the elongated member (210). One slide restrictor (220) may be disposed at the proximal end (40) of the elongated member (210) the other may be disposed at the distal end (20) of the elongated member (210). One slide restrictor (220) may be a stop member (250), the other slide restrictor may be an inflatable balloon assembly (230) or an expandable stent (240). Such arrangement allows the two slide restrictors (220) to flank tissue disposed between an entrance and exit of the canal effective clamp the elongated member (210) thereagainst. Two slide restrictors (220) are disposed at either end of the elongated member (210) in **FIGs. 8** (see combination of Tables 2a to 2f), **34, 35** and **38.** Preferably one of the two slide restrictors (220) is a proximal stop member (250). The arrangement of two slide restrictors (220) may contribute to increase the accuracy of uterus positioning, by reducing elongated member (210) freedom inside the uterine canal (604). In addition, it solves a problem observed when the proximal stop member (250) is sutured to the cervix; after a number of fractions, the cervical tumour begins to shrink and the sutures can become looser, allowing dislodgment of the elongated member; this may become important when the effector shaft (310) is withdrawn after a fraction. By inflating a distally placed the balloon assembly (230) or expandable stent (240), the elongated member (210) becomes fixed inside uterine canal (604) and resistant to tension applied for instance during withdrawal of the effector shaft (310).

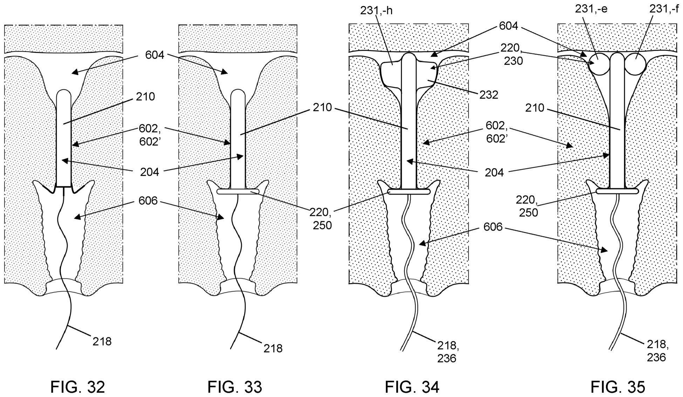

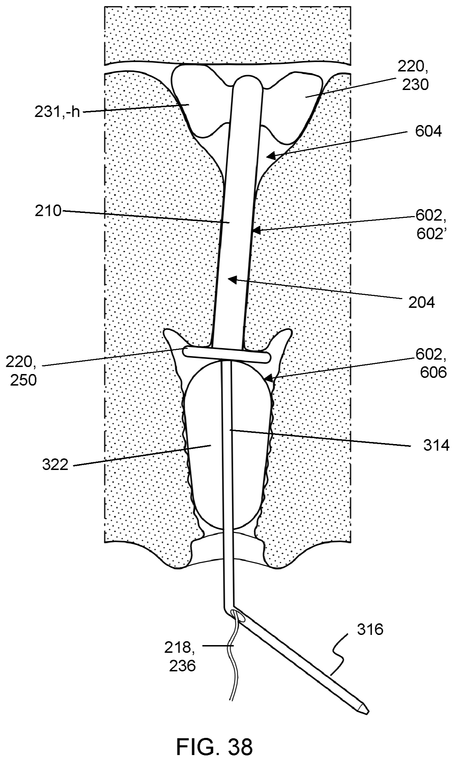

A slide restrictor (220) may be an inflatable balloon assembly (230). The inflatable balloon assembly (230) may comprise one or more (e.g. 2, 3, 4) inflatable balloons (231,-a to -i) provided around at least a distal part (20) of the elongated member (210), as exemplified in **FIGs. 8** (panels i, ii, iii, vi, vii, viii, xi, xii, xiii, xvi, xvii, xviii, xxi, xxii, xxiii, L, M, N), **28, 33, 35, 38.** Two inflatable balloons (231,-a, 231,-b) may be provided at the distal end of the elongated member (210), optionally arranged diametrically (e.g. **FIGs. 8** (panel i, vi, xi, xvi, xxi), **35).** One inflatable balloon may be provided at the distal end of the elongated member (210), optionally having an annular form e.g. conical **(****FIG. 8****,** panel ii, vii, xii, xvii, xxi 231,-c; **FIG. 8****,** panel M 231,-g) or barrel **(****FIG. 8****,** panel iii, viii, xiii, xviii, xxiii 231,-d; **FIG. 8****,** panel N 231,-h; **FIG. 34****;** **FIG. 38****).** A wall of the inflatable balloon (231,-a to -i) may be made from any suitable expandable or non-expandable material. Examples of expandable materials include polyurethane, any elastic polymer, thin film polymers (nylon, compliant polyamide or others) or other elastomers. The inflatable balloon (231,-a to -i) may have a limited maximum inflation size, whereby inflation at or above the maximum inflation size is resisted. Limited maximum inflation size may be achieved by forming the balloon wall from a non-expandable material such as PET, semi-compliant or non-compliant polyamide.

In fluid connection with an inflatable balloon (231,-a to -i) may be an inflation lumen (234). The inflation lumen (234) may extend via an inflation tube (236) such as a catheter or flexible tubing in a proximal (40) direction. The inflation lumen (232) may be formed within the guiding strand (218) as explained earlier; accordingly the guiding strand (218) may be an inflation tube (236) as shown, for instance, in **FIG. 8** panels K, L, M. The inflation tube (236) may alternatively be provided outside the elongated member (210) as shown, for instance, in **FIG. 8** panels i, ii, iii, vi, vii, viii, xi, xii, xiii, xvi, xvii, xviii. The inflation tube (236) may extend in a proximal direction outside the elongated member (210).

The inflation lumen (234) allows inflation of the inflatable balloon lumen (232) from outside the bodily canal after the elongated member (210) has been positioned. The inflatable balloon (231, -a to -i) may be deflated after treatment by release of inflation fluid (e.g. saline or sterile water) from the balloon lumen (232) via the inflation lumen (234). The inflation fluid may contain contrast agent. **FIGs. 34** to **35****,** **38** exemplify the positioning tool (200) provided with an inflatable balloon assembly (230) wherein the inflatable balloons (231-e, 231-f, 231-h) are located in the uterine canal (604), and inflated to prevent or reduce sliding movement of the elongated member (210). Also shown is an inflation tube (236) for controllable inflation and deflation of the balloon (231-e, 231-f, 231-h). The inflation tube (236) is the guiding strand (218), as described previously.

The inflatable balloon (231, -a to -i) may also be used to prevent the elongated member being ejected from the uterine canal during the effector shaft retrieval and to improve the positioning of the uterus. In this case, once the effector shaft (310) of the steering guide (300) has been introduced inside the elongated member (210), the balloon (231-a to -i) may be inflated. This may contribute to increase the accuracy of uterus positioning, by reducing elongated member (210) freedom inside the uterine canal (604). In addition, once the cervical tumour begins to shrink, the stitches fixing the elongated member (210) on the cervix can become looser and allow possible elongated member dislodgment. By inflating the balloon, the elongated member will be automatically fixed inside uterine canal. The balloon (231-a to -i) may be inflated permanently, during the treatment duration (e.g. 1-8 weeks), in order to prevent elongated member (210) from dislodgment from uterine canal (604), even between fractions.

A slide restrictor (220) may be an expandable stent (240). The expandable stent (240) may be provided around at least a distal part of the elongated member (210), as exemplified in **FIG. 8** **panels v, x, xv, xx, xxv.** It may be made from any suitable expandable material such as CoCr alloy, phynox, nitinol, a biodegradable metal such as magnesium alloy, zinc alloy, iron, biodegradable polymer. The expandable stent may be self-expanding or balloon expandable. The stent may be contracted after treatment by covering with a slidable sheath that restricts the outer profile. Expandable stents are well known in the art and typically have a tubular form, the walls having a mesh construction, cut from a tube or made from braided wire, that expand radially.

The stop member (250) may be provided at the proximal end of the elongated member (210), as exemplified in FIG. 8 panels a, b, d, e, g, h, k, l, n, o. The stop member acts as a distance limiter to prevent sliding of the elongated member (210) further into the canal as it abuts with the canal entrance. The stop member is disposed at the proximal end of the elongated member (210). The stop member protrudes from the outer surface of the elongated member (210). The stop member (250) is provided in fixed (non-moving) relation to the elongated member (210). The stop member (250) may be rigid. The stop member may comprise an annular structure. It may be formed from the same material as the member (210) or it may be formed from a different material. The stop member (250) may be provided with one or more suture channels (252). The suture channels allow the stop member to be sutured to the entrance to the canal e.g. to the banks of the cervix. **FIGs. 3, 4A to 4E****,** **10** and **33** to **35****,** **38** exemplify the inserter (204) provided with a stop member (250) at the proximal end of the elongated member (210). The positioning tool (200) is located in the cervical canal (602') or in the cervical canal (602') and uterine canal (604), and abutting of the stop member against the bank of the cervical canal (602') prevents or reduces sliding movement of the elongated member (210).

A slide restrictor (220) may be a region at a distal part of the elongated member (210) containing one or more distal protrusions. The protrusion may be a lateral protrusion (245), as exemplified in **FIG. 8** panel iv, ix, xiv, xix, xxv. The protrusion may be an annular ring or segment. The distal protrusion (245) acts as a restrictor to prevent or reduce sliding of the elongated member (210) further into the canal as it abuts with the canal wall. The distal protrusion (245) is disposed at the distal end of the elongated member (210). The distal protrusion (245) protrudes from the outer surface of the elongated member (210). The distal protrusion (245) is provided in fixed (non-moving) relation to the elongated member (210). The distal protrusion (245) may be rigid. The distal protrusion (245) may comprise an annular structure. It may be formed from the same material as the member (210) or it may be formed from a different material.

The elongated member (210) may be provided with one or more drainage channels (270, -a, - b, -c) at the distal end (20) as shown for instance in **FIG. 8** panels B, C, E, F, H, J, O, R, S, T. The drainage channel fluidly connects the elongated member lumen (214) with an exterior surface of the elongated member (210). A drainage channel (270, -a, -b) may be provided towards the distal end of the elongated member (210). The distal terminal end of the elongated member (210) may be open to the elongated member lumen (214), thereby forming a drainage channel (270, -b) *(e.g.* **FIG. 8** panels C, F, J). The guiding strand (218) where present may be attached to a strut (213) attached to the elongated member lumen (214) that does not occlude the passage of fluid (e.g. **FIG. 8** panel C). A drainage channel (270, -c) may be provided on a side wall of elongated member lumen (214). The drainage channel(s) (270) allow drainage of fluids that may arise inside the uterine canal to be safely removed. The fluids exit the proximal end of the elongated member lumen (214) *e.g.* into the vaginal passage (606) after simulation and/or radiotherapy treatment, when effector shaft is not inside the elongated member. A drainage channel allows draining of liquid from the canal, significantly reducing a risk of infection. A drainage channel may also function as an exit port for the guiding strand (218), and *vice versa.* A drainage channel may also function as threaded passage (272,-c) for a dismountable guiding strand (218) and *vice versa.*

The inserter may be provided with any one of a number of different arrangements of drainage channels (270), exit ports (272), threaded passage (272,-c), side restrictors (220), and guiding strand (218). For instance, a distal slide restrictor, a proximal slide restrictor, an exit port, and one or more drainage channels may be provided or absent. Where both drainage channel and distal slide restrictor (i to xv) are present, the drainage channels may flank one of both ends of the distal slide restrictor.

In **FIG. 8****,** any one the elongated members (A, B, C, D) may be combined with a proximal slide restrictor (a, b) or may not be combined with a proximal slide restrictor (c); the elongated members (A, B, C, D) may or may not contain a distal slide restrictor (i, ii, iii, iv, v).

Further, in **FIG. 8****,** any one the elongated members (E, F) may be combined with a proximal slide restrictor (d, e) or may not (f) be combined with a proximal slide restrictor; the elongated members (E, F) may or may not contain a distal slide restrictor (vi, vii, viii, ix, x); the guiding strand is removeable.

Further, in **FIG. 8****,** any one the elongated members (G, H, J, K) may be combined with a proximal slide restrictor (g, h) or may not (j) be combined with a proximal slide restrictor; the elongated members (G, H, J, K) may or may not contain a distal slide restrictor (xi, xii, xiii, xiv, xv); the guiding strand is absent.

Further, in **FIG. 8****,** any one the elongated members with distal slide restrictors (L, M, N) may be combined with a proximal slide restrictor (k, l, m), or may not be combined with a proximal slide restrictor (m); the guiding strand is an inflation tube.

Further, in **FIG. 8****,** any one the elongated members (O, P) may be combined with a proximal slide restrictor (n, o) or may not be combined with a proximal slide restrictor (p); the elongated members (O, P) may or may not contain a distal slide restrictor (xvi, xvii, xviii, xix, xx); the guiding strand is detachable.

Further, in **FIG. 8****,** any one the elongated members (Q, R, S, T) may be combined with a proximal slide restrictor (q, r) or may not be combined with a proximal slide restrictor (s); the elongated members (Q, R, S, T) may or may not contain a distal slide restrictor (xxi, xxii, xxiii, xix, xx); the guiding strand is a flaccid tube.

Where both a distal slide restrictor (i to xxv) and drainage channels or exit ports or are present, the distal slide restrictor (i to xxv) may be disposed within a region (e.g. 211) of the elongated member (210) that does not block the drainage channels.

Examples of different combinations is provided in **Tables 2a to 2f** below. Exemplary elements referred to in **Tables 2a to 2e** are depicted in **FIG. 8****.**

**Table 2a exemplary combinations of inserter (204) features when the guiding strand (218) is a flexible cord (219). Key: A- Elongated member, no drainage channels, B - Elongated member drainage channels at distal end, C - Elongated member open at distal end, D - Elongated member drainage channels at distal end at side wall; a - proximal stop member (slide restrictor) with suture channels, b - proximal stop member (slide restrictor) without suture channels, c - no proximal stop member (slide restrictor); i - distal pair of balloons (slide restrictor), ii - distal conical balloon (slide restrictor), iii - distal barrel balloon (slide restrictor), iv - distal protrusion (slide restrictor), v - expandable stent (slide restrictor), GS guiding strand. FIG. 8 shows exemplary implementations of each feature (A, B, C, D, a, b, c, i, ii, iii, iv, v).**

| **(+) proximal slide restrictor, (+) suture channels** | | | | **(+) proximal slide restrictor, (-) suture channels** | | | | **(-) proximal slide restrictor** | | | |
|---|---|---|---|---|---|---|---|---|---|---|---|
| | | | | | | | | **(+) GS** | | | |
| **(+) GS** | | | | **(+) GS** | | | | | | | |
| Aa | Ba | Ca | Da | Ab | Bb | Cb | Db | Ac | Bc | Cc | Dc |
| Aa-i | Ba-i | Ca-i | Da-i | Ab-i | Bb-i | Cb-i | Db-i | Ac-i | Bc-i | Cc-i | Dc-i |
| Aa-ii | Ba-ii | Ca-ii | Da-ii | Ab-ii | Bb-ii | Cb-ii | Db-ii | Ac-ii | Bc-ii | Cc-ii | Dc-ii |
| Aa-iii | Ba-iii | Ca-iii | Da-iii | Ab-iii | Bb-iii | Cb-iii | Db-iii | Ac-iii | Bc-iii | Cc-iii | Dc-iii |
| Aa-iv | Ba-iv | Ca-iv | Da-iv | Ab-iv | Bb-iv | Cb-iv | Db-iv | Ac-iv | Bc-iv | Cc-iv | Dc-iv |
| Aa-v | Ba-v | Ca-v | Da-v | Ab-v | Bb-v | Cb-v | Db-v | Ac-v | Bc-v | Cc-v | Dc-v |

**Table 2b exemplary combinations of inserter features when the guiding strand is dismountable. Key: E - Elongated member with exit port as passage, F - Elongated member with exit port open at distal end; d - proximal stop member (slide restrictor) with suture channels and reciprocating stop, e - proximal stop member (slide restrictor) without suture channels and with reciprocating stop, f - no proximal stop member (slide restrictor) and elongated member with reciprocating stop; vi - distal pair of balloons (slide restrictor), vii - distal conical balloon (slide restrictor), viii - distal barrel balloon (slide restrictor), ix - distal protrusion (slide restrictor), x - expandable stent (slide restrictor), GS guiding strand. FIG. 3 shows exemplary implementations of each feature (E, F, d, e, f, vi, vii, viii, ix, x).**

| **(+) proximal slide restrictor** | | **(+) proximal slide restrictor** | | **(-) proximal slide restrictor** | |
|---|---|---|---|---|---|
| **(+) reciprocating stop** | | **(+) reciprocating stop** | | **(+) reciprocating stop** | |
| **(+) suture channels** | | **(-) suture channels** | | **(+) GS** | |
| **(+) GS** | | **(+) GS** | | | |
| Ed | Fd | Ee | Fe | Ef | Ff |
| Ed-vi | Fd-vi | Ee-vi | Fe-vi | Ef-vi | Ff-vi |
| Ed-vii | Fd-vii | Ee-vii | Fe-vii | Ef-vii | Ff-vii |
| Ed-viii | Fd-viii | Ee-viii | Fe-viii | Ef-viii | Ff-viii |
| Ed-ix | Fd-ix | Ee-ix | Fe-ix | Ef-ix | Ff-ix |
| Ed-x | Fd-x | Ee-x | Fe-x | Ef-x | Ff-x |

**Table 2c exemplary combinations of inserter features when the guiding strand is absent. Key: G- Elongated member, no drainage channels, H - Elongated member drainage channels at distal end, J - Elongated member open at distal end, K - Elongated member drainage channels at distal end at side wall; g - proximal stop member (slide restrictor) with suture channels, h - proximal stop member (slide restrictor) without suture channels, j - no proximal stop member (slide restrictor); xi - distal pair of balloons (slide restrictor), xii - distal conical balloon (slide restrictor), xiii - distal barrel balloon (slide restrictor), xiv - distal protrusion (slide restrictor), xv - expandable stent (slide restrictor), GS guiding strand. FIG. 8 shows exemplary implementations of each feature (G, H, J, K, g, h, j, xi, xii, xiii, xiv, xv).**

| **(+) proximal slide restrictor, (+) suture channels** | | | | **(+) proximal slide restrictor, (-) suture channels** | | | | **(-) proximal slide Restrictor** | | | |
|---|---|---|---|---|---|---|---|---|---|---|---|
| **(-) GS** | | | | **(-) GS** | | | | **(-) GS** | | | |
| Gg | Hg | Jg | Kg | Gh | Hh | Jh | Kh | Gj | Hj | Jj | Kj |
| Gg-xi | Hg-xi | Jg-xi | Kg-xi | Gh-xi | Hh-xi | Jh-xi | Kh-xi | Gi-xi | Hi-xi | Jj-xi | Ki-xi |
| Gg-xii | Hg-xii | Jg-xii | Kg-xii | Gh-xii | Hh-xii | Jh-xii | Kh-xii | Gi-xii | Hi-xii | Jj-xii | Ki-xii |
| Gg-xiii | Hg-xiii | Jg-xiii | Kg-xiii | Gh-xiii | Hh-xiii | Jh-xiii | Kh-xiii | Gj-xiii | Hj-xiii | Jj-xiii | Kj-xiii |
| Gg-xiv | Hg-xiv | Jg-xiv | Kg-xiv | Gh-xiv | Hh-xiv | Jh-xiv | Kh-xiv | Gi-xiv | Hi-xiv | Ji-xiv | Ki-xiv |
| Gg-xv | Hg-xv | Jg-xv | Kg-xv | Gh-xv | Hh-xv | Jh-xv | Kh-xv | Gi-xv | Hi-xv | Ji-xv | Ki-xv |

**Table 2d exemplary combinations of inserter (204) features when the guiding strand (218) is a inflation tube (236). Key: L - Elongated member with distal pair of balloons (slide restrictor), M - Elongated member with distal conical balloon (slide restrictor), N - Elongated member with distal barrel balloon (slide restrictor); k - proximal stop member (slide restrictor) with suture channels, l - proximal stop member (slide restrictor) without suture channels, m - no proximal stop member (slide restrictor). FIG. 8 shows exemplary implementations of each feature (L, M, N, k, l, m).**

| **(+) proximal slide restrictor, (+) suture channels** | **(+) proximal slide restrictor, (-) suture channels** | **(-) proximal slide restrictor, (+) GS inflation tube** |
|---|---|---|
| **(+) GS inflation tube** | **(+) GS inflation tube** | |
| Lk | LI | Lm |
| Mk | MI | Mm |
| Nk | NI | Nm |

**Table 2e exemplary combinations of inserter features when the guiding strand is dismountable. Key: O - Elongated member with threaded passage (272,-c), P - Guiding strand attached to elongated member with breakable connection; n - proximal stop member (slide restrictor) with suture channels, o - proximal stop member (slide restrictor) without suture channels, p - no proximal stop member (slide restrictor); xvi - distal pair of balloons (slide restrictor), xvii - distal conical balloon (slide restrictor), xviii - distal barrel balloon (slide restrictor), xix - distal protrusion (slide restrictor), xx - expandable stent (slide restrictor), GS guiding strand. FIG. 8 shows exemplary implementations of each feature (O, P, n, o, p, xvi, xvii, xviii, xix, xx).**

| **(+) proximal slide restrictor, (+) suture channels** | | **(+) proximal slide restrictor, (-) suture channels** | | **(-) proximal slide restrictor** | |
|---|---|---|---|---|---|
| | | | | **(+) GS detachable: threaded (O) or pullable (P)** | |
| **(+) GS detachable: threaded (O) or pullable (P)** | | **(+) GS detachable: threaded (O) or pullable (P)** | | | |
| On | Pn | Oo | Po | Op | Pp |
| On-xvi | Pn -xvi | Oo -xvi | Po -xvi | Op -xvi | Pp -xvi |
| On-xvii | Pn -xvii | Oo -xvii | Po -xvii | Op -xvii | Pp -xvii |
| On-xviii | Pn -xviii | Oo -xviii | Po -xviii | Op -xviii | Pp -xviii |
| On-xvix | Pn -xvix | Oo -xvix | Po -xvix | Op -xvix | Pp -xvix |
| On-xx | Pn -xx | Oo -xx | Po -xx | Op -xx | Pp -xx |

**Table 2f exemplary combinations of an inserter (204) features when the guiding strand (218) is a flaccid tube (237). Key: Q- Elongated member, no drainage channels, R - Elongated member drainage channels at distal end, S - Elongated member open at distal end, T - Elongated member drainage channels at distal end at side wall; q - proximal stop member (slide restrictor) with suture channels, r - proximal stop member (slide restrictor) without suture channels, s - no proximal stop member (slide restrictor); xxi - distal pair of balloons (slide restrictor), xxii - distal conical balloon (slide restrictor), xxiii - distal barrel balloon (slide restrictor), xxiv - distal protrusion (slide restrictor), xxv - expandable stent (slide restrictor). FIG. 3 shows exemplary implementations of each feature (Q, R, S, T, q, r, s, xxi, xxii, xxiii, xxiv, xxv).**

| **(+) proximal slide restrictor, (+) suture channels** | | | | **(+) proximal slide restrictor, (-) suture channels** | | | | **(-) proximal slide restrictor, (+) GS flaccid tube** | | | |
|---|---|---|---|---|---|---|---|---|---|---|---|
| **(+) GS flaccid tube** | | | | **(+) GS flaccid tube** | | | | | | | |
| Qq | Rq | Sq | Tq | Qr | Rr | Sr | Tr | Qs | Rs | Ss | Ts |
| Qq-xxi | Rq-xxi | Sq-xxi | Tq-xxi | Qr-xxi | Rr-xxi | Sr-xxi | Tr-xxi | Qs-xxi | Rs-xxi | Ss-xxi | Ts-xxi |
| Qq-xxii | Rq-xxii | Sq-xxii | Tq-xxii | Qr-xxii | Rr-xxii | Sr-xxii | Tr-xxii | Qs-xxii | Rs-xxii | Ss-xxii | Ts-xxii |
| Qq- xxiii | Rq- xxiii | Sq- xxiii | Tq- xxiii | Qr- xxiii | Rr- xxiii | Sr- xxiii | Tr- xxiii | Qs- xxiii | Rs- xxiii | Ss- xxiii | Ts- xxiii |
| Qq- xxiv | Rq- xxiv | Sq- xxiv | Tq- xxiv | Qr- xxiv | Rr- xxiv | Sr- xxiv | Tr- xxiv | Qs- xxiv | Rs- xxiv | Ss- xxiv | Ts- xxiv |
| Qq-xxv | Rq-xxv | Sq-xxv | Tq-xxv | Qr-xxv | Rr-xxv | Sr-xxv | Tr-xxv | Qs-xxv | Rs-xxv | Ss-xxv | Ts-xxv |

Movement of the canal by the positioning tool (200) causes movement of bodily tissue of the subject in relation to the external ionising radiotherapy beam, in particular in relation to a beam intersection volume of an external ionising radiotherapy beam. A position and/or orientation (pose) of the positioning tool is adjustable and fixable for a duration of at least a part of the treatment session, which stably fixed the position of the bodily tissue relative to the ionising radiotherapy beam. **FIGs. 36** and **37** exemplify the invention as described elsewhere herein. Movement of the canal by the positioning tool (200) may bring the bodily tissue of a subject into alignment with an ionising radiotherapy beam, e.g. with an ionising radiotherapy isodose volume, as it has been defined during patient simulation under CT scan or MR **(****FIG. 36****).** Alternatively, movement of the canal by the positioning tool (200) may move and/or fix the bodily tissue of a subject away from an ionising radiotherapy beam, e.g. to protect that structure **(****FIG. 37****).**

The canal (602) can be accurately moved and fixed by the positioning tool (200) in order to change and fix the position of canal and bodily tissue relative to the external ionising radiotherapy beam. The bodily tissue may be in the canal, or may be a structure that moves when the canal is moved (e.g. prostate that moves with the rectal canal or uterus that moves with cervical canal).

The canal (602) of the subject is preferably a canal (passageway) of a natural bodily structure such as cervical canal (602') and/or uterus (604), vagina (606), rectal canal, or anal canal, or a canal made inside a vaginal vault by resection. In **FIGs. 32** to **35****,** **38****,** the positioning tool (200) is located within the cervical canal (602'). The canal may be a canal formed by surgery e.g. in the vaginal vault or in a tissue mass such as a breast.

The bodily tissue of a subject in connection with the canal refers to tissue that is moveable by changing the spatial position and/or orientation of the canal *i.e.* by changing the spatial position and/or orientation (pose) of the positioning tool (200). In other words, the canal is in moveable connection with the bodily tissue. The bodily tissue may be part of a wall of the canal, or a different tissue structure whose position and/or orientation is affected by movement of the canal. For instance, the position and/or orientation of the bladder, vagina, uterus and rectum can be changed by changing the changing the spatial position and/or orientation (pose) of the inserter (204) or effector shaft (310) in the cervical canal. For instance, the position and/or orientation of the anus, rectum, lower colon, bladder, prostate, vagina, cervix, uterus can be changed by changing the changing the spatial position and/or orientation (pose) of the inserter (204) or effector shaft (310) in the rectal canal.

The bodily tissue (608) may be tissue that is a target (610) of treatment (e.g. a tumour) and is to be brought into the external ionising radiotherapy beam for exposure to the beam; accordingly, the target(s) for receiving treatment is (are) highly accurately positioned, allowing receipt of maximal dose and reducing damage to healthy structures. Alternatively, the bodily tissue may be tissue that is to be moved out of the external ionising radiotherapy beam thereby avoiding exposure to the beam; accordingly, healthy tissue can be displaced away from a target, allowing a more isolated exposure.

The bodily tissue (608) may be a tissue or tissue of an organ. The bodily tissue may be a tissue structure comprised in the pelvic region such the cervix uteri, corpus uteri, rectum, bladder, vagina that can be moved by changing the position and/or orientation of the vagina and/or cervix and/or uterus. The bodily tissue may be tissue structures comprised in the rectal region such the anus, rectum, lower colon, bladder, prostate, vagina, cervix, uterus that can be moved by changing the position and/or orientation of the rectum. The bodily tissue may be a tissue structure comprised in the pelvic region such as the bladder in female subjects that can be moved by changing the position of the positioning tool (200) placed inside the bladder. It is appreciated that the positioning tool (200) may be used in the treatment of multiple tissues that lie adjacent to the canal.

The system, positioning tool and method describe herein may be used to treat one or more tumours present in the bodily tissue.

A treatment pose corresponds a spatial alignment by the positioning tool (200) of the bodily tissue of the subject (50) on the radiotherapy treatment table (512) in relation to an ionising radiation beam emitted by an ionising-radiation treatment head (518) during an external radiotherapy treatment session, in particular relative to the positional reference during an external radiotherapy treatment session.

The RA arm (400, 400a) moves the positioning tool (200) to one or more treatment poses relative to the subject (50) or relative to the subject (50) or treatment table (512) when the subject is accurately aligned on the treatment table (512). At each treatment pose, the subject is exposed to the ionising-radiation beam emitted by an ionising-radiation treatment head (518). When the bodily tissue is the target, the ionising-radiation beam passes through the bodily tissue. When the bodily tissue is to be protected, the treatment pose causes the bodily tissue to at least partially avoid exposure to the ionising-radiation beam.

There may be one treatment pose, for instance when the target can be sufficiently exposed by directing the bodily tissue into one position. There may be two or more treatment poses, which allow to direct the bodily tissue into a plurality of different positions, split and spread the dose each day on different surrounding healthy tissues which further contributes to reduce side effects.

A treatment pose is derived from a corresponding pose of the positioning tool (200) during simulation *i.e.* in relation to the subject (50) disposed on the simulation table (522), or in relation to the simulation table (522) when the subject is accurately aligned on the simulation table (522). Each pose of the positioning tool (200) during simulation is known as an empirically-determined simulation pose of the positioning tool (200). Each empirically-determined simulation pose is determined from a medical image of the positioning tool (*in situ*) acquired while the subject is on the simulation table (522); the pose or poses of the positioning tool (200) that move and/or fix bodily tissue target for optimal exposure to the ionising radiation beam or that move and/or fix bodily tissue away from the ionising radiation beam are recorded. The pose or poses are later transferred to the RA (400, 400a) mounted in relation to the radiotherapy treatment table (512).

The one or more pre-determined treatment poses may be determined from one or more empirically-determined simulation poses of the positioning tool (200).

The one or more pre-determined treatment poses may be determined by:
- receiving (a) a set of simulation-determined parameters comprising one or more empirically-determined simulation poses of the positioning tool (200) with respect to a positional reference such as a simulation of a beam intersection volume, a simulation of an isocentre, or a bony pelvis of the subject placed on and in known position with a simulation table (522), and
- receiving (b) a positional relationship (SPx,y,z) of the RA base end (422, 422b) of the RA mounted on the simulation table (522) with respect to a positional reference such as a simulation of a beam intersection volume, a simulation of an isocentre, or a bony pelvis of the subject placed on and in known position with a simulation table (522),
- receiving (c) data on a positional relationship (TPx,y,z) of the RA base end (422, 422a) of the RA mounted on the radiotherapy treatment table (512) with respect to a positional reference such as a beam intersection volume, an isocentre, or a bony pelvis of the subject placed on and in known position with the radiotherapy treatment table (512),
- determining from (a), (b) and (c) one or more treatment pose of the positioning tool (200) that corresponds with the one or more empirically-determined simulation poses of the positioning tool (200).

The same set of simulation-determined parameters may be used to determine the one or more treatment poses for at least 1, 2 or preferably all of the external radiotherapy treatment sessions. The processing unit (440) may be configured for this.

The processing unit (440) may be configured to determine from (a), (b) and (c) the one or more treatment poses of the positioning tool (200) relative to the positional reference.

Each of the one or more treatment poses of the positioning tool (200) may be fixed during a period of continuous or pulsed exposure to ionising radiation beam. The processing unit (440) may be configured for this.

As mentioned earlier, the positioning tool (200) may be provided with transponders (260, 352) and/or imaging markers (206, 350) to allow realtime capture of the positioning tool (200) pose during simulation or treatment. The transponders (260, 352) may be provided on the inserter (204) and/or steering guide (300). The imaging markers (206, 350) may be provided on the inserter (204) and/or steering guide (300). On the steering guide, transponders and/or imaging markers may be provided on the effector shaft (310) and/or on or inside the transmission (314) and/or on or inside the handle portion (316) and/or on the inflatable transmission balloon (322) where present.

The transponders (260, 352) and/or imaging markers (206, 350) allow real-time guidance of the positioning tool (200) during treatment so that it aligns with a reference pose determined during simulation. The guidance provided may be manual, semi-automatic or automatic by a robotic arm. The transponders (260, 352) and/or imaging markers (206, 350) allow the position and/or orientation of the effector shaft (310) or inserter (204) to be guided, changed and fixed in real-time according to the position and orientation information captured by the spatial transponder detector or imaging unit. For instance, a closed feed-back loop, wherein a continuous input is the pose of the effector shaft (310) or inserter (204) as determined by from the one or more (e.g. 2, 3 or more) position-determining radio transponders, may provide guidance to bring the pose of the effector shaft (310) or inserter (204) into agreement with the pose determined during simulation. This can allow a fine-tuning of the positioning tool (200) pose during simulation or treatment *in situ.*

The real-time guidance may be manual, providing information (e.g. graphical, audible, force-feedback) to guide the operator to manually move the positioning tool (200). In this scenario, the steering guide (300) may be attached by the handle portion (316) to a positioning device that is manually controllable (e.g. the robotic arm operating in a manual zero-gravity mode).

The same transponders and/or imaging markers and manual control may also allow capture and storage of the pose of the steering guide (300) and hence of the effector shaft (310) during simulation.

The real-time guidance may be automatic, providing information to the robotic arm, automatically moving the positioning tool (200) by activation of the joints of the robotic arm.

The real-time guidance may be semi-automatic, providing information to the robotic arm and to the operator, to allow partial automatically and partial manual moving of the positioning tool (200).

Transponders and imaging markers function well below the surface of the subject.

All transponders do not need to be placed inside patient's body. The transponders located on or inside the positioning tool (200) do not need to be all located inside the body. For instance, 1 or 2 transponders may be located on or inside the positioning tool (200) on a part that will be inside the patient's body (e.g. on the inserter (204), effector shaft (310) or on or inside the distal part of the transmission (314) of the steering guide (300)), and 1 or 2 transponders may be located outside the patient's body (e.g. on or inside the proximal part of the transmission (314) of the steering guide (300)).

All imaging markers do not need to be placed inside patient's body. The imaging markers located on or inside the positioning tool (200) do not need to be all located inside the body. For instance, 1 or 2 imaging markers may be located on or inside the positioning tool (200) on a part that will be inside the patient's body (e.g. on the inserter (204), effector shaft (310) or on or inside the distal part of the transmission (314) of the steering guide (300)), and 1 or 2 imaging markers may be located outside the patient's body (e.g. on or inside the proximal part of the transmission (314) of the steering guide (300)).

With certain prior art techniques, transponders may be implanted inside the body to track the position of an organ. The present positioning tool (200) avoids the need for implantation; transponders are located on or inside a part of the positioning tool (e.g. inserter (204), steering guide (300)) outside the body, and are introduced temporarily inside the body for only a few minutes during each fraction. These transponders track an object inside the body, they are located on the object, they are not implanted, and some of them, 1 or 2 may remain outside the body for tracking the said object. Hence, it avoids the need to implant transponders into the subject.

A treatment pose of the positioning tool (200) may be adjusted while the subject (50) is on the treatment table (512). Although the robotic arm, RA, (400, 400a) replicates the pose of the positioning tool (200) determined by the simulation of treatment, the pose of the positioning tool (200) may be subsequently fine-tuned based on real-time positional information of the positioning tool (200) obtained while the subject is on the treatment table (512). After the adjustment has been made, the tissue target is irradiated.

The inventors have found that, in a certain percentage of cases (around 30 - 40 %), the positioning of the subject on the treatment table cannot be repeatably reproduced. There may a difference in positioning in a range of several mm between the position of the subject on the simulation table and treatment table, and/or between the position of the subject on the treatment table in successive fractionated treatments. The difference can depend on the patient; sometimes there is a slight difference in the body contour because the subject has waited some weeks after simulation to start the treatment, and during this period the patient has gained or lost weight. Alternatively, the subject may experience a new pain, and some muscles are locally tensed, so the subject is able to reach exactly the same position. The difference can depend on the user (e.g. technician) who might be introducing placement error because the patient is difficult to move, or other inherent errors. The small distance deviation is significant, because the target would not receive the maximal dose and there may be an increase in damage to healthy structures.

The ability to fine-tune the pose of the positioning tool (200) based on real-time positional information of the positioning tool (200) obtained while the subject is fitted with the positioning tool (200) on the treatment table (512) hence increases efficacy and reduces side-effects.

Manual, non-robotic positioning devices (manual positioner), are available to adjust and lock a pose of the positioning tool (200), but which disadvantageously are less accurate, more time consuming, are less safe, and expensive to employ compared with a robotic arm.

A manual positioner is more time consuming to position because each axis is adjusted separately (e.g. by turning a thread shaft connected to each axis (x, y, z)), making it difficult and time consuming to bring the positioning tool (200) into the precise position. With the manual positioner, the user (physician) would need manually to displace and fix the pose of manual arm several times. A new medical image would need to be taken to confirm each new pose to determine whether the positioning tool (200) is held correctly in relation to bony structure; this is time consuming as the staff need to leave the treatment room while each image is taken. Hence, the procedure using a manual positioner is iterative and time consuming. With a robotic arm, the angles adopted by each joint does not need to be set separately; once the pose of the positioning tool (200) is known, the angles of the joints are set automatically using standard methods.

A manual positioner is more time difficult to reach, because the treatment/simulation table is usually placed high for alignment with the ionising-radiation treatment head. Either the user (physician) has to stretch over to make adjustments which is sub-optimal, or the table is lowered which increases time spent. The robotic arm allow the pose of the positioning tool (200) to be adjusted and fixed remotely.

Using the manual positioner, there is a high exposure to X-ray because the correct pose is determined iteratively, with an X-ray being taken after each iteration. With the robotic arms, the treatment pose may be based on the simulation pose and does not require X-rays.

Because the present robotic arm controls movement of the positioning tool (200), movements are more accurate. With a manual positioner, each adjustment to the pose requires a manual unlocking of each joint (usually 3), followed by manual locking after adjustment. The locking and unlocking is sequential: when a first joint is locked, a force is applied to a second joint to change its position, which will cause slight movement and displacement of the position of the first joint. Each time a joint is adjusted, it causes a slight movement in another joint. Adjustment are hence made iteratively to all three joints until the correct pose is realized. The maneuvering is highly complex for manual execution, it is time consuming and not accurate.

Experience shows that if a manual positioner applies the same pose to the positioning tool (200) at each treatment session, the positioning tool (200) will mostly not be positioned exactly in the same position inside the subject body relative to bony structures, because the body will often have a very slight shift in shape compared to the previous positions, a few degrees at least. These daily changes are because the subject patient is more tense, has weight loss, has weight increase, or oedema, or other factors. With the manual positioner, the pose used at the first treatment session is manually locked and is used in subsequent treatment sessions, hence, there is no account for this observed body change, hence less of the target is irradiated and there is more toxicity. Because the robotic arm controls movement of the positioning tool (200), pose of the positioning tool (200) can be fine tuned quickly at the start of every session, by reference, for instance, to bony structures. With the robotic arm, and markers and/or transponder, the fine-tuned pose is reached automatically would be can be completed in a few seconds, by one technician. The user (physician) does not have to be there. Further, there is less irradiation exposure.

Because the robotic arm control movement of the positioning tool (200), movements may be along a fixed trajectory, and be executed very slowly such a few (1-3) mm per minute. With a manual arm, the user (physician) will aim to reach the optimum pose in the smallest number of moves and in the shortest possible time. This will cause pain to the subject because the subject's tissue does not have time to accommodate large displacements. Moreover, the physician can unintentionally apply excessive force when meeting resistance in order to quickly reach a pose, which can be highly painful for the subject.

The *in situ* pose of the positioning tool (200) may be fine-tuned based on one or more bony-references, which is visible by a built-in medical imaging unit (e.g. X-ray system, low resolution CT scan). An example of a bony reference is the pelvis. The pose of the positioning tool (200) is adjusted during treatment until it has the same pose in relation to the one or more bony-references as it had during simulation.

Real-time information as to an *in situ* pose of the positioning tool (200) may be obtained from a medical imaging unit disposed in known relation to the positional reference (e.g. radiotherapy treatment table (512) or in the relation to the ionizing radiation treatment head (518)).

Some radiotherapy devices (510) are equipped with a built-in medical image unit (e.g. X-ray system, low resolution CT scan) allowing medical images (e.g. X-ray images) of the bodily tissue and positioning tool (200) to be taken while the subject is on the radiotherapy treatment table. The real-time *in situ* pose is updated as the fine adjustment of the positioning tool (200) can be determined form the medical image. As mentioned elsewhere herein, at least a part of the positioning tool (200) may be visible by medical imaging and/or the positioning tool (200) may be disposed with one or more imaging markers. The effector shaft (310) may be disposed with one or more (preferably at least three) imaging markers (350, a, b, c). The transmission (314) may be disposed with one or more (preferably at least three) imaging markers (350, a, b, c).

Real-time information as to an *in situ* pose of the positioning tool (200) may be obtained from a positional transponder reader disposed in known relation to the positional reference (e.g. radiotherapy treatment table (512) or in the relation to the ionizing radiation treatment head (518)). The radiotherapy device (510) may be equipped with a positional transponder reader allowing accurate positional determination of one or more position-determining radio transponders present in the positioning tool (200) to be determined while the subject is on the radiotherapy treatment table (512). As described elsewhere herein, the transponders of the positioning tool (200) may be provided on the inserter (204) and/or on the steering guide (300) to guide the fine-tuning movement. In some radiotherapy devices (510) the transponder reader is a system that is brought close to the radiotherapy treatment table when needed, and placed aside when not needed. The position of the transponder reader position relative to the positional reference is communicated to the radiotherapy devices (510) when the transponder reader is switched on. A fine adjustment of the positioning tool (200) can be derived from the positional transponder reader data.

The fine-tune adjustment may be performed manually (e.g. guided movement by the operator of the robotic arm operating in a manual zero-gravity mode), automatically (e.g. by the robotic arm alone), or semi-automatically (e.g. both manually and robotically).

The fine adjustment may be stored and applied during a subsequent treatment session.

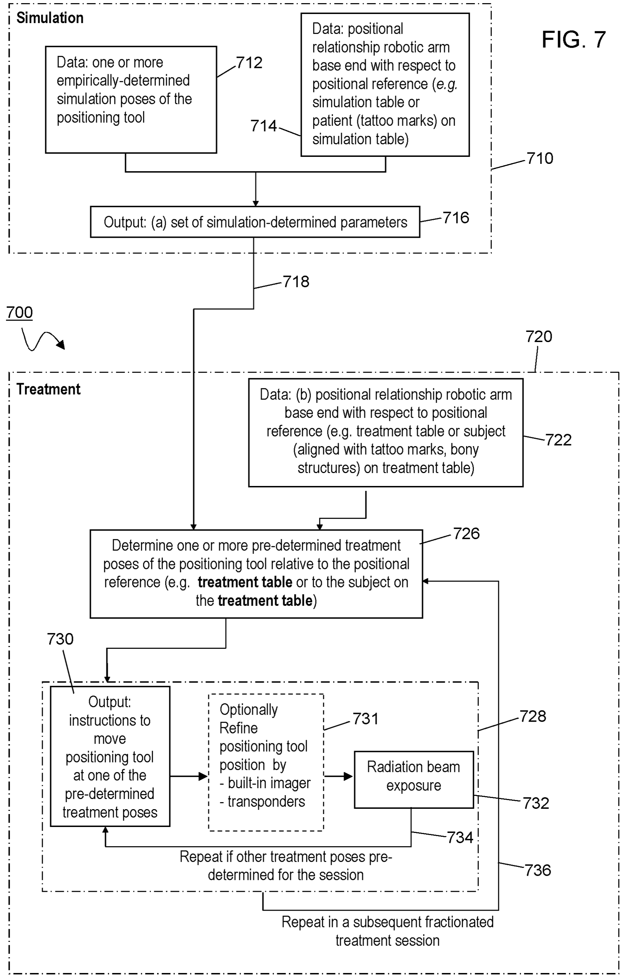

One or more of the methods described here may be implemented on a computer. Provided herein is computer implemented method (700) for controlling a radiotherapy treatment system (100) as described herein for assisting an external radiotherapy programme comprising one or more external radiotherapy treatment sessions applied to a subject. An exemplary computer implemented method (700) is depicted in the flow chart of **FIG. 7****.** The positioning tool (200) comprises the steering guide (300), and optionally the inserter (204), depending on the application..

Provided herein is computer implemented method (700) comprising:
- receiving (a) a set of simulation-determined parameters (716) comprising one or more empirically-determined simulation poses of the positioning tool (200) with respect to a positional reference such as a simulation of a beam intersection volume, a simulation of an isocentre, or a bony pelvis of the subject placed on and in known position with a simulation table (522) and
- (b) a positional relationship (SPx,y,z) of the RA base end (422, 422b) with respect to a positional reference (714) such as a simulation of a beam intersection volume, a simulation of an isocentre, or a bony pelvis of the subject placed on and in known position with a simulation table (522),
- receiving (c) data (722) on a positional relationship (TPx,y,z) of the RA base end (422, 422a) on the radiotherapy treatment table (512) with respect to the positional reference such as a beam intersection volume, an isocentre, or a bony pelvis of the subject placed on and in known position with the radiotherapy treatment table (512);
- determining (726) from (a), (b) and (c) one or more treatment poses of the positioning tool (200) that corresponds with the one or more empirically-determined simulation poses of the positioning tool (200).

The computer implemented method (700) may further comprise the steps:
- outputting instructions (730) to actuate the robotic arm, RA, mountable or mounted on the treatment table (512) in fixed and known (722) relation to the positional reference (e.g. to the beam intersection volume, the isocentre, or bony pelvis of the subject placed on and in known position with the radiotherapy treatment table (512)) to move and/or fix positioning tool (200) to one of the treatment poses, prior to emission of radiation from the radiotherapy device (510),
- optionally (731) outputting instructions (730) to actuate the robotic arm, RA, to fine-tune the in-situ pose of the positioning tool (200) by (manual) actuation of the RA, based on the actual pose of the positioning tool (200) in situ (e.g. determined by a medical imaging unit or transponder reader built into the radiotherapy device system (510)).
- optionally outputting instructions (734) to actuate the robotic arm, RA, to move and/or fix positioning tool, (200) to another of the treatment poses prior to emission of radiation from the radiotherapy device (510).
- optionally repeating (736) the above steps for at least 1, 2 or preferably all of the treatment sessions.

The step that may be repeated in each treatment session may be the steps of
- outputting instructions (730) to actuate the robotic arm, RA, mountable or mounted on the treatment table (512) in fixed and known (722) relation to the positional reference (e.g. to the beam intersection volume, the isocentre, or bony pelvis of the subject placed on and in known position with the radiotherapy treatment table (512)) to move and/or fix the positioning tool (200) to one of the treatment poses, prior to emission of radiation from the radiotherapy device (510),
- optionally (731) outputting instructions (730) to actuate the robotic arm, RA, to fine-tune the in-situ pose of the positioning tool (200) by (manual) actuation of the RA, based on the actual pose of the positioning tool (200) in situ (e.g. determined by a medical imaging unit or transponder reader built into the radiotherapy device system (510)).
- optionally outputting instructions (734) to actuate the robotic arm, RA, to move and/or fix the positioning tool, (200) to another of the treatment poses prior to emission of radiation from the radiotherapy device (510).

The same set of simulation-determined parameters (716) may be used to determine the one or more treatment poses for at least 1, 2, and preferably all of external radiotherapy treatment sessions.

The one or more empirically-determined simulation poses of the positioning tool (200) may be determined comprising:
(i) outputting instructions to actuate the robotic arm, RA, mountable in fixed relation to the simulation table (522) to move and/or fix the positioning tool (200) (responsive to input from the specialist),
(ii) receiving an instruction to record one or more poses of the positioning tool (200) relative to the subject (50) as the one or more empirically-determined simulation poses.

Computer program or computer program product is also provided having instructions which when executed by a computing device or system cause the computing device or system to perform the computer-implemented method.

A computer readable medium is also provided having stored thereon the computer program or computer program product.

A data stream is also provided which is representative of the computer program or computer program product.

Provided is a method for assisting an external radiotherapy programme comprising one or more external radiotherapy treatment sessions applied to a subject comprising:
(a) obtaining a set of simulation-determined parameters for the subject (50) comprising the steps:
   - inserting a positioning tool (200) into a canal of the subject (50),
   - aligning the subject (50) on a simulation table (522) (and marking the position of the relative to at least one projected laser reference line by a tattoo marks on the subject),
   - moving the robotic arm (400, 400b) RA fitting (430, 430b) towards the positioning tool (200) and attaching robotic arm (400, 400b) RA fitting (430, 430b) to the positioning tool (200),
   - obtaining one or more medical images of the subject (50) on the simulation table (522),
   - determining (by the specialist) from the medical images one or more empirically-determined simulation poses of the positioning tool (200),
   - determining a set of simulation-determined parameters from the one or more empirically-determined simulation poses and a positional relationship (SPx,y,z) of a RA base end (422, 422b) with respect to the positional reference (e.g. simulation of a beam intersection volume, a simulation of an isocentre, or a bony pelvis of the subject placed on and in known position with a simulation table (522))),
   - detaching the RA fitting (430b) from the positioning tool (200),
(b) carrying out a session of radiotherapy treatment on the subject (50) comprising the steps:
   - optionally inserting a positioning tool (200) into a canal of the subject (50) (if not already *in situ*),
   - aligning the subject (50) on a radiotherapy treatment table (512) (using e.g. by tattoo marks on the subject),
   - moving the robotic arm (400, 400a) RA fitting (430, 430a) towards the positioning tool (200) to and attaching robotic arm (400, 400a) RA fitting (430, 430a) to the positioning tool (200),
   - determining one or more treatment poses of the positioning tool (200) from set of simulation-determined parameters and a positional relationship (TPx,y,z) of a RA base end (422, 422a) with respect to the positional reference (e.g. such as a beam intersection volume, an isocentre, or a bony pelvis of the subject placed on and in known position with the radiotherapy treatment table (512)),
   - setting the positioning tool (200) to each of one of the treatment poses,
   - treating the subject by exposure to an ionising radiation beam at each treatment pose,
   - detaching the positioning tool (200) from the RA fitting (430a),
(c) optionally carrying out one or more subsequent sessions of radiotherapy treatment comprising the steps under (b).

Provided is a method for assisting treatment a subject in an external radiotherapy programme comprising one or more external radiotherapy treatment sessions comprising:
(a) obtaining a set of simulation-determined parameters for the subject (50), wherein
   - the subject is positioned on a radiotherapy simulation table (522),
   - a distal end of a positioning tool (200) is inserted into a canal (602) of the subject (50),
   - a set of simulation-determined parameters for the subject (50) comprising one or more empirically-determined treatment poses in relation to the positional reference (*e.g*. simulation table (522), subject (50) on the simulation table (522) (e.g. a simulation of a beam intersection volume, a simulation of an isocentre, or a bony pelvis of the subject placed on and in known position with a simulation table (522)) is obtained, wherein an empirically-determined treatment pose corresponds to a pose of the positioning tool (200) such that, during the external radiotherapy treatment session, the canal (602) and/or bodily tissue (608) connected to the canal of the subject (50) on the radiotherapy table (512) is in known position and/or orientation in relation to *(e.g.* in or away from) an ionising radiation beam emitted by an ionising-radiation treatment head (518)), more in particular to an intersection volume or isocentre.
(b) carrying out an external radiotherapy treatment session on the subject, wherein
   - the subject is positioned on a radiotherapy table (512),
   - a distal end of a positioning tool (200) is inserted into a canal (602) of the subject (50),
   - the pose of the positioning tool (200) is adjusted to a treatment pose that corresponds with the one or more empirically-determined simulation poses of the positioning tool (200), such that, during an external radiotherapy treatment session, the canal (602) and/or bodily tissue (608) of the subject (50) is in known position and/or orientation in relation to *(e.g.* in or away from) the positional reference (*e.g.* the beam intersection volume, the isocentre, or the bony pelvis of the subject placed on and in known position with a treatment table (512).

The pose of the positioning tool (200) may be adjusted by a robotic arm, RA, (400, 400a) having a base end (422, 422a), wherein the base end (422, 422a) is mounted in a known and/or defined and/or the same position on radiotherapy treatment table (512) for each external radiotherapy treatment session.

The subject (50) may be placed on the radiotherapy treatment table (512) in a known and/or defined and/or at the same position for at least 1, 2, and preferably all of external radiotherapy treatment sessions.

The treatment pose of the positioning tool (200) may be the same for at least 1, 2, and preferably all of external radiotherapy treatment sessions.

The longitudinal distance between the subject (50), more preferably the bony structures of the subject (50), and the base end (422, 422a) of the RA may be the same for each external radiotherapy treatment session.

The bodily tissue (608) of the subject (50) may be comprised in the uterus, the cervix uteri, the vagina, the bladder, the anus, the rectum, a vaginal vault, a vaginal vault mass, or the prostate.

The method may employ the system described herein. The positioning tool (200) may be that defined herein.

Prior to the step of treating the subject by exposure to an ionising radiation beam at each treatment pose, the treatment pose of the positioning tool (200) may be adjusted or fine-tuned (e.g. manually) while the subject (50) is on the treatment table (512), as described elsewhere herein .

The pose of the positioning tool (200) may be subsequently fine-tuned after the pose of the positioning tool (200) has been adjusted to correspond with the one or more empirically-determined simulation poses of the positioning tool (200). The fine-tuned pose is the treatment pose of positioning tool (200)).

The fine-tuned pose may be determined from real-time *in situ* measurement information of the pose of the positioning tool (200) relative to subject on the radiotherapy treatment table (512), wherein the *in situ* measurement information is obtained from:
- a medical imaging unit built-in to a radiotherapy device (510) comprising the ionising-radiation treatment head (518).
- a positional transponder reader built-in to a radiotherapy device (510) comprising the ionising-radiation treatment head (518).

The pose of the positioning tool (200) obtained by the medical imaging unit may be relative to the positional reference (e.g. one or more bony structures of the subject (50)).

Further provided is a method for aligning a subject (50) and robotic arm, RA, (400, 400a, 400b) on a radiotherapy treatment table (512) for an external radiotherapy programme comprising a treatment simulation and one or more external radiotherapy treatment sessions applied to a subject using a system described herein comprising:
(a) during simulation of treatment:
   - attaching a measurement gauge (524) to a long edge of the simulation table (522), so it crosses at least one projected laser reference line (526) and extends towards the expected position of an RA base end (422, 422b) or base support (432, 432b) of the RA (400, 400b) on the simulation table (522),
   - recording as a "gauge position", the position measurement gauge (524) relative to the positional reference,
   - recording as a "laser position", the position indicated by the measurement gauge (524) where the at least one projected laser reference line (526) crosses it,
   - placing the subject (50) on the simulation table (522) such that a midline of the subject is aligned with a longitudinal midline of the simulation table (522),
   - marking the subject (50) with at least one tattoo mark (52) aligned with the at least one projected laser reference line (526),
   - placing the RA (400, 400b) on the simulation table (522) such that an RA fitting (430, 430b) of the RA (400, 400b) is positioned for dismountable attachment to the positioning tool (200) for simulation of treatment,
   - recording, as a "base position", the position indicated by the measurement gauge (524) of the RA base end (422, 422b) or base support (432, 432b) on the simulation table (522),
(b) during radiotherapy treatment:
   - attaching the same or an identical measurement gauge (514) to a long edge of the treatment table (512) at the "gauge position" on the treatment table (512),
   - adjusting the longitudinal position of the treatment table (512) such that the at least one projected laser reference line (526) crosses the measurement gauge (514) on the treatment table (512) at the "laser position",
   - placing the subject (50) on the treatment table (512) such that a midline of the subject is aligned with a longitudinal midline of the treatment table (512),
   - moving the subject (50) such that that tattoo marks (52) on the subject (50) are aligned with the at least one projected laser reference line (516),
   - placing the RA (400, 400a) on the treatment table (512) such that an RA fitting (430, 430b) of the RA (400, 400a) is positioned for dismountable attachment to the positioning tool (200) for radiotherapy treatment,
   - adjusting the position of an RA base end (422, 422a) or base support (432, 432a) such that it is equal to the "base position" as indicated by the measurement gauge (514) on the treatment table (512).

During subsequent treatment sessions or fractions, steps under part (b) are repeated. It is noted that it is standard practice that both the simulation table (522) and the radiotherapy treatment table (512) are typically identical in shape, and the arrangement of projected laser reference lines (516, 526) is also identical in both simulation room and radiotherapy treatment room.

The present invention allows an effective treatment of patients with inoperable cancers because it can accurately target large tumours that are normally excluded from external radiation therapy because of the high dose. Where external radiation is not suitable, brachytherapy (internal radiotherapy) is an alternative, however such treatment has little/no effect on large a tumour mass.

In one example, one patient presented with a 15 cm tumour mass outer diameter and another patient had presented with an 8 cm tumour mass outer diameter. Such patients were considered impossible to treat curatively by standard external radiotherapy because the last part of the treatment is delivered using brachytherapy (internal radiotherapy) which can hardly boost such large tumour diameters. Using the present system and methods, both patients were free of cancer, attributable to the accuracy of delivery of the ionising radiation. Other examples are given in **FIGs. 42A, 42B****,** and **43** described elsewhere herein.

## Claims

1. A radiotherapy treatment system (100) for assisting treatment of a subject in an external radiotherapy programme comprising one or more external radiotherapy treatment sessions, the system (100) comprising:
- a robotic arm, RA, (400, 400a) having a base end (422, 422a) and an effector end (424, 424a), wherein
- the base end (422, 422a) is mounted on or mountable in fixed relation to a radiotherapy treatment table (512) for treating a subject (50),
- the effector end (424, 424a) is disposed with a RA fitting (430, 430a) for dismountable attachment to a positioning tool (200) having a proximal and distal end, the distal end configured for insertion into a canal of the subject (50),
**characterised in that** it further comprises:
- a processing unit (440) comprising at least one processor and a memory, wherein the processing unit (440) is configured to control and fix movement of the robotic arm, RA, (400, 400a) during the external radiotherapy treatment session, and
- the positioning tool (200) comprising:
- a steering guide (300) having a proximal (40) and distal (20) end, comprising:
- a rigid effector shaft (310) at the distal end (20) configured for insertion into the canal (602) of the subject (50), or for attachment to an inserter (204) configured for insertion into the canal (602) of the subject (50),
- a rigid handle portion (316) disposed at the proximal end (40) in fixed relation to the effector shaft (310) for controlling the position and/or direction of the effector shaft (310) and configured for attachment to the RA fitting (430, 430a),
- a rigid transmission (314) joining the handle portion (316) to the effector shaft (310),
wherein the positioning tool (200) is configured to move and/or fix the canal (602) for the external radiotherapy treatment session responsive to movements of the robotic arm.

2. The radiotherapy treatment system (100) according to claim 1, wherein the processing unit (440) is configured to position the positioning tool (200) at one or more treatment poses, wherein a treatment pose corresponds a spatial alignment by the positioning tool (200) of a canal (602) of the subject (50) for the external radiotherapy treatment.

3. The radiotherapy treatment system (100) according to claim 1 or 2, wherein the processing unit (440) is configured to determine the one or more treatment poses from one or more empirically-determined simulation poses of the positioning tool (200).

4. The radiotherapy treatment system (100) according to any one of claim 1 to 3, wherein the processing unit (440) is configured to:
- receive real-time information as to the *in-situ* pose of the positioning tool (200) after the pose of the positioning tool (200) has been adjusted to correspond with the one or more empirically-determined simulation poses of the positioning tool (200),
- provide real-time manual, automatic or semi-automatic guidance to fine-tune the pose of the positioning tool (200),
- optionally wherein the real-time information as to the *in-situ* pose of the positioning tool (200) is obtained from a medical imaging unit disposed in known relation to the radiotherapy treatment table (512) or from a positional transponder reader disposed in known relation to the radiotherapy treatment table (512).

5. The radiotherapy treatment system (100) according to claim 4, wherein the real-time information as to the *in-situ* pose of the positioning tool (200) is obtained from a medical imaging unit disposed in known relation to the radiotherapy treatment table (512) or from a positional transponder reader disposed in known relation to the radiotherapy treatment table (512).

6. The steering guide (300) according claim 5, wherein the rigid effector shaft (310) and/or rigid transmission (314) and/or rigid handle portion (316) is provided with one or more position-determining radio transponders (352, a, b, c, d; ) whose positions can be determined and tracked using the positional transponder reader.

7. The radiotherapy treatment system (100) according to any one of claims 1 to 6, further comprising a knee support (450) configured to support the knees of the subject lying in a semi-supine position on the radiotherapy treatment table (512) or simulation table (522), wherein the knee support (450):
- comprises a body (451) having a base end (453) and opposing supporting side (457) configured to support both posterior each knee of the subject on the radiotherapy treatment table (512) or simulation table (522),
- is configured for dismountable attachment to and in fixed relation to the radiotherapy treatment table (512) or simulation table (522),
- body (451) comprises a slot (454) into which a base support (432, 432a, 432b) of the RA can lockably slide in one or more positions defined in relation to the radiotherapy treatment table (512) or simulation table (522) and/or to the body (451) of the knee support (451),
and
one or both lateral sides (456, 458) of the knee support (450) may be markable or be predisposed with a reference marking (464, 465) to indicate a position where a projected laser reference line (516, 526) crosses the one or both two lateral sides (456, 458).

8. The radiotherapy treatment system (100) according to any one of claims 1 to 7, further comprising a knee support (450) configured to support the knees of the subject lying in a semi-supine position on the radiotherapy treatment table (512) or simulation table (522), wherein the knee support (450):
- comprises a body (451) having a base end (453) and opposing supporting side (457) configured to support both posterior each knee of the subject on the radiotherapy treatment table (512) or simulation table (522),
- the body contains the base end (422, 422a) of the robotic arm, RA, (400, 400a).

9. The radiotherapy treatment system (100) according to 8, wherein the knee support (450) contains two or more parts that mutually attach together to form the knee support (450), wherein
- one part of the knee support (450, a) contains two knee rests (466, 468), one supporting each knee posterior, and optionally a battery pack and optionally the processing unit,
- another part of the knee support (450, b) contains a base support (432, 432a, 432b) of the robotic arm, RA, (400, 400a).

10. The radiotherapy treatment system (100) according to any one of claims 1 to 9, wherein the RA fitting (430, 430a) is connected to the RA (400, 400a) via linkage fuse, wherein the linkage fuse is configured to:
- tolerate a working load without breaking, and
∘ break with an application of an abusive load releasing mechanical connection between the RA fitting and the RA (400, 400a) and/or
∘ break responsive to activation of an emergency button.

11. The radiotherapy treatment system (100) according to any one of claims 1 to 10, wherein the positioning tool (200) comprises an inserter (204) having a proximal (40) and distal (20) end which inserter (204) comprises:
- an elongated member (210) configured for insertion through an entrance of and into the canal; and
wherein the effector shaft (310) at the distal end (20) is attached or dismountably attachable to the elongated member (210).

12. The radiotherapy treatment system (100) according to any one of claims 1 to 11, wherein the positioning tool (200) is made at least partly from a material that is MRI compatible material, and
- is MRI visible and/or
- contains one or more MRI visible markers.

13. The radiotherapy treatment system (100) according to any one of claims 1 to 12, wherein the steering guide (300) is provided with an image capture system (360) configured to capture images from a distal tip (361) of the effector shaft (310) allowing insertion of the effector shaft (310) into the inserter (204) elongated member lumen (214) under guidance of images captured.

14. The radiotherapy treatment system (100) according to any one of claims 1 to 13, wherein the handle portion (316) of the steering guide (300) is disposed with a docking beacon (340) configured to provide real-time information as to the position and optionally orientation of the steering guide (300) relative to the RA fitting (430), wherein the controller (440) is configured to provide manual, semi-automatic or automatic guidance to allow docking of the RA fitting (430) to the handle portion (316).

15. The radiotherapy treatment system (100) according to any one of claims 1 to 14, wherein the RA (400, 400a) is provided with a switchable zero-gravity mode, wherein:
- said RA (400, 400a) in a zero-gravity-on mode:
- allows manual guidance of the RA fitting (430, 430a, 430b) for docking with the handle portion (261); and
- continues to register the pose of the RA fitting (430, 430a, 430b),
- said RA (400, 400a) in a zero-gravity-off mode:
- initially determines the pose of the RA fitting (430, 430a, 430b) from a last registration of the pose of the RA fitting (430, 430a, 430b) upon exiting the zero-gravity-on mode, without an intervening calibration manoeuvre.

16. The radiotherapy treatment system (100) according to any one of claims 1 to 15, wherein the processing unit (440) is configured to receive an input of a pivot point assignable to the positioning tool (200), wherein the pivot point is a point or region of the positioning tool (200) around which movements of positioning tool (200) by the RA, (400, 400a) are pivoted.
